(19) Europäisches Patentamt
European Patent Office
Office européen des brevets

(11) Publication number: 0 117 228
B1

(12) # EUROPEAN PATENT SPECIFICATION

(45) Date of publication of the patent specification: 05.11.86

(21) Application number: 84810039.2

(22) Date of filling: 23.01.84

(51) Int. Cl.⁴: C 07 C 59/56, C 07 C 69/732, C 07 C 103/38, C 07 D 261/08, C 07 C 47/546, C 07 D 309/10, C 07 C 39/367, C 07 C 43/29, C 07 C 59/64, C 07 C 33/46, C 07 C 25/18

(54) **Analogs of mevalonolactone and derivatives thereof, processes for their production, pharmaceutical compositions containing them and their use as pharmaceuticals.**

(30) Priority: 24.01.83 US 460600

(43) Date of publication of application: 29.08.84 Bulletin 84/35

(45) Publication of the grant of the patent: 05.11.86 Bulletin 86/45

(84) Designated Contracting States: AT BE CH DE FR GB IT LI LU NL SE

(56) References cited: EP-A-0 011 928

(73) Proprietor: SANDOZ AG, Lichtstrasse 35, CH-4002 Basel (CH)
(84) Designated Contracting States: BE CH FR GB IT LI LU NL SE

(73) Proprietor: SANDOZ-PATENT-GMBH, Humboldtstrasse 3, D-7850 Lörrach (DE)
(84) Designated Contracting States: DE

(73) Proprietor: SANDOZ-ERFINDUNGEN Verwaltungsgesellschaft m.b.H., Brunner Strasse 59, A-1235 Wien (AT)
(84) Designated Contracting States: AT

(72) Inventor: Anderson, Paul LeRoy, 264 Center Grove Road, Randolph, N.J., 07869 (US)

EP 0 117 228 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1986

**Description**

The invention concerns naphthalene and tetrahydronaphthalene analogs of mevalonolactone and derivatives thereof, processes for their production, pharmaceutical compositions containing them and their use as pharmaceuticals in particular as hypolipoproteinemic and antiatherosclerotic agents.

The invention is especially concerned with compounds of formula I

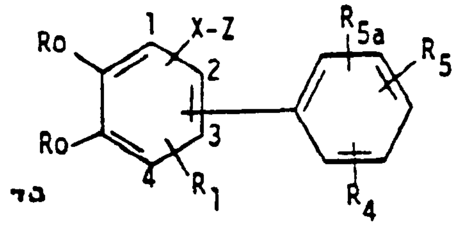

I

wherein the two groups Ro together form a radical of formula

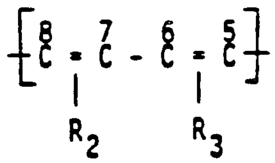

or $-(CH_2)_4-$ ,

wherein $R_2$ is hydrogen, $C_{1-4}$alkyl, $C_{1-4}$alkoxy, (except t-butoxy), trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the provisos that not more than one of $R_2$ and $R_3$ is trifluoromethyl, not more than one of $R_2$ and $R_3$ is phenoxy, and not more than one of $R_2$ and $R_3$ is benzyloxy,

$R_1$ is hydrogen, $C_{1-6}$alkyl, fluoro, chloro or benzyloxy,

$R_4$ is hydrogen $C_{1-4}$alkyl, $C_{1-4}$alkoxy, (except t-butoxy), trifluoromethyl, fluoro chloro phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_{5a}$ is hydrogen, $C_{1-2}$alkyl, $C_{1-2}$alkoxy, fluoro or chloro,

with the provisos that not more than one of $R_4$ and $R_5$ is trifluoromethyl, not more than one of $R_4$ and $R_5$ is phenoxy and not more than one of $R_4$ and $R_5$ is benzyloxy,

X is $\overset{5}{-(CH_2)_n-}$ or 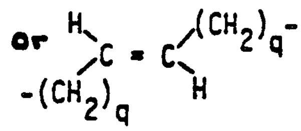

wherein n is 0, 1, 2 or 3 and

both q's are 0 or one is 0 and the other is 1, and

Z is 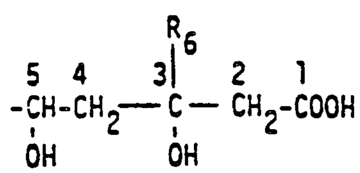 II

wherein $R_6$ is hydrogen or $C_{1-3}$alkyl,

with the general proviso that -X-Z and the $R_4$-bearing phenyl group are ortho to each other;

in free acid form or in the form of a physiologically-hydrolysable and -acceptable ester or a γ lactone thereof or in salt form.

By the term "physiologically-hydrolysable and -acceptable ester" is meant an ester of a compound in

accordance with the invention in which the carboxyl moiety is esterified, and which is hydrolysable under physiological conditions to yield an alcohol which is itself physiologically acceptable, e.g. nontoxic at desired dosage levels. Preferred such esters as Z can be represented together with the free acid by formula IIa

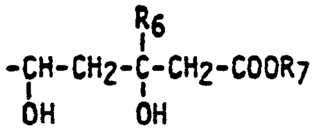

IIa

wherein $R_7$ is hydrogen, $C_{1-4}$alkyl or benzyl preferably hydrogen, $C_{1-3}$alkyl, n-butyl, i-butyl, t-butyl or benzyl and $R_6$ is as defined above.
When in salt form R7 represents a cation.
When Z is in lactone form it forms a γ-lactone of formula IIb

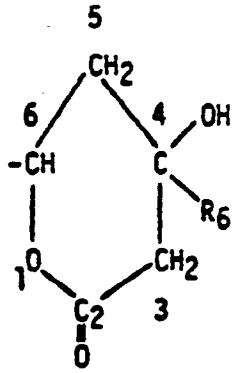

IIb

--and references to "lactone" hereinafter refer to γ-lactones.

Salts of the compounds of the invention, e.g. of the compounds of formula I, include in particular their pharmaceutically acceptable salts. Such pharmaceutically acceptable salts include e.g. alkali metal salts such as the sodium and potassium salts and ammonium salts.

X-Z, $R_1$ and the $R_4$-bearing phenyl ring occupy any of positions 1 to 4 subject to the proviso that X-Z and the $R_4$-bearing phenyl group are ortho to each other. $R_2$ and $R_3$ occupy any of positions 5 to 8. References to compounds of formula I, II and sub-species thereof are intended to cover all forms unless otherwise stated.

The compounds of formula I may be divided into two groups, the compounds of formula IA and IB:

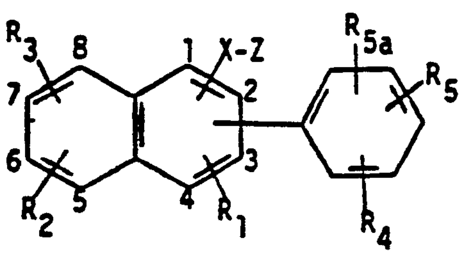

and

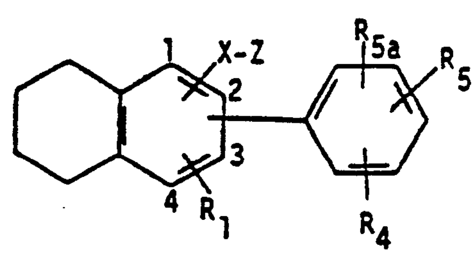

(IA) (IB)

wherein $R_1$ to $R_{5a}$, X and Z are as defined above.

The compounds of formula IA may be divided into two subgroups, the compounds wherein Z is a group of formula II in other than lactone form (Group IAa) and those wherein Z is a group of formula IIb(Group IAb). Likewise, the compounds of formula IB may be divided into two sub-groups, the compounds wherein Z is a group of formula II in other than lactone form (Group IBa) and those wherein Z is a group of formula IIb (Group IBb).

Each of those four sub-groups may be further divided into three further sub-groups, viz., the compounds wherein the -X-Z group is in the 1-position and the $R_4$-bearing phenyl group is in the 2-position (Groups IAa1, IAb1, IBa1 and IBb1), the compounds wherein the -X-Z group is in the 2-position and the $R_4$-bearing phenyl group is in the 1-position (Groups IAa2, IAb2, IBa2, IBb2) and the compounds wherein the -X-Z group is in the 2-position and the $R_4$-bearing phenyl group is in the 3-position (Groups IAa3, IAb3, IBa3 and IBb3).

As is self-evident to those in the art, each compound of formula I (and every sub-scope and species thereof) has at least two centers of asymmetry (e.g. the two carbon atoms bearing the hydroxy groups in the group of

formula IIa and the carbon atom bearing the hydroxy group and the carbon atom having the free valence in the group of formula IIb) and these lead (e.g. with two centers) to four stereoisomeric forms (enantiomers) of each compound (two racemates or pairs of diastereoisomers). In the preferred compounds having only two such centers of asymmetry these four stereoisomers may be designated as the R, R; R,S; S,R; and S,S enantiomers, all four stereoisomers being within the scope of this invention. Depending on the nature of substituents further asymmetric carbon atoms may be present and the resulting isomers and mixtures thereof also form part of the invention. Compounds containing only two centers of asymmetry (four mentioned stereoisomers) are preferred.

$R_1$ is preferably $R_1'$, where $R_1'$ is hydrogen, $C_{1-6}$alkyl not containing an asymmetric carbon atom or chloro, more preferably $R_1''$, where $R_1''$ is hydrogen or $C_{1-3}$alkyl, and most preferably $R_1'''$, where $R_i''$ is hydrogen, $C_{1-2}$alkyl or isopropyl.

Preferably, $R_1$ (R1' etc.), when other than hydrogen, is in the 3-position in compounds of Groups IAa2, IAb2, IBa2 and IBb2 and in 1-position in compounds of Groups IAa3, IAb3, IBa3, IBb3. Alkyl as $R_2$ is preferably $C_{1-3}$ or n-, i- or t-butyl and alkoxy $C_{1-3}$ or n- or i-butoxy.

$R_2$ is preferably $R_2'$, where $R_2'$ is hydrogen, $C_{1-3}$alkyl, $C_{1-3}$-alkoxy, trifluoromethyl, fluoro, chloro, phenoxy or benzyloxy, more preferably $R_2'$, where $R_2'$ is hydrogen, methyl, methoxy, fluoro or chloro, and most preferably hydrogen.

$R_3$ is preferably $R_3'$, where $R_3'$ is hydrogen, $C_{1-2}$alkyl, $C_{1-2}$-alkoxy, fluoro or chloro, more preferably $R_3''$, where $R_3''$ is hydrogen, methyl, methoxy, fluoro or chloro, and most preferably hydrogen.

Preferably, when both $R_2$ and $R_3$ are other than hydrogen, at least one of them is in the 6- or 7-position and not more than one of them is a member of the group consisting of t-butyl, trifluoromethyl, phenoxy and benzyloxy.

Alkyl as $R_4$ is preferably $C_{1-3}$ or n-, i- or t-butyl and alkoxy $C_{1-3}$ or n- or i-butoxy.

$R_4$ is preferably $R_4'$, where $R_4'$ is hydrogen, $C_{1-3}$alkyl, $C_{1-3}$-alkoxy, trifluoromethyl, fluoro, chloro, phenoxy or benzyloxy. more preferably $R_4''$, where $R_4''$ is hydrogen, methyl, methoxy, fluoro or chloro, and most preferably $R_4''$, where $R_4''$ is hydrogen or fluoro, especially hydrogen or 4-fluoro and most especially 4-fluoro.

$R_5$ is preferably $R_5'$, where $R_5'$ is hydrogen, $C_{1-2}$alkyl, $C_{1-2}$alkoxy, fluoro or chloro, more preferably $R_5''$, where $R_5''$ is hydrogen, methyl, methoxy, fluoro or chloro, and most preferably hydrogen.

$R_{5a}$ is preferably $R_{5a}'$, where $R_5'$ is hydrogen or methyl and most preferably hydrogen.

Preferably, when $R_4$ ($R_4'$, $R_4''$, etc.) is other than hydrogen and $R_5$ ($R_5'$, $R_5'$, etc.) and $R_{5a}$ ($R_{5a}'$, etc.) are both hydrogen, $R_4$ ($R_4'$, etc.) is in a meta or para position, more preferably the para position. The most preferred monosubstituted phenyl group is 4-fluorophenyl.

Preferably, when both $R_4$ ($R_4'$, $R_4'$, etc.) and $R_5$ ($R_5'$, $R_5''$, etc.) are other than hydrogen and $R_{5a}$ ($R_{5a}'$, etc.) is hydrogen, at least one of $R_4$ (R', etc.) and $R_5$ ($R_5'$, etc.) is in a meta or para position (more preferably both are), and not more than one of them is a member of the group consisting of t-butyl, trifluoromethyl phenoxy and benzyloxy; more preferably, $R_4$ ($R_4'$, etc.) and $R_5$ ($R_5'$, etc.) are not ortho to each other when neither of them is a member of the group consisting of methyl, methoxy, fluoro and chloro.

Preferably, when each of $R_4$ ($R_4'$, etc.), $R_5$ ($R_5'$, etc.) and $R_{5a}$ ($R_{5a}'$, etc.) is other than hydrogen, at least two of them (more preferably all three) are in meta or para positions, and not more than one of them is a member of the group consisting of t-butyl, trifluoromethyl, phenoxy and benzyloxy; more preferably, no two of them are ortho to each other unless at least one member of each of the pair of substituents that are ortho to each other is a member of the group consisting of methyl, methoxy, fluoro and chloro.

$R_6$ is preferably $R_6'$, where $R_6'$ is hydrogen or $C_{1-2}$alkyl, more preferably $R_6''$, where $R_6'$ is hydrogen or methyl, and most preferably hydrogen.

$R_7$ is preferably $R_7'$, where $R_7'$ is hydrogen or $C_{1-3}$alkyl more preferably $R_7'$ is hydrogen or $C_{1-2}$alkyl.

Compounds of formula I wherein Z is of formula II or IIa are most preferably in salt form. Preferred salt-forming cations are those free from centers of asymmetry especially e.g. sodium, potassium or ammonium, most preferably sodium.

X is preferably X', where X' is $-(CH_2)_{m7}-$ or

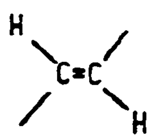 , wherein m is 1, 2 or 3, especially 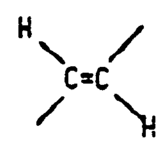 .

Z is preferably a group of formula IIa wherein $R_6$ is $R_6'$ and $R_7$ is $R_7'$ or a group of formula IIb wherein $R_6$ is $R_6'$, more preferably a group of formula IIa wherein $R_6$ is $R_6''$ and $R_7$ is $R_7''$ or agroup of formula IIb wherein $R_6$ is $R_6'$ and most preferably a group of formula IIa wherein $R_6$ is hydrogen and $R_7$ is $R_7''$ or a group of formula IIb wherein $R_6$ is hydrogen, especially a group of formula IIa in sodium salt form wherein $R_6$ is hydrogen or a group of formula IIb wherein $R_6$ is hydrogen.

n is preferably m, where m is 1, 2 or 3, preferably 2 or 3 and most preferably 2.

Insofar as the compounds of Groups IAa and IBa and each of the sub-groups thereof are concerned, the erythro isomers are preferred over the threo isomers, erythro and threo referring to the relative positions of the

hydroxy groups in the 3- and 5-positions (of the groups of formulae II and IIa).

As between otherwise identical compounds of formula I, free acid, salt and ester forms are generally preferred to lactone forms.

The trans lactones are generally preferred over the cis lactones, cis and trans referring to the relative positions of $R_6$ and the hydrogen atom in the 6-position of the group of formula IIb.

The preferred stereoisomers of the compounds of formula I having only two centers of asymmetry wherein X is a direct bond,

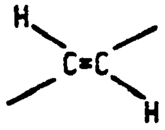

or

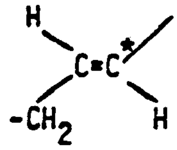

,

wherein the * denotes the bond to the Z group, and Z is in other than lactone form are the 3R,5S and 3R,5R isomers and the racemate of which each is a constituent, i.e., the 3R,5S-3S,5R (erythro) and 3R,5R-3S,5S (threo) racemates, with the 3R,5S isomer and the racemate of which it is a constituent being more preferred and the 3R,5S isomer being most preferred.

The preferred stereoisomers of the compounds of formula I having only two centers of asymmetry wherein X is

$-(CH_2)_m-$ or

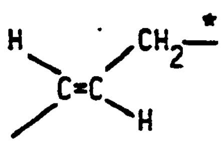

,

and Z is in other than lactone form are the 3R,5R and 3R,5S isomers and the racemate of which each is a constituent, i.e., the 3R,5R-3S.5S (erythro) and 3R,5S-3S,5R (threo) racemates, with the 3R,5R isomer and the racemate of which it is a constituent being more preferred and the 3R,5R isomer being most preferred.

The preferred stereoisomers of the compounds of formula I having only two centers of asymmetry wherein X is a direct bond,

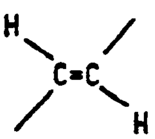

or

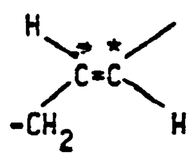

,

wherein the * denotes the bond to the Z group, and Z is a group of Formula IIb are the 4R,6S and 4R,6R isomers and the racemate of which each is a constituent, i.e., the 4R,6S-4S,6R (trans lactone) and 4R,6R-4S,6S (cis lactone) racemates, with the 4R,6S isomer and the racemate of which it is a constituent being more preferred and the 4R,6S isomer being most preferred.

The preferred stereoisomers of the compounds of formula I having only two centers of asymmetry wherein X is $-(CH_2)_m-$ or

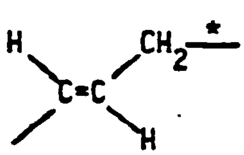

,

, and Z is a group of formula IIb are the 4R,6R and 4R,6S isomers and the racemate of which each is a constituent, i.e., the 4R,6R-4S,6S (trans lactone) and 4R,6S-4S,6R (cis lactone) racemates, with the 4R,6R isomer and the racemate of which it is a constituent being more preferred and the 4R,6R isomer being most preferred.

The preferences set forth in the preceding four paragraphs also apply to the compounds of formula I having more than two centers of asymmetry and represent the preferred configurations of the indicated positions.

Each of the preferences set forth above applies not only to the compounds of formula I, but also to the

compounds of formulae IA and IB and those of Groups IAa, IAb, IBa, IBb, IAal, IAa2, IAa3, IAbl, IAb2, IAb3, IBal, IBa2, IBa3, IBbl, IBb2 and IBb3 as well as to every other subgroup thereof set forth infra, e.g. Groups (i) et seq., unless otherwise indicated. When any preference contains a variable, the preferred significances of that variable apply to the preference in question, unless otherwise indicated.

Preferred groups of compounds of formula I include the compounds (i) of Group IAa1 wherein $R_1$ is $R_1''$, $R_2$ is $R_2''$, $R_3$ is $R_3''$, $R_4$ is $R_4''$, $R_5$ is $R_5'$, $R_{5a}$ is $R_{5a}'$, $R_6$ is $R_6''$, $R_7$ is $R_7'$, and X is X'.

(ii) of (i) wherein when both $R_2''$ and $R_3'$ are other than hydrogen, at least of of them is in the 6- or 7-position, when both $R_4'$ and $R_5'$ are other than hydrogen and $R_{5a}''$ is hydrogen, at least one of $R_4'$ and $R_5''$ is in a meta or para position, and when each of $R_4''$, $R_5''$ and $R_{5a}''$ is other than hydrogen, at least twoof them are in meta or para positions,

(iii)-(iv) of (i) and (ii) wherein $R_6$ is $R_6''$, especially hydrogen. (v)-(vi) of (i) and (ii) wherein $R_1$ is $R_1''$, $R_2$ is $R_2'$, $R_3$is $R_3''$,

$R_4$ is $R_4''$, $R_5$ is $R_5$, $R_{5a}$ is hydrogen, $R_6$ is $R_6$, especially hydrogen, $R_7$ is $R_7''$ and X is

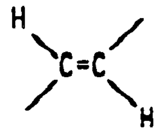 ,

(vii) of (i) wherein $R_1$ is $R_1''$, $R_2$ is hydrogen, $R_3$ is hydrogen, $R_4$ is $R_4''$, $R_5$ is hydrogen, $R_{5a}$ is hydrogen, $R_6$ is hydrogen, $R_7$ is $R_7''$, and X is

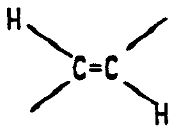 ,

(viii)-(xiii) of (i)-(vi) wherein any salt form is preferably a sodium, potassium or ammonium, especially a sodium salt form,

(xiv) of Group IAb1 wherein $R_1$ is $R_1''$, $R_2$ is $R_2''$, $R_3$ is $R_3'$, $R_4$ is $R_4''$, $R_5$ is $R_5''$, $R_{5a}$ is $R_{5a}''$, $R_6$ is $R_6''$, andX is X',

(xv) of (xiv) wherein when both $R_2'$ and $R_3'$ are other than hydrogen, at least one of them is in the 6- or 7-position, when both $R_4$ and $R_5''$ are other than hydrogen and $R_{5a}''$ is hydrogen, at least one of $R_4'$ and $R_5''$ is in a meta or para position; and when each of $R_4''$, $R_5''$ and $R_{5a}''$ is other than hydrogen, at least two of them are in meta or para positions,

(xvi)-(xvii) of (xiv) and (xv) wherein $R_6$ is $R_6''$, especially hydrogen,

(xviii)-(xix) of (xiv) and (xv) wherein $R_1$ is $R_1'$,$R_2$ is $R_2''$, $R_3$ is $R_3''$, $R_4$ is $R_4''$, $R_5$ is $R_5''$, $R_{5a}$ is hydrogen, $R_6$ is $R_6''$, especially hydrogen, and X is

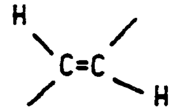 ,

(xx) of (xiv) wherein $R_1$ is $R_1'''$, $R_2$ is hydrogen, $R_3$ is hydrogen, $R_4$ is $R_4'''$, $R_5$ is hydrogen, $R_{5a}$ is hydrogen, $R_6$ is hydrogen, and X is

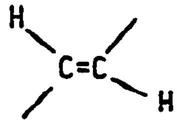 ,

(xxi) of Group IBa1 wherein $R_1$ is $R_1'$, $R_4$ is $R_4'$, $R_5$ is $R_5'$, $R_{5a}$ is $R_{5a}'$ $R_6$ is $R_6'$, $R_7$ is $R_7'$, and X is X',

(xxii) of (xxi) wherein when both $R_4'$ and $R_5'$ are other than hydrogen and $R_{5a}'$ is hydrogen, at least one of $R_4'$ and $R_5'$ is in a meta or para position, and when each of $R_4'$, $R_5'$ and $R_{5a}'$ is otherthan hydrogen, at least two of them are in meta or para positions,

(xxiii)-(xxiv) of (xxi) and (xxii) wherein $R_6$ is $R_6''$, especially hydrogen,

(xxv)-(xxvi) of (xxi) and (xxii) wherein $R_1$ is $R_1''$, $R_4$ is $R_4''$, $R_5$ is $R_5''$, $R_{5a}$ is hydrogen, $R_6$ is R-6'', especially

hydrogen, $R_7$ is $R_7'''$, and X is

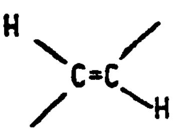

,

(xxvii) of (xxi) wherein $R_1$ is $R_1'$, $R_4$ is $R_4'$, $R_5$ is hydrogen, $R_{5a}$ is hydrogen, $R_6$ is hydrogen, $R_7$ is $R_7''$, and X is

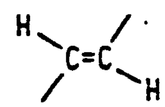

,

(xxviii)-(xxxiii) of (xxi)-(xxvi) wherein any salt form is preferably a sodium, potassium or ammonium, especially a sodium, salt form,

(xxxiv) of Group IBb1 wherein $R_1$ is $R_1'$, $R_4$ is $R_4'$, $R_5$ is $R_5'$, $R_{5a}$ is $R_{5a}'$, $R_6$ is $R_6'$, and X is X',

(xxxv) of (xxxiv) wherein when both $R_4'$ and $R_5'$ are other than hydrogen and $R_{5a}'$ is hydrogen, at least one of $R_4'$ and $R_5'$ is in a <u>meta</u> or <u>para</u> position, and when each of $R_4'$, $R_5'$ and $R_{5a}'$ is other than hydrogen, at least two of them are in <u>meta</u> or <u>para</u> positions,

(xxxvi)-(xxxvii) of (xxxiv) and (xxxv) wherein $R_6$ is $R_6''$, especially hydrogen,

(xxxviii)-(xxxix) of (xxxiv) and (xxxv) wherein $R_1$ is $R_1$, $R_4$ is $R_4$, $R_5$ is $R_5'$, $R_{5a}$ is hydrogen, $R_6$ is $R_6'$, especiallyhydrogen, and X is

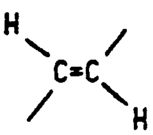

,

(xl) of (xxxiv) wherein $R_1$ is $R_1''''$, $R_4$ is $R_4''''$, $R_5$ is hydrogen, $R_{5a}$ is hydrogen, $R_6$ is hydrogen, and X is

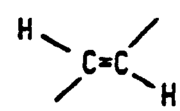

,

(xli)-(lxvi) of (i)-(xiii) and (xxi)-(xxxiii) wherein the hydroxy groups in the 3- and 5-positions (of the group of formula IIa) have the <u>erythro</u> configuration,

(lxvii)-(xcii) the 3R,5S enantiomers of the compounds of (xli)-(lxvi) wherein X is

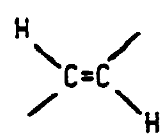

and the 3R,5R enantiomers of the compounds of these groups wherein X is $-(CH_2)_m$

(xciii)-(cvi) of (xiv)-(xx) and (xxxiv)-(xl) wherein the hydroxy group on the lactone ring is <u>trans</u> to X (the <u>trans</u> lactones) and

(cvii)-(cxx) the 4R,6S enantiomers of the compounds of (xciii)(cvi) wherein X is

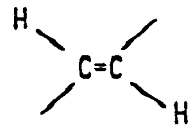

and the 4R,6R enantiomers of the compounds of these groups wherein X is $-(CH_2)_m$

Groups of (xli)-(lxvi) embrace the 3R,5S-3S,5R racemate and the-3R,5S and 3S,5R enantiomers of the compounds wherein X is

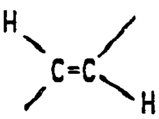

(the 3S,5R enantiomer being least preferred) and the 3R,5R-3S,5S racemate and the 3R,5R and 3S,5S enantiomers of the compounds wherein X is $-(CH_2)_m-$ (the 3S,5S enantiomer being least preferred).

Groups (xciii)-(cvi) embrace the 4R,6S-4S,6R racemate and the 4R,6S and 4S,6R enantiomers of the compounds wherein X is

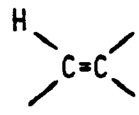

(the 4S,6R enantiomer being least preferred) and the 4R,6R-4S,6S racemate and the 4R,6R and 4S,6S enantiomers of the compounds wherein X is $-(CH_2)_m-$ (the 4S,6S enantiomer being least preferred).

Insofar as Groups IAa2, IAb2, IBa2, IBb2, IAa3, IAb3, IBa3 and IBb3 are concerned, the preferred sub-groups are those that correspond to Groups (i)-(cxx). As should be evident, the preferred groups of compounds of Groups IAa2 and IAa3 are those that correspond to Groups (i)-(xiii), (xli)-(liii) and (lxvii)-(lxxix), the preferred groups of compounds of Groups IAb2 and IAb3 are those that correspond to Groups (xiv)-(xx), (xciii)-(xcix) and (cvii)-(cxiii), the preferred groups of compounds of Groups IBa2 and IBa3 are those that correspond to Groups (xxi)-(xxxiii), (liv)-(lxvi) and (lxxx)-(xcii) and the preferred groups of compounds of Groups IBb2 and IBb3 are those that correspond to Groups (xxxiv)-(xl), (c)-(cvi) and (cxiv)-(cxiv)(cxx). It is as if each of these additional groups were set forth herein in its entirety.

A particular compound group covers those of formula I wherein the two Ro groups together form a radical of the formula

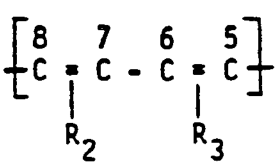 or $-CH_2CH_2CH_2CH_2-$,

wherein $R_2$ is hydrogen, $C_{1-3}$alkyl, n-butyl, i-buty; $C_{1-.3}$alkoxy, n-butoxy, i-butoxy, trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the provisos that not more than one of $R_2$ and $R_3$ is trifluoromethyl, not more than one of $R_2$ and $R_3$ is phenoxy, and not more than one of $R_2$ and $R_3$ is benzyloxy,

$R_1$ is hydrogen, $C_{1-3}$alkyl, fluoro, chloro or benzyloxy,

$R_4$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_{5a}$ is hydrogen,

with the proviso that not more than one of $R_4$ and $R_5$ is trifluoromethyl, not more than one of $R_4$ and $R_5$ is

phenoxy, and not more than one of $R_4$ and $R_5$ is benzyloxy,
X is $-(CH_2)_n-$,

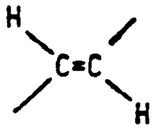, 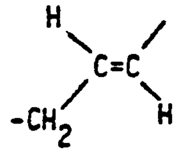 or 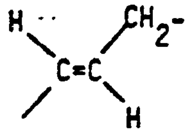,

wherein n is 0, 1, 2 or 3, and
Z is

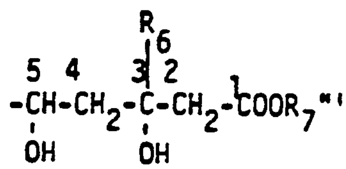 or

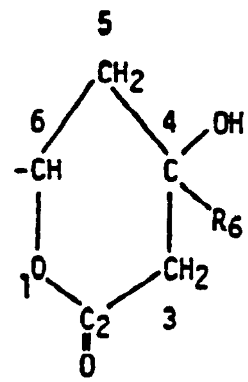 IIb ,

wherein $R_6$ is hydrogen or $C_{1-3}$alkyl, and
$R_7''''$ is hydrogen, $C_{1-3}$alkyl, n-butyl, i-butyl, t-butyl, benzyl or M,
wherein M is a pharmaceutically acceptable cation, with the proviso that the -X-Z group and the $R_4$-bearing phenyl group are ortho to each other.

The compounds of formula I can be prepared by the following methods whereby

BR represents the basic ring structure

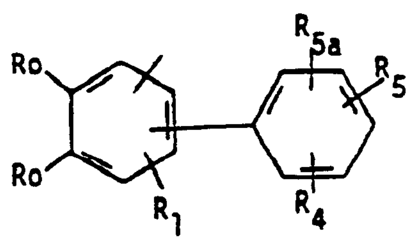

Ro, $R_1$, $R_4$, $R_{5a}$ and $R_5$ being as defined above.

Thus 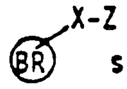 stands for

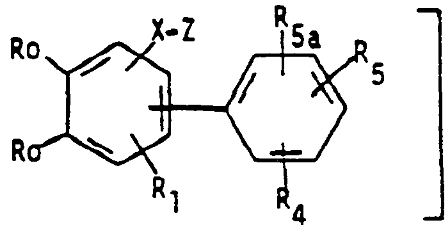

a) When $R_6$ is hydrogen reducing a compound of formula VI

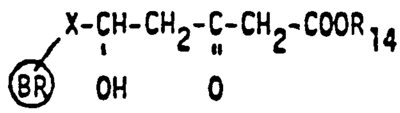

VI

wherein $R_{14}$ is a radical forming a physiologically-hydrolysable and acceptable ester and X is as defined above,

b) when $R_d$ = $C_{1-3}$alkyl, hydrolysing a compound of formula XVII

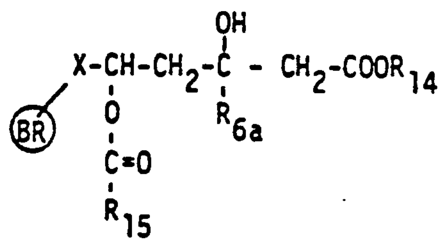

XVII

wherein $R_{6a}$ is $C_{1-3}$alkyl, $R_{15}$ is $C_{1-2}$alkyl and X and $R_{14}$ are as defined above,

c) when X is

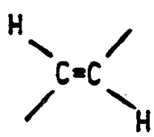

deprotecting a compound of formula LVIII

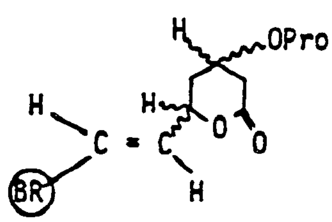

LVIII

wherein Pro is a protecting group

d) hydrolysing a compound of formula I in the form of a physiologicallyhydrolysable ester or a lactone or

e) esterifying or lactonising a compound of formula I in free acid form, and when a free carboxyl group is present, recovering the compound obtained in free acid form or in the form of a salt. In processes a) and b) $R_{14}$ is preferably $C_{1-3}$alkyl, n-butyl, i-butyl, t-butyl or benzyl more preferably $C_{1-3}$alkyl and most preferably $C_{1-2}$alkyl and $R_{15}$ is preferably methyl.

It will readily be appreciated that the various forms of the compounds of formula I may be interconverted as indicated in d) and e) above.

In the same way compounds obtained according to a), b), and c) may be hydrolysed to free acid forms and free acid forms may be esterified or lactonised to produce a desired end-product. The invention thus also provides a process for preparing a compound of formula I which comprises hydrolysing a compound of formula I in ester or lactone form or esterifying or lactonising a compound of formula I in free acid form and when a free carboxyl group is present recovering the compound obtained in free acid form or in the form of a salt.

Unless otherwise stated, reactions are performed in a manner conventional for the type of reaction involved. Molar ratios and reaction times are as a rule conventional and non-critical and are chosen according to principles well established in the art on the basis of reactants and conditions employed.

Solvents, alone or as mixtures, are generally chosen which remain inert and liquid during the reaction in question.

Examples of inert atmospheres are carbon dioxide (some reactions) and more usually nitrogen or a noble gas, nitrogen being preferred. Most reactions, including those wherein use of an inert atmosphere is not mentioned, are carried out under such for convenience.

Reduction according to a) is preferably carried out using a mild reducing agent such as sodium borohydride or a complex of t-butylamine and borane in an inert organic solvent such as a lower alkanol, preferably ethanol, conveniently at a temperature of -10- to 30° C, under an inert atmopshere.

Use of an optically pure starting material will lead to only two optical isomers (diastereoisomers) of the resulting end product. However, if stereospecificity is desired it is preferred to utilize a stereoselective reduction in order to maximize production of a mixture of the erythro stereoisomers (racemate) of which the preferred stereoisomer (as set forth above) is a constituent. Stereoselective reduction is carried out in three steps. For example in the first step, the ketoester of formula VI is treated with a tri(primary or secondary $C_{2-4}$alkyl)borane, preferably tri-n-butylborane, and air to form a complex. The reaction temperature is suitably 0° to 50° C, preferably 20° to 30° C. The first step is carried out in an anhydrous inert organic solvent, preferably an ether solvent such as tetrahydrofuran, diethyl ether, 1,2-dimethoxyethane or 1,2-diethoxyethane, with tetrahydrofuran being the most preferred solvent. In the second step, for example, the complex is reduced with sodium borohydride, preferably in the same solvent as utilized for the first step, at -80° to -40° C, preferably -80° to -70° C. In the third step, the product of the second step is, for example, treated with aqueous e.g. 30% $H_2O_2$, an aqueous buffer, preferably a phosphate buffer, to maintain a pH of 7.0 to 7.2, and a lower alkanol preferably methanol. The $H_2O_2$ is in large molar excess e.g. 50 to 70 moles per mole of VI. The reactants are added slowly to the mixture from step 2 at e.g. -80° to -40° C, preferably -80° to -70° C with subsequent warming to 20° to 30° C.

Hydrolysis according to b) or d) is carried out in a manner conventional for such reactions e.g. employing an inorganic hydroxide such as NaOH or KOH with, if desired, subsequent acidification to give the free acid form. Suitable solvents are mixtures of water and water miscible solvents such as lower alkanols e.g. methanol or ethanol and reaction conveniently takes place at temperatures from 0° C to reflux preferably not more than 80° C. most preferably 20° to 30° C. If it is desired to recover the compound in a salt form corresponding to the cation of the hydroxide employed then slightly less than an equivalent amount of the latter may be employed. In b) $R_{14}$ will conveniently be the same as $R_{15}$ e.g. $C_{1-3}$alkyl, especially $C_{1-2}$alkyl, preferably methyl.

Lactonisation according to e) is carried out in conventional manner e.g. by heating the corresponding acid in an anhydrous inert organic solvent e.g. a hydrocarbon such as benzene, toluene or a xylene or mixtures thereof, preferably at temperatures of 75° C to reflux although more preferably not above 150° C.

As is evident to those in the art, a racemic threo 3,5-di-hydroxycarboxylic acid yields a racemic cis lactone and a racemic erythro 3,5-dihydroxycarboxylic acid yields a racemic trans lactone. Use of a mixture of threo and erythro 3,5-dicarboxylic acid yields a mixture of cis and trans lactones (all four possible diastereoisomers). Likewise if a single enantiomer of the 3,5-dihydroxycarboxylic acid is utilized, a single enantiomer of the lactone is obtained. For example, lactonisation of a 3R,5S erythro dihydroxycarboxylic acid yields a 4R,6S lactone.

Esterification according to e) is conventional employing e.g. a large excess of a compound $R_{14}OH$ wherein $R1_4$ is as defined above at 20° C to 40° C optionally in a solvent (especially when $R_{14}OH$ is not liquid) and in the presence of a catalytic amount of an acid such as p-toluenesulfonic acid. Where methyl esters are required these can also be obtained e.g. using diazomethane in an anhydrous inert ether solvent such as tetrahydrofuran, 1,2-dimethoxyethane or 1,2-diethoxyethane and especially diethylether at e.g. 0° to 30° C preferably 20° to 30° C.

Examples of protecting groups in reaction c) are diphenylt-butylsilyl, tri-isopropylsilyl or dimethyl-t-butylsilyl, $C_{1-6}$n-alkyl, benzyl, triphenylmethyl, tetrahydrofuran-2-yl, tetrahydropyran-2-yl, 4-methoxytetrahydropyran-4-yl, Cl-6n-alkanoyloxy. Especially preferred are trisubstituted silyl radicals in particular diphenyl-t-butylsilyl.

Deprotection is carried out in conventional manner e.g. by cleavage under mild conditions such as employing e.g. for removal of a silyl containing group such as diphenyl-t-butylsilyl a fluoride reagent e.g. tetra-n-butylammonium fluoride in an anhydrous inert organic medium, preferably tetrahydrofuran containing glacial acetic acid at temperagures of 20° to 60° C especially 20° to 30° C. Preferably 1-5 moles of fluoride are used per mole of silyl group with 1 to 1.8 moles of glacial acetic acid to each mole of fluoride.

The required starting materials may be prepared for example as illustrated in the following reaction schemes. The symbols used are defined as follows

R, $R_0$, $R_1$, $R_2$, $R_3$, $R_4$, $R_5$, $R_{5a}$, $R_6$, $R_{6a}$, $R_{11}$, $R_{12}$, X,

(BR) – as defined above,

$M_2$ = a cation, preferably Na or K

$R_{13}$ = $C_{1-6}$alkyl, preferably $C_{1-2}$alkyl

Y = chloro or bromo

Ac = acetyl

ϕ = phenyl

+ = t-butyl

lac = 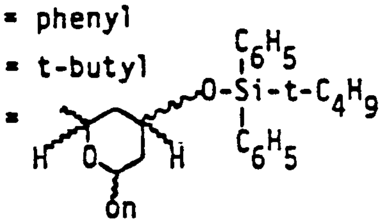

[on = $OCH_3$ (LVI), OH (LVII) or = 0 (LVIII)]

Xa = 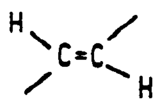 , 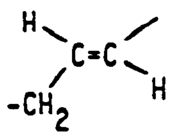 , 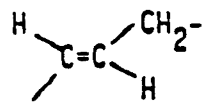

* - these substituents are ortho to each other.

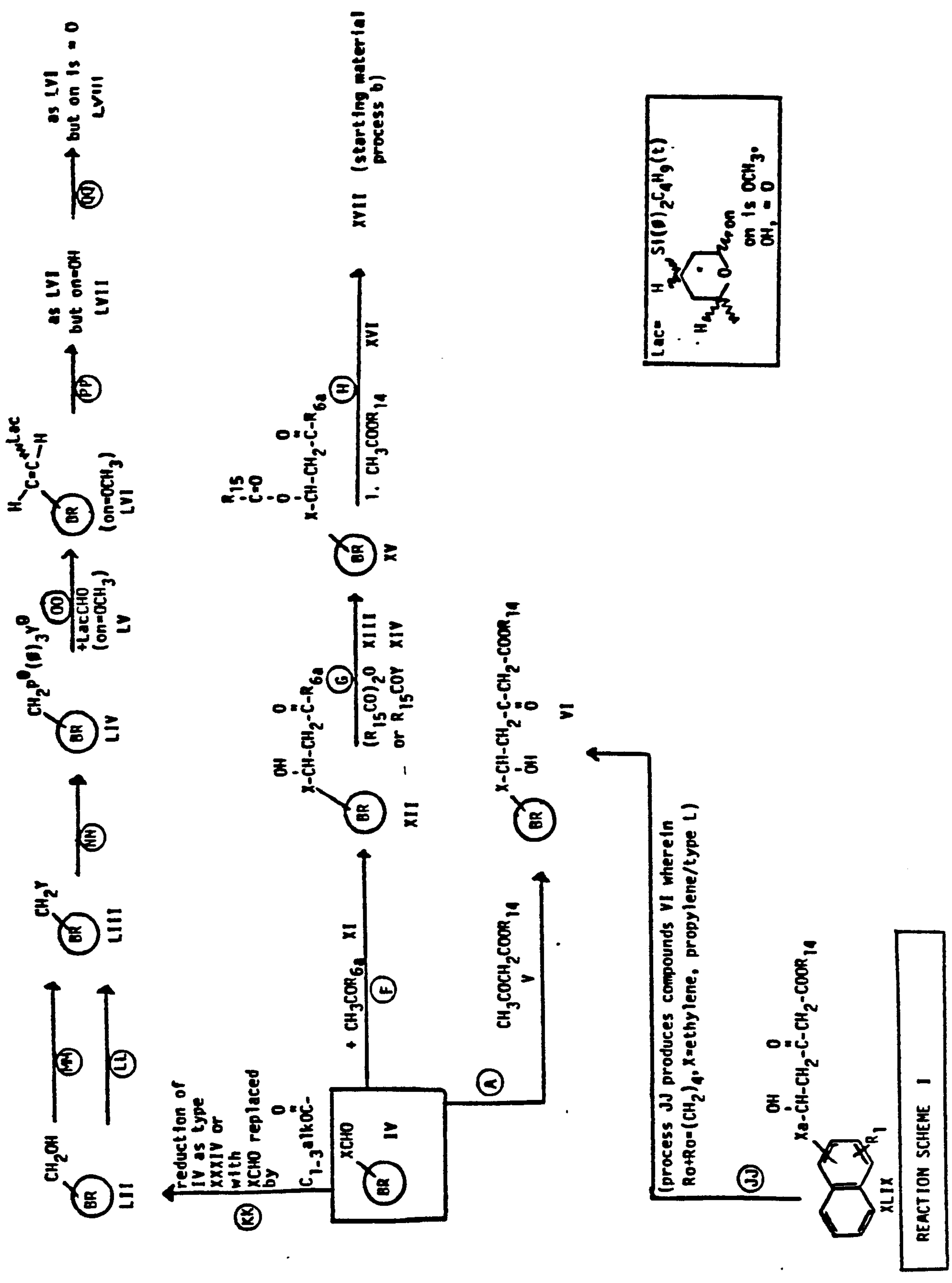
LII
LIII
LIV
(on=$OCH_3$) LVI
as LVII but on=OH LVII
as LVI but on is = O LVIII
+LacCHO (on=$OCH_3$) LV
reduction of IV as type XXXIV or with XCHO replaced by $C_{1-3}$alkOC-
IV
+ $CH_3COR_{6a}$ XI
XII
($R_{15}CO)_2O$ XIII or $R_{15}COY$ XIV
XV
1. $CH_3COOR_{14}$ XVI
XVII (starting material process b)
$CH_3COCH_2COOR_{14}$ V
VI
(process JJ produces compounds VI wherein Ro+Ro=$(CH_2)_4$, X=ethylene, propylene/type L)
XLIX
Lac=
on is $OCH_3$, OH, = O
REACTION SCHEME I

REACTION SCHEME II

Interconversion of compounds IV

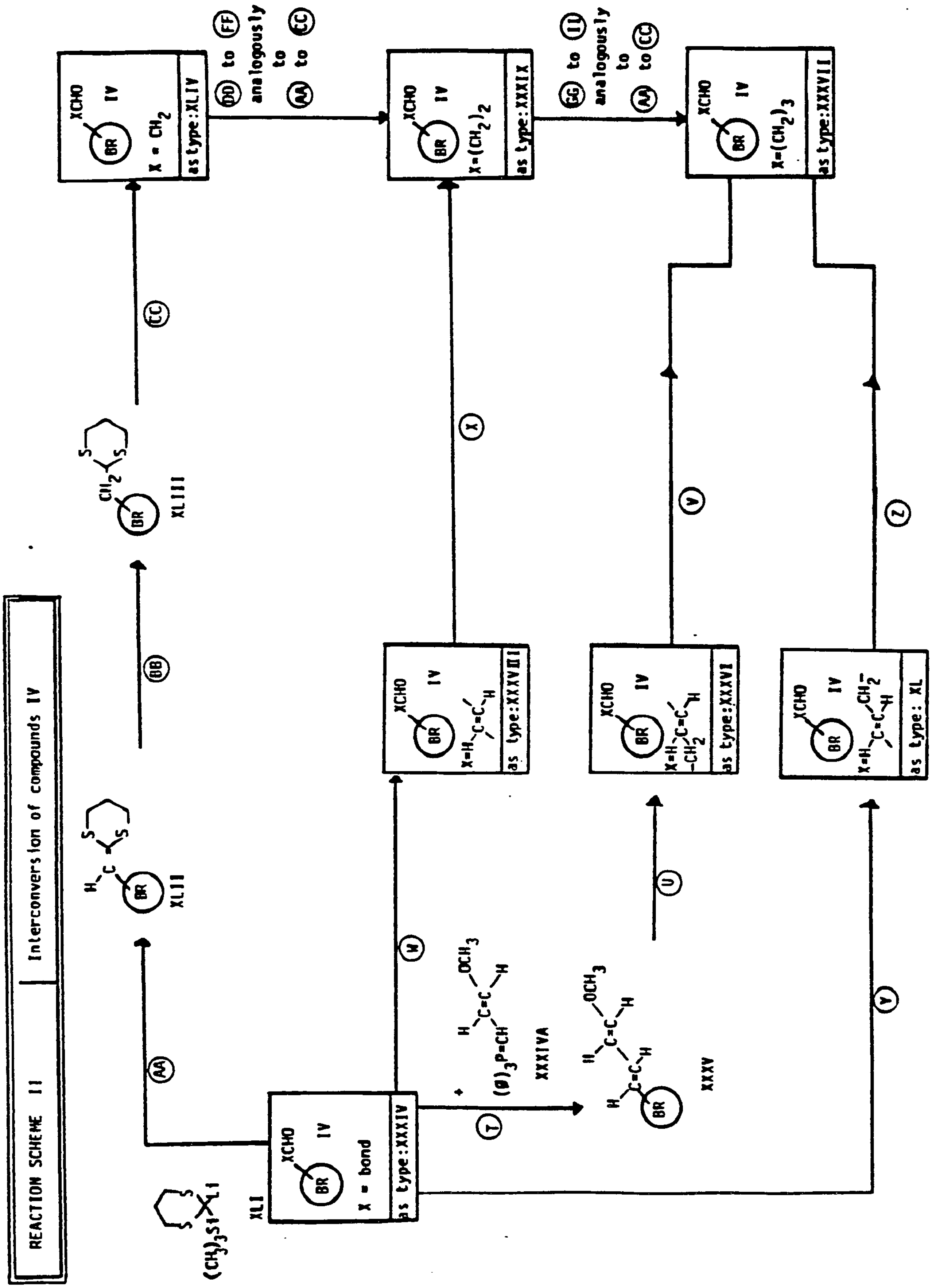

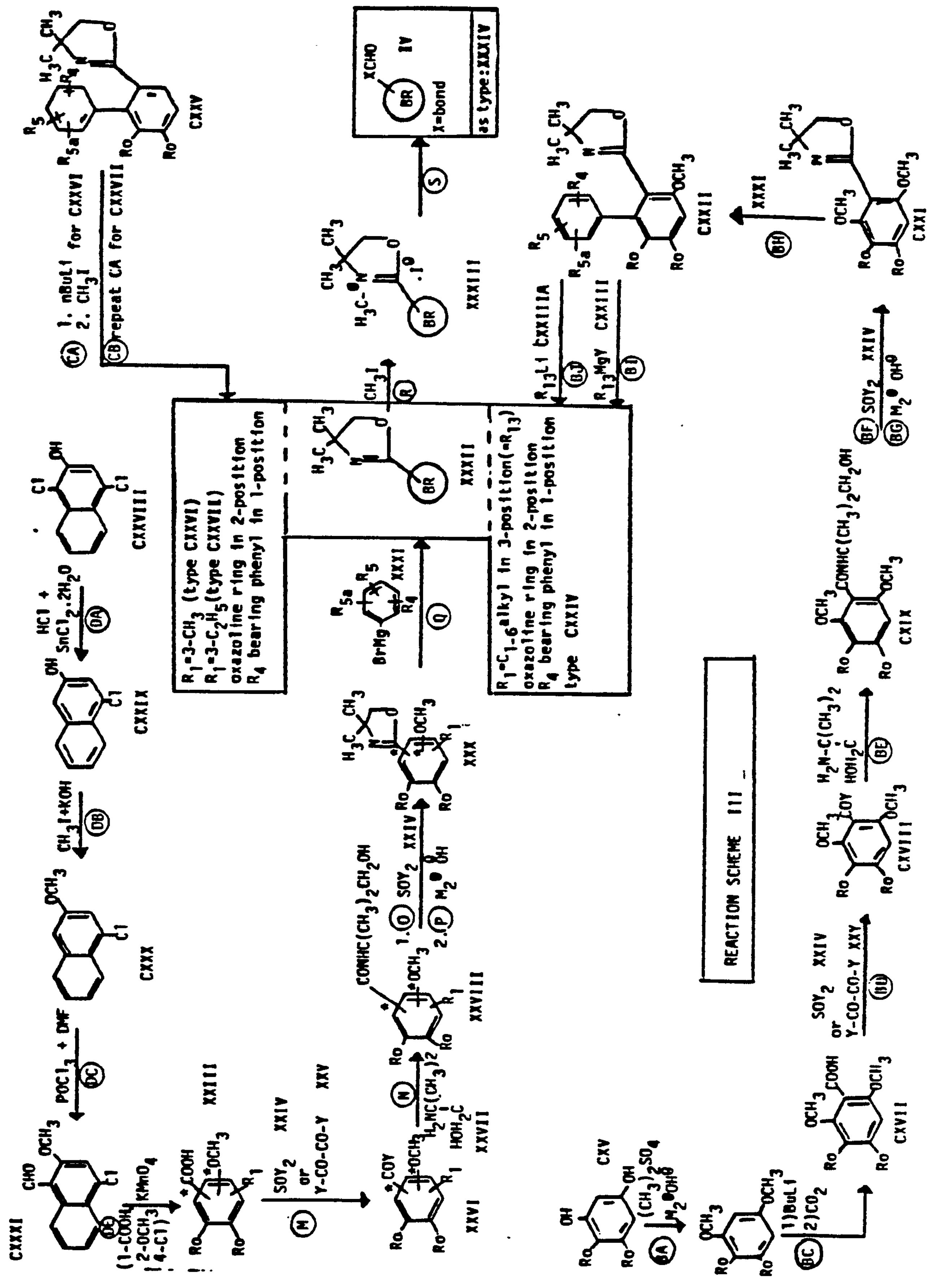
REACTION SCHEME III
CXXV
CA 1. nBuLi for CXXVI
2. $CH_3I$
CB repeat CA for CXXVII
$R_1$=3-$CH_3$ (type CXXVI)
$R_1$=3-$C_2H_5$(type CXXVII)
oxazoline ring in 2-position
$R_4$ bearing phenyl in 1-position
XXII
$CH_3I$ R
XXIII
S
X-bond
as type: XXXIV
IV
$R_1$=$C_{1-6}$alkyl in 3-position(=$R_{13}$)
oxazoline ring in 2-position
$R_4$ bearing phenyl in 1-position
type CXXIV
BJ $R_{13}$Li CXXIIIA
BI $R_{13}$MgY CXXIII
CXXII
BH XXXI
CXXI
Q XXXI
CXXVIII
DA HCl + $SnCl_2.2H_2O$
CXXIX
DB $CH_3I$+KOH
CXXX
DC $POCl_3$ + DMF
CXXXI
DD KMnO$_4$ (1-COOH, 2-OCH$_3$, 4-Cl)
XXIII
M $SOY_2$ XXIV or Y-CO-CO-Y XXV
XXVI
N XXVII
XXVIII
1. O $SOY_2$ XXIV
2. P $M_2^{\oplus}$ OH
XXX
CXV
BA $(CH_3)_2SO_4$ $M_2^{\oplus}OH^{\ominus}$
BC 1)BuLi 2)$CO_2$
CXVII
BD $SOY_2$ XXIV or Y-CO-CO-Y XXV
CXVIII
BE
CXIX
BF $SOY_2$ XXIV
BG $M_2^{\oplus}$ $OH^{\ominus}$

**Reaction Scheme IV**

Two isomers of the compound of formula LV may be synthesized by the following series of reactions:

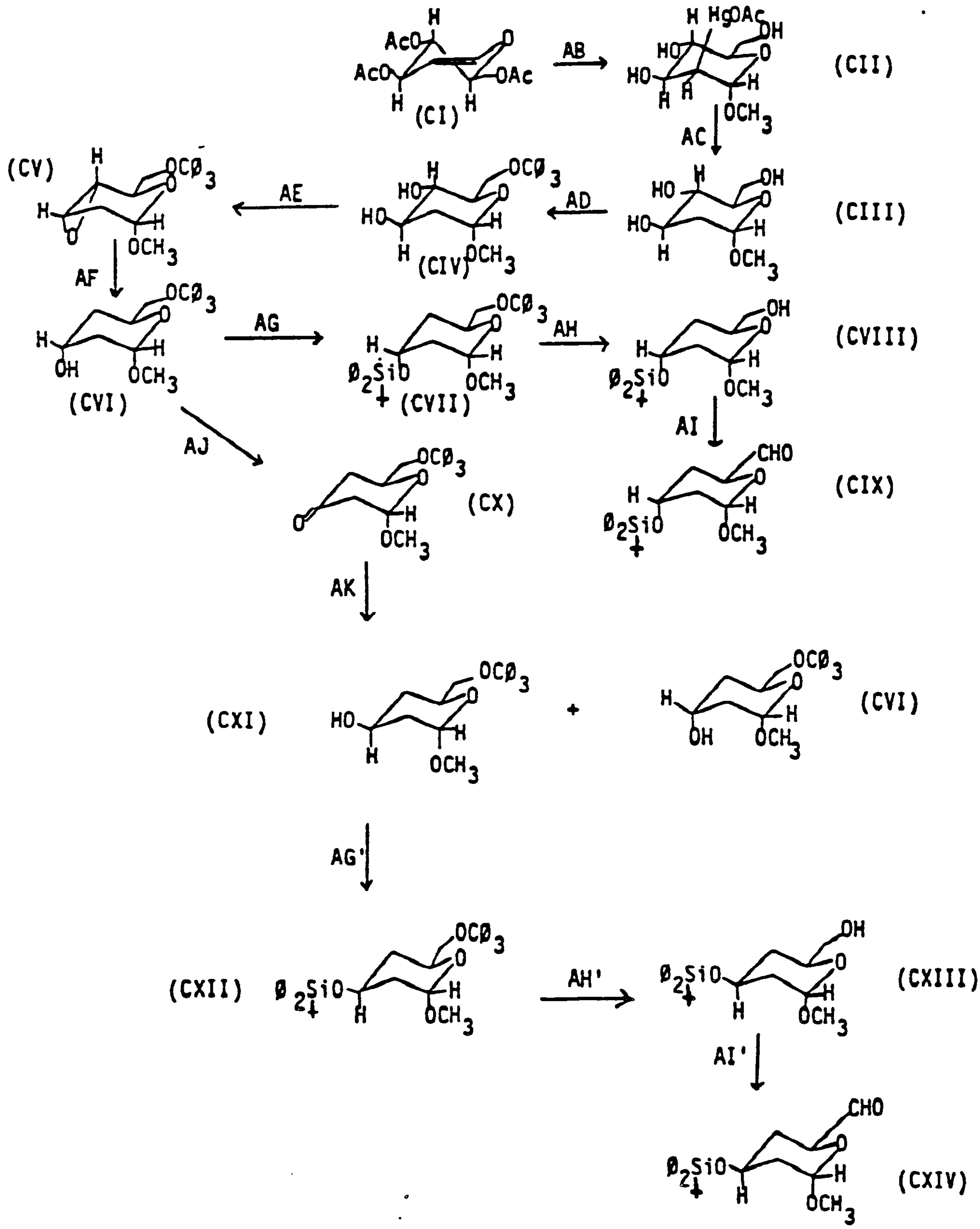


(CI) (CII) (CIII) (CIV) (CV) (CVI) (CVII) (CVIII) (CIX) (CX) (CXI) + (CVI) (CXII) (CXIII) (CXIV)

Unless otherwise stated reactions are performed in a manner conventional for the type of reaction involved. Mol ratios and reaction times are as a rule conventional and non-critical and are chosen according to principles well established in the art on the basis of reactants and conditions employed.

Solvents, alone or as mixtures, are generally chosen which remain inert and liquid during the reaction in question.

Examples of inert atmospheres are carbon dioxide (some reactions) and more usually nitrogen or a noble gas, nitrogen being preferred. Most reactions, including those wherein use of an inert atmosphere is not mentioned, are carried out under such for convenince.

The following tables give examples of typical reaction conditions. In the reaction schemes temperatures are in degrees centigrade.

**Abbreviations**

THF (tetrahydrofuran)
DMF (dimethylformamide)
LDA (lithium diisopropylamide)
DEA (diethylacetamide)
BuLi (n-butyllithium)
TsOH (p-toluenesulfonic acid)
NCS (N-chlorosuccinimide)
NBS (N-bromosuccinimide)
DIBAH (diisobutylaluminium hydride)
DMSO (dimethylsulfoxide)

| Reaction | Type/Steps | Special conditions/ Reagents | Temperatures | Atmosphere | Solvents |
|---|---|---|---|---|---|
| (A) | 1. V + strong base<br>2. add IV | 1. Strong base e.g. n-butyl lithium, lithium diisopropyl amide (LDA) and NaH (latter for generation of mono-anion only). Pref. LDA (generated in situ cf. Examples) | 1. -50° to 10° esp. -5° to 5°<br>2. -80° to -20° esp. -50° to -20° pref. -40° to -30° | inert | Anhydrous e.g. THF, diethylether, 1,2-dimethoxy ethane, 1,2-diethoxy ethane<br>Preferred THF |
| (F) | 1. XI + strong base<br>2. add IV | 1. Strong base e.g. LDA<br>2. Mol ratio XI to IV esp. 3:1 | -80° to -40° | inert | as (A) |
| (G) | Esterification | base e.g. pyridine, triethylamine | 20° to 50° | | excess pyridine |
| (H) | 1. XVI + strong base<br>2. add XV | 1. Strong base e.g. LDA<br>2. Mol ratio XVI to XV esp. 3:1 | 1. -80° to 0°<br>2. -80° to -40° esp. -80° to -70° | inert | as (A) pref. THF |
| (M) | Halogenation | XXIV or XXV pref. in excess | 50° to reflux pref. < 150° | | Anhydrous hydrocarbon e.g. benzene toluene, xylene or mixture thereof |
| (N) | Amidation | XXVII pref. in excess | 20° to 50° esp. 20° to 30° | | Anhydrous e.g. halo carbon esp. $CH_2Cl_2$ |
| (O) | Cyclisation | XXIV pref. $SOCl_2$ in excess | 0° to 75° esp. 20° to 30° | | neat or more usually as (A) or $CH_2Cl_2$, $CHCl_3$ or $CCl_4$ esp. $CH_2Cl_2$ |

| Reaction | Type/Steps | Special conditions/ Reagents | Temperatures | Atmosphere | Solvents |
| --- | --- | --- | --- | --- | --- |
| (P) | Liberation | as hydroxide e.g. NaOH, KOH pref. in excess. Can be run directly from crude hydrohalide of (O) | 0° to 40° esp. 20° to 30° | | $H_2O$ or $H_2O$ + e.g. $CH_3OH$ or $C_2H_5OH$ |
| (Q) | Grignard | | 10° to reflux esp. < 75° pref. 20° to 50° | inert | as (A) pref. THF |
| (R) | N-methylation | $CH_3I$ pref. in large excess. | 20° to reflux esp. < 100° pref. 55° to 90° | | Anhydrous polar e.g THF, 1,2-dimethoxy-ethane, 1,2-dieth-oxyethane, DMA, DEA pref. nitromethane |
| (S) | Ring cleavage<br>1. Mild reducing agent<br>2. $H^⊕$ | 1) $NaBH_4$ (esp. 1-1.1 mole), $LiBH_4$ (1.8-2.1 mole),<br>2) dil. aq. acid e.g. 2N HCl pref. in large excess | 1) -40° to 10°C esp. 0° to 10° ($NaBH_4$) -40° to -20° ($LiBH_4$)<br>2) 0° to 100° esp. 20° to 100° | | 1) Anhydrous alkanol e.g. abs. $C_2H_5OH$ + optionally ether e.g. THF<br>2) e.g. as 1) or just aqueous acid |
| (T) | Wittig | XXXIVA pref. generated from 3-methoxypropen-2-yl-tri-phenylphosphonium bromide + e.g. nBuLi | 2) -80° to -30° esp. -60° to -40° with warming to 20° to 30° | inert | as (A) pref. THF |
| (U) | ether-cleavage $H^⊕$ | dil. acid e.g. 1N HCl in excess on crude solution from (T) | reflux | | as (T) |

| Reaction | Type/Steps | Special conditions/ Reagents | Temperatures | Atmosphere | Solvents |
|---|---|---|---|---|---|
| (V) (X) | Hydrogenation | excess $H_2$ under pressure + Pd/C(pref. 5%) + $FeCl_2.4H_2O$ Termination after 1 mole $H_2$ taken up | 20° to 30°C esp. 25° | | 1. alkanol e.g. $CH_3OH$ |
| (W) | 1. cis-1-Li-2-ethoxyethylene* 2) pTsOH<br>*prepared e.g. as described in the examples. | 1) pref.isolate prior to 2)<br>2) pTsOH in catalytic amounts. | 1) -80° to -40° esp. -80° to -70°<br>2) 20° to 40° esp. 20°-30° | inert | anhydrous THF<br>2. $H_2O$ + THF |
| (Y) | 1. trans-2-methoxy cyclopropyl lithium<br>2. Methanesulfonyl-chloride<br>3. Hydrolysis | 1)<br>2. tertiaryamine e.g. $N(C_2H_5)_3$<br>3. e.g. ammonium hydrogen oxalate + oxalic acid (pref. 2 moles of each) | 1) -80° to 25° (esp. start -78° and warm to 25°)<br>2) -60° to -20° esp. -40°<br>3) -50° to -30° esp. -40° | inert | 1. Anhydrous e.g. THF, 1,2-dimethoxyethane, 1,2-diethoxyethane or pref. diethyl ether<br>2. Anhydrous e.g. halocarbon e.g. $CH_2Cl_2$<br>3. e.g. excess acetone and water |
| (Z) | Hydrogenation | excess $H_2$ under pressure + Pd/C (pref. 5%)<br>Termination as V | 20°-30° | | Anhydrous 1. alkanol e.g. ethanol |

| Reaction | Type/Steps | Special conditions/ Reagents | Temperatures | Atmosphere | Solvents |
|---|---|---|---|---|---|
| (AA) | 2-Li-2-trimethylsilyl-1,3-dithiane* | *Prepared from 2-trimethylsilyl-1,3-dithiane and n-BuLi/n-hexane | -10° to 30° | inert | as (A) esp. THF |
| (BB) | | anhydrous $CF_3COOH$ + $(C_2H_5)_3SiH$ | 0° to 50° esp. 20° to 30° | | as (N) |
| (CC) | | N-chlorosuccinimide ~~(of)~~ (NBS) or pref. N-bromosuccinimide (NCS) in excess | 0° to 50° esp. 20° to 30° | | $H_2O$ + acetonitrile |
| (JJ) | Hydrogenation | excess $H_2$ under pressure + $PtO_2$ until 3 moles taken up | 20° to 30° | | glacial $CH_3COOH$ |
| (KK) | Reduction | a) $NaBH_4$ (type XXXIV) pref. 2-4 moles; b) $LiAlH_4$; DIBAH (alkoxycarbonyl type) at least 2 equivalents | a) 0° to 30° esp. 20° to 30° b) -80° to reflux pref. < 70° esp. -80° to 25° | inert | a) ℓ. alkanol e.g. $CH_3OH$, $C_2H_5OH$ b) as (A) pref. THF |
| (LL) | Halogenation | phosphorous trihalide, thionyl halide (halide = Y) e.g. $PCl_3$ or $PBr_3$ or $SOCl_2$ or $SOBr_2$ | 20° to 35° | | diethylether or THF (for $PY_3$) or halocarbon e.g. $CH_2Cl_2$ (for $SOY_2$) |

| Reaction | Type/Steps | Special conditions/ Reagents | Temperatures | Atmosphere | Solvents |
|---|---|---|---|---|---|
| (MM) | Halogenation | triphenylphosphine $+CY_4$ (e.g. $CCl_4$ or $CBr_4$) | -10° to reflux pref. < 120° | | neat, excess $CY_4$, diethylether, THF, benzene, toluene |
| (NN) | | triphenylphosphine | 60° to reflux pref. < 150° | inert | as (M) |
| (OO) | Wittig<br>1. strong base<br>2. LV | 1. e.g. NaH, nBuLi (esp. nBuLi) | 1. -10° to 5°<br>2. -60° to 80° esp. -55° to 25° (pref. -55° to -50° rising to 20° to 25°) | inert | e.g. THF, benzene, toluene |
| (PP) | Hydrolysis | e.g. 10% HCl in THF or pref. $CH_3COOH$, $H_2O$, THF (e.g. 3:2:4) | 10° to 100° esp. 20° to 30° (HCl); 60° to 65° ($CH_3COOH$) | | e.g. THF + $H_2O$ |
| (QQ) | oxidation | a) mild conditions e.g. pyridinium chlorochromate pref. in excess, or<br>b) Swern's oxidation oxalylchloride, DMSO, triethylamine | a) 10° to 80° esp. 20° to 30°<br>b) -60° to -40° esp. -50° | | Halocarbon e.g. $CH_2Cl_2$ |
| (BA) | | pref. hydroxide is NaOH | 0°-30° (start 0° to 10° rising to 20°-30°) | | water plus alkanol e.g. ethanol |

| Reaction | Type/Steps | Special conditions/ Reagents | Temperatures | Atmosphere | Solvents |
|---|---|---|---|---|---|
| (BC) | Two step carboxylation | 1. Strong base e.g. n.BuLi<br>2. $CO_2$ pref. in excess | 1. 0°-25° (start 0° to 5° rising to 20°-25°)<br>2. 10°-25°, pref. 20°-25° | | as (A) pref. diethylether |
| (BD) to (BG) | as (M) to (P) | as (M) to (P) | as (M) to (P) | | as (M) to (P) |
| (BH) | Grignard | | 10° to 40° esp. 20° to 30° (low temp. maximises selective displacement of $OCH_3$ <u>ortho</u> to Ro | inert | as (Q) |
| (BI) | Grignard — pref. when $R_{13}$ is primary or secondary | Pref. 6-12 moles esp. 6 moles of CXXIII per mole of CXXII | 60° to 90° pref. 70-80° | inert | pref. anhydrous THF + toluene |
| (BJ) | Alkylation [pref. when $R_{13}$ is tertiary] | Pref. large molar excess of CXXIIIA, esp. 4.4 moles (1.1 at beginning, 2.2 after 3 hrs., 1.1 after further 2 hrs.) | -80° to -20° pref. -50° to -40° | inert | as (A) esp. THF |
| (CA) | Two step methylation | 1. strong base e.g. n-BuLi<br>2. $CH_3I$ | 1. 0°-25° pref. 20°-25°<br>2. 0°-30° pref. commence 0° rise to 20°-25° | inert<br>" | 1. as A esp. diethylether<br>2. " |

| Reaction | Type/Steps | Special conditions/ Reagents | Temperatures | Atmosphere | Solvents |
|---|---|---|---|---|---|
| (CB) | as (CA) | as (CA) | 1. -10° to 0° pref. -50<br>2. as (CA) | as (CA) | as (CA) |
| (DA)-(DC), (DE) | see examples | | | | |
| | | | | | |
| | | | | | |
| | | | | | |
| | | | | | |

CI is the commercially available compound tri- -acetyl-D-glucal.

The preferred reactions conditions for Reactions AB-AI are:

AB: (1) sodium, methanol, 20°C, 15 minutes; (2) mercuric acetate, 25°C.

AC: sodium chloride, sodium borohydride, methanol + isopropanol, 20°C.

AD: triphenylmethyl chloride, pyridine, 35°C.

AE: (1) sodium hydride, tetrahydrofuran, 20°C, (2) 1-(2',4',6'-triisopropylbenzenesulfonyl)imidazole, -30° rising to +20°C.

AF: lithium aluminium hydride, methyl t-butyl ether, -10°C.

AG: t-butyldiphenylchlorosilane, imidazole, N,N,dimethylformamide, 20°C

AH: 70% aqueous trifluoroacetic acid, methylene chloride, commence at -80°-to -50°C, especially -55°C rising over 1 hour to -10° to +10° and keeping at latter for 3-5 hours Epimerisation can be minimized by employing low temperatures and/or short times and terminating the reaction before completion.

AI: pyridinium chlorochromate or especially chromium trioxide (e.g. as Collins oxidation) in molar excess (e.g. 8 mole per mole of CVIII)/pyridine, pyridine, methylene chloride, 20°-25°C.

AJ: oxidation cf. AI.

AK: reduction cf. a) and (S)
above especially $NaBH_4$.

Resulting compounds may be conventionally separated (e.g. HPLC or column chromatography) or directly further reacted.

The compounds of formulae V, XI, XIII, XIV, XVI, XX, XXIII, XXV, XXVII, XXXI, XXXIVA, XLI, CI, CXV, CXXIII, CXXIIIA and CXXVIII and the reagents not designated by a Roman numeral are known or, if unknown, may be synthesized by processes analogous to those described in the literature for similar known compounds. As for the compound of formula LV, one isomer is disclosed in Yang et al., Tetrahedron Letters 23, 4305-4308 (1982) and the synthesis of two other isomers is disclosed in Reaction Scheme IV.

The isomer of Yang et al. and one isomer disclosed in Reaction Scheme IV yield lactones having the 4R,6S configuration. Lactones having the 4S,6S configuration may be obtained from the other isomer whose synthesis is disclosed in Reaction Scheme IV.

The availability of these intermediates enables synthesis of optically pure end products.

Reaction products both intermediate and final can be isolated and purified in conventional manner whereby intermediates can where appropriate be employed directly in a subsequent reaction.

Mixtures of stereoisomers (cis, trans and optical) may be separated by conventional means at whatever stage of synthesis is appropriate. Such methods include re-crystalisation, chromatography, formation of esters with optically pure acids and alcohols or of amides and salts (cf also Sommer et al. J.A.C. S. 80, 3271 (1958)) with subsequent reconversion under retention of optical purity. For example diastereoisomeric (-)-α-naphthylphenylmethylsilyl derivatives of a lactone type end product of formula I may be separated by conventional means.

Salts may be prepared in conventional manner from free acids, lactones and esters and vice-versa. Whilst all salts are covered by the invention, pharmaceutically acceptable salts especially sodium, potassium and ammonium, particularly sodium, salts are preferred.

The various forms of the compounds of formula I are by virtue of their interconvertability useful as intermediates in addition to the use set out below.

Also within the scope of this invention are the intermediates of formulae VI, XII, XV, XVII, XXVIII-XXX, XXXII, XXXIII, XXXV-XL, XLII-L, LII-LIV, LVI-LVIII, CXIX-CXXII, CXXIV-CXXVII, CXXXI and CXXXII. The preferences for each variable are the same as those set forth for the compounds of formula I, with the preferred groups of such compounds including those that correspond to Groups (i)-(xiii), (xxi)-(xxxiii) and (xli-xcii) (for formulae VI, XII, XV, XVII, XXVIII-XXX, XXXII, XXXIII, XXXV-XL, XLII-L, LII-LIV, CXIX-CXXII and CXXIV-CXXVII) and Groups (xiv)-(xx), (xxxiv)-(xl) and (xciii)-(cxx) (for formulae LVI-LVIII), to the extent consistent therewith, and the corresponding groups for the compounds of Groups IAa2, IAb2, IBa2, IBb2, IAa3, IAb3, IBa3 and IBb3.

The compounds of formula I possess pharmacological activity in particular they are inhibitors of 3-hydroxy-3-methyl-glutaryl coenzyme A (HMG-CoA) reductase and as a consequence inhibitors of cholesterol biosynthesis as demonstrated in the following three tests.

Test A: In Vitro Microsomal Assay of HMG-CoA Reductase Inhibition:

200 ul. aliquots (1.08-1.50 mg./ml.) of rat liver microsomal suspensions, freshly prepard from male Spargue-Dawley rats (150-225 g. body weight), in Buffer A with 10 mmol. dithiothreitol are incubated with 10 ul. test substance dissolved in dimethylacetamide and assayed for HMG-CoA reductase activity as described by Ackerman et al., J. Lipid Res. 18, 408-413 (1977). In the assay the microsomes are the source of the HMG-CoA reductase enzyme which catalyses the reduction of HMG-CoA to mevalonate. The assay employs a chloroform extraction to separate the product, [$^{14}C$]mevalonolactone, formed by the HMG-CoA reductase reaction from the substrate, [$^{14}C$]HMG-CoA. [$^{3}H$]mevalonolactone is added as an internal reference.

Inhibition of HMG-CoA reductase is calculated from the decrease in specific activity [$^{14}C/^{3}H$]mevalonate) of test groups compared to controls.

Test B: In Vitro Cell Culture Cholesterol Biosynthesis Screen:

The cell culture is prepared as follows: Stock monolayer cultures of the Fu5AH rat hepatoma cell line (originally obtained from G. Rothblat; see Rothblat, Lipids 9, 526-535 (1974) are routinely maintained in Eagle's Minimum Essential Medium (EMEM) supplemented with 10% fetal bovine serum (FBS) in 75 $cm^2$ tissue culture flasks. For these studies, when the cultures reach confluence, they are removed by mild enzymatic treatment with 0.25% trypsin in Hanks' balanced salt solution (without calcium and magnesium). After centrifugation of the cell suspension and aspiration of the enzymatic solution, the cell pellet is resuspended in an appropriate volume of media for seeding into 60 mm. tissue culture dishes. The cultures are incubated at 37°C in an atmosphere of high humidity and 5% carbon dioxide. When the cultures are confluent (approximately 5 days), they are ready for use. The culture media is aspirated from the dishes and replaced with 3 ml of EMEM suplemented with 5 mg/ml of dilipidized serum protein (DLSP) prepared by the method of Rothblat et al., In Vitro 12, 554-557 (1976). Replacement of the FBS with DLSP has been shown to stimulate the incorporation of [$^{14}$C]acetate into sterol by removing the exogenous sterol supplied by the FBS, thereby requiring the cells to synthesize sterol. Enthanced 3-hydroxy-3-methylglutaryl Coenzyme A reductase (HMG-CoA reductase) activity is measurable in the cells in response to the lack of exogenous sterol. Following approximately 24 hours incubation at 37°C in the DLSP supplemented media, the assay is initiated by the addition of 3μCi of [$^{14}$C]acetate and the test substances solubilized in dimethylsulfoxide (DMSO) or distilled water. Solvent controls and compactin-treated controls are always prepared. Triplicate 60mm. tissue culture dishes are run for each group. After 3 hours incubation at 37°C, the cultures are examined microscopically using an inverted phase contrast microscope. Notations are made of any morphological changes which may have occurred in the cultures. The media is aspirated and the cell layer is gently washed twice with 0.9% sodium chloride solution (saline). The cell layer is then harvested in 3 ml. of 0.9% saline by gentle scraping with a rubber policeman and transferred to a clean glass tube with Teflon lined cap. The dishes are rinsed with 3 ml. of 0.9% saline and rescraped, and the cells are combined with the first harvest. The tubes are centrifuged at 1500 r.p.m. for 10 minutes in an IEC PR-J centrifuge, and the supernatant is aspirated.

The cells are then extracted as follows: One ml. of 100% ethanol is added to the cell pellet followed by sonication for 10 seconds with a "LO" setting of 50 on a Bronwell Biosonik IV. One hundred μl. are taken for protein determination. One ml. of 15% potassium hydroxide (KOH) is added, and the samples are thoroughly vortexed. Saponification is accomplished by heating the ethanol-KOH treated samples at 60°C for 60 minutes in a water bath. Following dilution of the samples with 2ml. of distilled water, they are extracted three times with 7 ml. of petroleum ether. The petroleum ether extracts are then washed three times with 2 ml. of distilled water and finally taken to dryness under a stream of nitrogen.

The obtained samples are then analyzed by thin layer chromatography (TLC) as follows: Residues from the petroleum ether extraction are taken up in a small volume of hexane and spotted on silica gel 60 TLC plates (E. Merck). Development of the plates is carried out in a 150 parts by volume hexane: 50 parts by volume diethyl ether: 5 parts by volume galcial acetic acid solvent system using a three phase development procedure. Visualization is accomplished in an iodine vapor chamber. The plates are divided into five sections such that each section contains the molecules having the following approximate Rf values: section 1- 0-0.4, section 2- 0.4-0.55, section 3-0.55-0.7, section 4- 0.7-0.9 and section 5- 0.9-1.0. Section 2 contains the non-saponifiable sterols. The five sections of the TLC plates are scraped into scintillation vials. Blanks are also prepared from scrapings of chromatographed non-labelled standards. ACS® scintillation cocktail is added, and the radioactivity is determined in a liquid scintillation spectrometer. [$^{14}$C]hexadecane standards are used to determine counting efficiencies. The total protein content of the samples is determined employing the Bio-Rad Protein Assay System.

The results are reported as disintegrations per minute per mg protein (d.p.m./mg protein) for each of the live TLC sections. Mean d.p.m./mg protein ± standard error of the mean are compared for percentage change (%Δ) and statistical significance with solvent control means. TLC section 2 data is taken as a measure of HMG-CoA reductase activity inhibition.

Test C: In Vivo Cholesterol Biosynthesis Inhibition Tests:

In vivo studies utilize male Wistar Royal Hart rats weighing 150 ± 20 g which have been kept for 7-10 days on an altered light cycle (6:30 a.m. - 6:30 p.m. dark) house; two per cage and fed powdered Purina Rat Chow and water ad libitum. Three hours before the diurnal maximum of cholesterol synthesis at mid-dark, the rats are administered the test substances dissolved or as a suspension in 0.5% carboxymethylcellulose in a volume of 1 ml/100 g body weight. Controls receive vehicle alone. One hour after receiving the test substance, the rats are injected intraperitoneally with about 25 μCi/100 g body weight of sodium [1-$^{14}$C]acetate 1-3 mCi/mmol. Two hours after mid-dark, blood samples are obtained under sodium hexobarbitol anesthesia and the serum separated by centrifugation.

Serum samples are saponified and neutralized, and the 3β-hydroxy sterols are precipiated with digitonin basically as described by Sperry et al., J. Biol. Chem. 187, 97 (1950). The [$^{14}$C]digitonides are then counted by liquid scintillation spectrometry. After correcting for efficiencies, the results are calculated in nCi (nanocuries) of sterol formed per 100 ml of serum. Inhibition of sterol synthesis is calculated from the reduction in the nCi of sterols formed from test groups compared to controls.

The compounds are thus indicated for use as hypolipoproteinemic and anti-atherosclerotic agents.

An indicated suitable daily dosage for use in the treatment of pyperlipoproteinemia and athersclerosis is from about 4 to 2000 mg suitably 4-200, e.g. 10 to 100 mg for the more active compounds suitably administered in divided dosages of 1 to 1000 mg, suitably 2.5 to 50 mg, two to four times daily or in retard form.

They may be administered in free acid form or in the form of a physiologically-hydrolysable and -acceptable ester or a lactone thereof or in pharmaceutically acceptable salt form.

The invention therefore also concerns a method of treating hyperliproproteinemia or atherosclerosis by administration of a compound of formula I in free acid form or in the form of a physiologically-hydrolysable and -acceptable ester or a lactone thereof or in pharmaceutically acceptable salt form and such compounds for use as pharmaceuticals e.g. as hypolipoproteinemic and anti-atherosclerotic agents.

The compounds may be administered alone, or in admixture with a pharmaceutically acceptable diluent or carrier, and, optionally other excipients, and administered orally in such forms as tablets, elixirs, capsules or suspensions or parenterally in such forms as injectable solutions or suspensions.

The preferred pharmaceutical compositions from the standpoint of ease of preparation and administration are solid compositions, particularly tablets and hard-filled or liquidfilled capsules.

Such compositions also form part of the invention. The following examples, in which all temperatures are in °C illustrate the invention.

**Example 1**

**Ethyl erythro-(E)-3,5-dihydroxy-7-(2''-[4''-fluorophenyl]naphth-1'-yl)hept-6-enoate (compound no. 1)**

**Step 1: 2-Methoxy-1-naphthoyl chloride (Reaction M; compound XXVIa)**

6.06 g of 2-methoxy-1-naphthoic acid and 7.62 ml of oxalyl chloride are added to 50 ml of anhydrous toluene, and the obtained reaction mixture is refluxed for 2 hours and evaporated to dryness at reduced pressure to obtain the crude product.

**Step 2: 2-Methoxy-1-naphthoic acid N-1,1-dimethyl-2-hydroxyethylamide (Reaction N; compound XXVIIIa)**

50 ml of methylene chloride (dried over molecular sieves) and 5.4 g of 2-amino-2-methyl-1-propanol are added to the crude 2-methoxy-1-naphthoyl chloride produced in Step 1 while cooling in an ice bath. The reaction mixture is stirred at room temperature overnight, 50 ml of methylene chloride is added, and the reaction mixture is quenched with water. The methylene chloride phase is separated, washed twice with 10% aqueous sodium bicarbonate, dried over anhydrous sodium sulfate and evaporated at reduced pressure to near dryness. Diethyl ether is added to the residue, and the precipitate is dried under vacuum to obtain the colourless product, m.p. 160°-163°C.

When this reaction is scaled up, the yield is improved if one adds a solution of 2-methoxy-1-naphthoyl chloride in methylene chloride to a solution of 2-amino-2-methyl-1-propanol in methylene chloride stirred at 0°-5°C.

**Step 3: 4,4-Dimethyl-2-(2'-methoxynaphth-1'-yl)-2-oxazoline.hydrochloride (Reaction 0; compound XXIXa)**

4.8 ml of thionyl chloride is slowly added to 6.0 g of Compound XXVIIIa, the obtained suspension is stirred under nitrogen at room temperature for 4 hours, 10 ml of methylene chloride dried over molecular sieves is added, and the reaction mixture is stirred overnight under nitrogen. 50 ml of diethyl ether is added, and the precipitated solid is washed with diethyl ether and dried under vacuum to obtain the colourless product, m.p. 168°-171°C.

**Step -4: 4,4-Dimethyl-2-(2'-methoxynaphth-1'-yl)-2-oxazoline (Reaction P; compound XXXa)**

75 ml of 20% aqueous sodium hydroxide is added to 13 g of Compound XXIXa and the reaction mixture is extracted four times with diethyl ether. The diethyl ether extracts are combined, dried over anhydrous sodium sulfate and evaporated to dryness at reduced pressure, and the residue is triturated with diethyl ether/petroleum ether. The resulting solid is dried under vacuum to obtain the colourless product, m.p. 98°-101°C.

**Step 5: 4,4-Dimethyl-2-(2'-[4''-fluorophenyl]naphth-1'-yl)-2-oxazoline (Reaction Q; compound XXXIIa)**

A Grignard reagent prepared from 4.2 g of p-bromo-fluorobenzene and 0.583 g of magnesium turnings in 20 ml of dry tetrahydrofuran (distilled over sodium) is slowly added to a solution of 5.1 g of Compound XXXa in 30 ml of dry tetrahydrofuran stirred at room temperature under nitrogen. The reaction mixture is stirred overnight at room temperature under nitrogen and, while slightly cooling, is quenched with 20 ml of saturated ammonium chloride solution. The reaction mixture is extracted with diethyl ether, and the diethyl ether extract is dried over anhydrous sodium sulfate and evaporated to dryness at reduced pressure. The residue is triturated with diethyl ether/petroleum ether, and the precipitate is dried under vacuum to obtain the colourless product, m.p. 115°-117°C.

**Step 6: 2-(2'-[4''-Fluorophenyl]naphth-1'-yl)-3,4,4-trimethyl-2-oxazolinium iodide (Reaction R; compound XXXIIIa)**

7 ml of methyl iodide is added to a solution of 4.34g of Compound XXXIIa in 30 ml of nitromethane, and the reaction mixture is stirred at 80°-90°C under nitrogen overnight. The reaction mixture is cooled to room temperature, and 200 ml of diethyl ether is added; the resulting gummy precipitate solidifies on standing. The solid is washed with diethyl ether, dried under vacuum and recrystallised from acetonitrile/ ether to obtain the yellow product, m.p. 220°-222°C.

**Step 7: 2-(4'-Fluorophenyl)-1-naphthaldehyde (Reaction S; compound IVa)**

0.936 g of sodium borohydride is added portion-wise over a 2 minute period to a suspension of 11.36 g of Compound XXXIIIa in 120 ml of absolute ethanol stirred at about 0°C. The reaction mixture is stirred at about 0°C under nitrogen for 2 hours, and 200 ml of 2N.hydrochloric acid is added, cooling being maintained during the addition. The reaction mixture is stirred at room temperature under nitrogen for 40 hours, concentrated at reduced pressure and extracted three times with diethyl ether. The diethyl ether extracts are combined, washed twice with 3% aqueous sodium thiosulfate, washed once with water, dried over anhydrous sodium sulfate and evaporated to dryness at reduced pressure. The residue is dissolved in methylene chloride, and the solution is treated with charcoal, filtered and evaporated at reduced pressure to a small volume. A small amount of isopropanol is added, and the solution is evaporated under vacuum without heating. The precipitated colourless solid is washed with cold isopropanol, washed with petroleum ether and dried under vacuum to obtain the product, m.p. 78°-80°C.

**Step 8: (E)-3-(2'-[4''-Fluorophenyl]naphth-1'-yl)prop-2-enal (Reaction W; compound IVb)**

3.16 ml of 1.3M n-butyllithium/n-hexane is added dropwise to a solution of 1.414 g of cis-1-ethoxy-2-tri-n-butylstannylethylene in 40 ml of dry tetrahydrofuran (distilled over sodium) stirred at -78°C under nitrogen, stirring is maintained for 2 hours under the same conditions, and a solution of 0.888 g of Compound IVa in 10 ml of dry tetrahydrofuran (distilled over sodium) is added. The reaction mixture is stirred at -78°C under nitrogen for 1.5 hours and allowed to warm to room temperature. 5 ml of saturated aqueous sodium bicarbonate is added followed by 50 ml of water. The reaction mixture is extracted twice with 50 ml portions of diethyl ether, and the diethyl ether extracts are combined, washed twice with 50 ml portions of saturated aqueous sodium chloride, dried over anhydrous sodium sulfate and evaporated to dryness at reduced pressure. The residue is distributed between acetonitrile and n-hexane. The acetonitrile layer is extracted twice with n-hexane and evaporated to dryness at reduced pressure to obtain a dark green gum. 30 ml of 80% aqueous tetrahydrofuran and 5 mg of p-toluenesulfonic acid monohydrate are added, and the reaction mixture is stirred at room temperature for 4 hours. Water and 0.5 g of solid sodium bicarbonate are added, and the reaction mixture is extracted with ethyl acetate. The ethyl acetate extract is dried over anhydrous sodium sulfate and evaporated to dryness at reduced pressure to obtain a yellow sticky solid. The solid is triturated with diethyl ether/petroleum ether to obtain the colourless product, m.p. 119°-122°C.

**Step 9: Ethyl (E)-7-(2'-[4''-fluorophenyl]naphth-1'-yl)-5-hydroxy-3-oxohept-6-enoate (Reaction A; compound VIa)**

29.1 ml of 1.7 M n-butyllithium/n-hexane is slowly added to a solution of 6.9 ml of diisopropylamine in 140 ml of dry tetrahydrofuran stirred at 0°C under nitrogen. The reaction mixture is stirred at 0°C under nitrogen for 20 minutes, 3.14 ml of ethyl acetoacetate is slowly added, and the reaction mixture is stirred under the same conditions for 1 hour and cooled to -40° to -30°C. A solution of 3.4 g of Compound IVb in 75 ml of dry tetrahydrofuran is slowly added to the reaction mixture stirred at -40° to -30°C under nitrogen. The reaction mixture is stirred for an additional 45 minutes under the same conditions, quenched with 150 ml of saturated aqueous ammonium chloride and allowed to warm to room temperature overnight. Water is added to the reaction mixture, and the reaction mixture is extracted with ethyl acetate. The ethyl acetate extract is washed with water, dried over anhydrous sodium sulfate and evaporated to dryness at reduced pressure. The gummy residue is triturated with a small amount of diethyl ether, and the precipitated colourless solid is washed with cold 1:1 (by volume) diethyl ether/petroleum ether and dried under vacuum to obtain the product, m.p. 84°-86°C.

The product is a racemate that may be resolved into its R and S components.

**Step 10: Ethyl erythro-(E)-3,5-dihydroxy-7-(2'-[4''-fluorophenyl]naphth1'-yl)hept-6-enoate (process a); compound no. 1)**

6.52 ml of IM tri-n-butylborane/tetrahydrofuran is added to a solution of 2.42 g of Compound VIa in 200 ml of dry tetrahydrofuran stirred at room temperature, and 34 ml of air (at 25°C and 760 mm Hg) is slowly bubbled in. The reaction mixture is stirred at room temperature for 2 hours and cooled to -78° to -75°C, and 0.248 g of sodium borohydride is added portion-wise. The reaction mixture is stirred at -78° to -75°C under nitrogen for 3 hours, and a solution of 33.8 ml of 30% aqueous hydrogen peroxide, 67.6 ml of an aqueous phosphate buffer having a pH of 7.2 (0.047M. sodium phosphate/0.024M. potassium phosphate/0.054M. sodium hydroxide) and 67.6 ml of methanol is slowly added, the aforementioned temperature being maintained during the addition. The reaction mixture is allowed to warm to room temperature overnight, water is added,and the reaction mixture is extracted three times with methylene chloride. The methylene chloride extracts are combined, dried over anhydrous sodium sulfate and evaporated to dryness at reduced pressure. Methanol is added to the residue, it is heated at 40° to 45°C for 30 seconds, and the methanol is evaporated at reduced pressure at room temperature; this procedure is repeated twice. The residue is triturated with a small amount of diethyl ether while being cooled. The precipitated colourless solid is washed with diethyl ether/petroleum ether and dried under vacuum to obtain the product, m.p. 114°-116°C.

The product is a racemic mixture which may be resolved into two optically pure enantiomers, the 3R,5S and 3S,5R isomers, of which the former is preferred. The use of a non-stereoselective reduction, a t-butylamine-borane complex in abs. $C_2H_5OH$ at 0° for 1 hour, affords a mixture of all four enantiomers (compound no. 12).

**Example 2: Erythro-(E)-3,5-dihydroxy-7-(2'-[4''-fluorophenyl]naphth-1'-yl)hept-6-enoic acid and its sodium salt (process d); compound nos. 2 and 3)**

A mixture of 0.30 g of Compound 1, 10 ml of ethanol and 0.88 ml of 1N. aqueous sodium hydroxide is stirred at room temperature for 1.5 hours, water is added and the reaction mixture is extracted with diethyl ether. The aqueous phase, which contains racemic sodium salt can either be evaporated to dryness, m.p. 210°-220°C (decomp.) (compound 3) or acidified with 2N. hydrochloric acid, to yield a gummy precipitate which is extracted with ethyl acetate. The ethyl acetate extract is dried over anhydrous sodium sulfate and evaporated to dryness at reduced pressure to obtain the crude free acid as a sticky pale yellow foam, m.p. 43°-102°C (compound 2).

The products are racemic mixtures which may be resolved into optically pure enantiomers, the 3R,5S and 3S,5R isomers, of which the former is preferred.

**Example 3: (E)-Trans-6-(2'-[2''-(4'''-fluorophenyl)naphth-1''-yl]ethenyl)4-hydroxy-3,4,5,6-tetrahydro-2H-pyran-2-one (process e); compound no. 4)**

A solution of 0.223 g of Compound 2 in 40 ml of dry toluene is refluxed for 5 hours, the water formed being removed by the use of a Dean-Starke apparatus. The reaction mixture is cooled and extracted with 10% aqueous sodium bicarbonate and with water. The toluene solution is dried over anhydrous sodium sulfate and evaporated to dryness at reduced pressure. The residue is dissolved in diethyl ether and evaporated at reduced pressure and room temperature until precipitation commences. The solution is cooled, and the

precipitated pale yellow solid is washed with petroleum ether and dried under vacuum to obtain the product, m.p. 152-154°C.

The product is a racemate that may be resolved by conventional means into two optically pure enantiomers, the 4R,6S and 4S,6R isomers, of which the former is preferred. The product contains about 1% of the corresponding cis racemate, which may be separated from the trans racemate by, for example, column chromatography. The cis racemate may be resolved by conventional means into two optically pure enantiomers, the 4R,6R and 4S,6S isomers. The cis racemate results from a small amount of the threo isomer of Compound 1 formed in Step 10 of Example 1 and not separated therefrom which is carried through process d) (Example 2) and process e) (this example). A sample of the trans lactone free of any detectable cis lactone melted at 153° to 156°C.

**Example 4: Ethyl erythro-3,5-dihydroxy-7-(2'-[4''-fluorophenyl]-5',6',7',8'tetrahydronaphth-1'-yl)heptanoate (compound no. 5)**

**Step 1: Ethyl 7-(2'-[4''-fluorophenyl]-5',6',7',8'-tetrahydronaphth-1'-yl5-hydroxy-3-oxoheptanoate (reaction JJ; compound VIb)**

A solution of 406 mg (10 mmole) of Compound VIa in 20 ml of glacial acetic acid is contacted with a large excess of hydrogen at an initial pressure of 50 p.s.i. at room temperature in the presence of 40 mg of platinum dioxide until 3 mmoles of hydrogen (7.5 p.s.i.) are taken up. The platinum dioxide is removed by filtration, and the filtrate is evaporated to dryness at reduced pressure. The residue (397 mg) is dissolved in a small amount of methylene chloride and applied to silica gel preparative thin layer chromatography plates; methylene chloride is used as the chromatography solvent. The bands containing the product are eluted with ethyl acetate. The ethyl acetate is evaporated at reduced pressure to obtain the crude product as a yellow gum.

The product is a racemate that may be resolved into its R and S components.

**Step 2: Ethyl erythro-3,5-dihydroxy-7-(2'-[4''-fluorophenyl]-5',6',7',8'tetrahydronaphth-1'-yl)heptanoate (process a); compound no. 5)**

Analogous to Example 1. Step 10 starting from compound VIb.

Product (compound no. 5) is obtained as a yellow gum.

The product is a racemate that may be resolved into two optically pure enantiomers, the 3R,5R and 3S,5S isomers, of which the former is preferred.

**Example 5: Ethyl erythro-(E)-7-(4'-chloro-2'-[4''-fluorophenyl]naphth-1'yl)-3,5-dihydroxy hept-6-enoate (compound no. 6)**

**Step 1: 4-Chloro-2-naphthol (Reaction DA; compound CXXIX)**

A mixture of 90 g (0.42 mole) of 1,4-dichloro-2-naphthol, 420 g of stannous chloride.dihydrate and 1.5 liters of glacial acetic acid is refluxed for about 80 hours, gaseous hydrogen chloride being slowly bubbled in throughout. The reaction mixture is added to 9 liters of ice water, the resulting mixture is stirred for 1 hour, and the product is collected by filtration, washed with 4 liters of water, washed with 2 liters of petroleum ether, air dried and dried at 40°C under high vacuum for 5 hours, m.p. 93°-95°C.

**Step 2: 1-Chloro-3-methoxynaphthalene (Reaction DB; compound CXXX)**

23.2 g (0.414 mole) of ground potassium hydroxide is added to a mixture of 65.4 (0.366 mole) of 4-chloro-2-naphthol and 370 ml of dimethylformamide stirred at 0°-5°C. The reaction mixture is stirred at 0°-5°C for 3 hours, 58.8 g (0.414 mole) of methyl iodide is added over a 5 minute period, and the reaction mixture is allowed to warm to 20° to 25°C and is stirred at that temperature for 16 hours. 2 Liters of water is added, and the mixture is extracted with diethyl ether. The diethyl ether is evaporated at reduced pressure until the onset of turbidity, and petroleum ether is added to obtain the solid product, m.p. 35°-39°C.

**Step 3: 4-Chloro-2-methoxy-1-naphthaldehyde (Reaction DC; compound CXXXI)**

50 g (0.326 mole) of phosphorus oxychloride is slowly added to a mixture of 24 g (0.125 mole) of 1-chloro-3-methoxynaphthalene and 30 g (0.411 mole) of dimethylformamide stirred at 20°-25°C. The reaction mixture is stirred at 80°C for 16 hours, cooled in an ice bath and made basic by the dropwise addition of 10% aqueous sodium hydroxide solution with vigorous stirring. The precipitate is collected by filtration, washed with water, washed with petroleum ether and air dried to obtain the crude product, m.p. 145°-160°C (dec.) (shrinks at 105°-145°C).

An analytical sample may be obtained by (a) dissolving the crude product in ethyl acetate, (b) adding petroleum ether, (c) decanting the solution from the insoluble tar, (d) repeating (b) and (c) until no more tar results and (e) adding additional petroleum ether to obtain the pure product, m.p. 154°-157°C.

**Step 4: 4-Chloro-2-methoxy-1-naphthoic acid (Reaction DE, compound XXIIIa)**

18.6 g (0.175 mole) of sodium carbonate is added to a mixture of 37.2 g (0.169 mole) of crude 4-chloro-2-methoxy-1-naphthaldehyde, 450 ml of acetone and 92 ml of water stirred at 20°-25°C. 27.9 g (0.177 mole) of potassium permanganate is added over a 2.5 hour period with stirring at 40°-45°C. The reaction mixture is stirred at room temperature for 16 hours, 400 ml of water is added, and the reaction mixture is filtered through Celite, acidified with 2N. hydrochloric acid and extracted with ethyl acetate. The ethyl acetate extract is extracted three times with 10% aqueous sodium carbonate solution, and the combined aqueous extracts are carefully acidified with 2N hydrochloric acid. The obtained product is washed with water and washed with petroleum ether, m.p. 199.5°-202°C.

**Step 5 to 14**

Proceed analogously to Steps 1 to 10 of Example 1 through the intermediates listed hereafter.

**Step 5: 4-Chloro-2-methoxy-1-naphthoyl chloride (Reaction M; compound XXVIb), oil)**

**Step 6: 4-Chloro-2-methoxy-1-naphthoic acid N-1,1-dimethyl-2-hydroxyethylamide (Reaction N; compound XXVIIIb), m.p. 142° to 146°**

**Steps 7 and 8: 2-(4'-Chloro-2'-methoxynaphth-1'-yl)-4,4-dimethyl-2-oxazoline and its hydrochloride salt (Reactions 0 and P; compound XXXb and XXIXb), m.p. 91-94° (XXXb)**

**Step 9: 2-(4'-Chloro-2'-[4''-fluorophenyl]naphth-1'-yl)-4,4-dimethyl-2oxazoline (Reaction Q; compound XXXIIb), m. p 150°-152°**

Gringard reagent cf. Example 10.7.

**Step 10: 2-(4'-Chloro-2'-[4''-fluorophenyl]naphth-1'-yl)-3,4,4-trimethyl2-oxazolinium iodide (Reaction R; compound XXXIIIb), m.p. 224°-226°**

**Step 11: 4-Chloro-2-(4'-fluorophenyl)-1-naphthaldehyde (Reaction S. compound IVc), m.p. 137°-139°**

**Step 12: (E)-3-(4'-Chloro-2'-[4''-fluorophenyl]naphth-1'-yl)prop-2-enal (Reaction W; compound IVd), m.p. 143-147°**

**Step 13: Ethyl (E)-7-(4'-chloro-2'-[4''-fluorophenyl]naphth-1'-yl)-5-hydroxy-3-oxohept-6-enoate (Reaction A; compound VIc), m.p. m.p. 87°-89°**

**Step 14: Ethyl erthro (E)-7-(4'-chloro-2'-[4''-fluorophenyl]naphth-1'-yl3,5-dihydroxyhept-6-enoate (process a); compound no. 6), m.p. 121-124°**

The principal (erythro) product is a racemate that may be resolved into two optically pure enantiomers, the 3R,5S and 3S,5R isomers, of which the former is preferred. The threo minor product is a racemate that may be resolved into the 3R,5R and 3S,5S isomers, of which the former is preferred. The use of a non-stereoselective reduction would afford all four stereoisomers in approximately equal amounts.

The crude reaction mixture of Step 14 also contains threo isomer.

**Example 6: Erythro-(E)-7-(4'-chloro-2'-[4''-fluorophenyl]naphth-1'-yl)3,5-dihydroxyhept-6-enoic acid and its sodium salt (process d); compounds nos. 7 and 8)**

Analogous to Example 2 starting from compound 6, m.p. 201°-204° (dec.) for salt. Free acid as crude oil containing a small amount of threo compound.

The principal (erythro) product is a racemate which may be resolved into two optically pure enantiomers, the 3R,5S and 3S,5R isomers, of which the former is preferred. The minor (threo) product is also a resolvable racemate, the two enantiomers being the 3R,5R and 3S,5S isomers, of which the former is preferred.

**Example 7: (E)-Trans-6-(2'-[4''-chloro-2''-(4''-fluorophenyl)naphth-1''-yl]ethenyl)-4-hydroxy-3,4,5,6-tetrahydro-2H-pyran-2-one (Process e); compound no. 9)**

Analogous to Example 3 starting from Compound 7, m.p. of product 131°-134°.

**Example 8: (E)-Trans-6S-(2'-[2''-(4''-fluorophenyl)naphth-1''-yl]ethenyl)4R-hydroxy-3,4,5,6-tetrahydro-2H-pyran-2-one (compound no. 10)**

**Step 1: 2-(4'-fluorophenyl)-1-naphthalenemethanol (Reaction KK, compound LIIa)**

75 g (1.94 moles) of powdered sodium borohydride is added to a mixture of 497 g (1.988 moles) of 2-(4'-fluorophenyl)-1-naphthaldehyde and 6.6 liters of absolute ethanol stirred at 20°-25°. The reaction mixture is stirred at this temperature for 16 hours, suction filtered through 1 kg. of silica gel (EM-60, 230-240 mesh ASTM) and evaporated at reduced pressure to obtain a crystalline residue. The solids are collected by filtration, washed with 100 ml of cold (0°) methylene chloride and taken up in 1 liter of diethyl ether. The insoluble residue is removed by filtration, and the filtrate is evaporated to dryness at reduced pressure. The residue is dissolved in 500 ml of methylene chloride, and the solution is cooled to 0° to obtain the product, m.p. 91-93°.

**Step 2: 1-Chloromethyl-2-(4'-fluorophenyl)naphthalene (Reaction LL; compound LIIIa)**

A solution of 45 g (0.378 mole) of thionyl chloride in 500 ml of methylene chloride is added over a 20 minute period to a mixture of 237 g (0.94 mole) of 2-(4'-fluorophenyl)-1-naphthalenemethanol and 3 liters of methylene chloride stirred at 20°-25° under nitrogen, and the reaction mixture is stirred at this temperature for 16 hours. An additional 25 g (0.31 mole) of thionyl chloride is added, the reaction mixture is stirred at 20°-25° for 2 hours and cooled to 0°. 1 liter of 10% aqueous sodium bicarbonate solution is cautiously added, the organic layer is separated, and the aqueous phase is extracted with 500 ml of methylene chloride. The two organic phases are combined, washed with 1 liter of saturated aqueous sodium chloride solution, dried over anhydrous sodium sulfate, filtered through 1 kg of silica gel (EM-60, 230-400 mesh ASTM) and concentrated at reduced pressure to obtain the crystalline product, m.p. 93-95°.

**Step 3: [(2-[4'-Fluorophenyl]-1-naphthalenyl)methyl]triphenylphosphonium chloride (Reaction NN; compound LIVa)**

A mixture of 218 g (0.81 mole) of 1-chloromethyl-2-(4'-fluorophenyl)-naphthalene, 214 g (0.81 mole) of triphenylphosphine and 4 liters of toluene is refluxed under nitrogen for 16 hours, cooled, concentrated at reduced pressure, to about 1/3 of its original volume, filtered through 1 kg of silica gel (EM-60, 230-400 mesh ASTM) and evaporated to dryness at reduced pressure 500 ml of anhydrous diethyl ether is added to the

crystalline residue, the mixture is cooled to 0°, and the precipitate is collected by filtration, washed twice with a total of 500 ml of anhydrous diethyl ether and vacuum dried at 70° for 5 hours to obtain the product as a colourless solid, m.p. > 250°.

**Step 4: (E)-4βR-(1',1'-Dimethylethyl-diphenylsilyloxy)-6αS-(2'-[2''-(4''fluorophenyl)naphth-1''-yl]ethenyl)-2-methoxy-3,4,5,6-tetrahydro -2H-pyran (Reaction 00; compound LVIa)**

115.6 ml of 1.65M. n-butyllithium/n-hexane (0.191 mole) is added over a period of 5 minutes to a mixture of 100 g (0.188 mole) of Compound LIVa in 1.5 liters of dry tetrahydrofuran stirred at -15° under nitrogen. The reaction mixture is stirred at about 0° for 1 hour and cooled to -55°, a solution of 71.5 g (0.179 mole) of Compound LVa

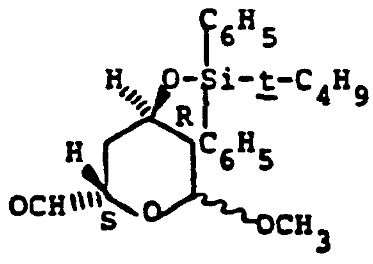

LVa

in 600 ml of dry tetrahydrofuran is added over a period of about 20 minutes, the temperature of the reaction mixture being -55° to -50° during the addition, and the reaction mixture is allowed to slowly warm to 20°-25° over a 16 hour period, stirring under nitrogen being maintained throughout. The reaction mixture is cooled to 0°, quenched with 1 liter of saturated aqueous ammonium chloride solution and filtered through Celite. The organic phase is separated, and the aqueous phase is extracted twice with 750 ml portions of diethyl ether. The three organic phases are combined, washed with 1 liter of saturated aqueous sodium chloride solution, dried over anhydrous sodium sulfate and evaporated to dryness at reduced pressure to obtain an oil. The oil is dissolved in 150 ml of 2:1 (by volume) methylene chloride/n-hexane and flash chromatographed on a column packed with 1 kg of silica gel (EM-60, 230-400 mesh ASTM) utilizing the same solvent as the eluant. The fractions containing the product (as determined by thin layer chromatography) are combined and evaporated to dryness at reduced pressure to obtain the product as a gummy solid, $[\alpha]_D^{26} = +24.51°$ ($CH_2Cl_2$, c = 0.01).

**Step 5: (E)-4βR-(1',1'-Dimethylethyl-diphenylsilyloxy)-6αS-(2'-[2''-(4''fluorophenyl)naphth-1''-yl]ethenyl)-2-hydroxy-3,4,5,6-tetrahydro-2H-pyran (Reaction PP; compound LVIIa)**

A mixture of 121 g (0.196 mole) of Compound LVIa and 2 liters of 3:2:4 (by volume) acetic acid/water/tetrahydrofuran is heated to 65° with stirring, stirred at 65° for 16 hours and cooled to 20°-25°, and 1.5 liters of methylene chloride is added. With stirring and cooling, the mixture is carefully washed with saturated aqueous sodium carbonate solution until slightly basic. The organic phase is separated, and the aqueous phase is extracted with 1 liter of methylene chloride. The organic phases are combined, washed with saturated aqueous sodium chloride solution, dried over anhydrous sodium sulfate and evaporated at reduced pressure to obtain a light brown oil. The oil is dissolved in 1:1 (by volume) diethyl ether/petroleum ether and flash chromatographed on a column packed with 1 kg of silica gel (EM-60, 230-400 mesh ASTM) utilizing the same solvent as the eluant. The fractions containing the product and little or nothing else (as determined by thin layer chromatography) are combined and evaporated at reduced pressure to obtain the product as a gummy solid,

$$[\alpha]_D^{26} = +5.00° \ (CH_2Cl_2, \ c = 0.01).$$

**Step 6: (E)-4βR-(1',1'-Dimethylethyl-diphenylsilyloxy)-6αS-(2'-[2''(4''-fluorophenyl)naphth-1''-yl]ethenyl)-3,4,5,6-tetrahydro-2H -pyran-2-one (Reaction QQ; compound LVIIIa)**

A mixture of 89 g (0.147 mole) of Compound LVIIa, 2 liters of methylene chloride, 64 g (0.317 mole) of pyridinium chlorochromate and 60 g of # 3A molecular sieves is stirred at 20°-25° under nitrogen for 16 hours and filtered through Celite and about 100 g of neutral aluminium oxide. The Celite and aluminium oxide are washed several times with methylene chloride and the filtrate and washings are combined and evaporated at reduced pressure to obtain an oil. The oil is dissolved in 100 ml of methylene chloride and flash chromatographed on a column packed with 800 g of silica gel (EM-60, 230-400 mesh ASTM) utilizing methylene chloride as the eluant. The fractions containing the product and little or nothing else (as determined by thin layer chromatography) are combined and evaporated at reduced pressure to obtain the product as an oil,

$$[\alpha]_D^{25} = +12.32° \ (CH_3OH, \ c = 0.0215).$$

**Step 7: (E)-Trans-6S-(2'-[2''-(4''-fluorophenyl)naphth-1''-yl]ethenyl) -4R-hydroxy-3,4,5,6-tetrahydropyran-2H-one (process c); compound no. 10)**

26.3 ml (0.46 mole) of glacial acetic acid is added with stirring to a mixture of 63 g (0.105 mole) of Compound LVIIIa in 2.3 liters of dry tetrahydrofuran stirred at 20°-25°. 425 ml of IM. tetra-n-butylammonium fluoride/tetrahydrofuran (0.425 mole) is added, and the reaction mixture is stirred at 20°-25° for 2 hours. 157 g of solid sodium bicarbonate is added, and the reaction mixture is stirred for 30 minutes and filtered through 800 g of silica gel (EM-60, 230-400 mesh ASTM). The silica gel is washed twice with 500 ml portions of diethyl ether, and the washings are combined with the filtrate. The combined filtrate and washings are evaporated to dryness at reduced pressure, 200 ml of diethyl ether is added, and the mixture is cooled to 0°. The waxy crystals are collected by filtration, washed twice with 100 ml portions of diethyl ether and dissolved in 300 ml of ethyl acetate. The ethyl acetate solution is washed twice with 500 ml portions of saturated aqueous sodium bicarbonate solution and once with saturated aqueous sodium chloride solution, dried over anhydrous sodium sulfate and evaporated at reduced pressure to about 1/3 of its original volume. 100 ml of diethyl ether is added, the mixture is cooled to 0°, and the obtained solids are collected by filtration and washed twice with diethyl ether to obtain the product, m.p. 184°-186°,

$$[\alpha]_D^{26} = +44.31° \ (CH_2Cl_2, \ c = 0.0058).$$

**Example 9: Sodium erythro-(E)-3R,5S-dihydroxy-7-(2'-[4''-fluorophenyl]-naphth-1'-yl)hept-6-enoate (process d); compound no. 11)**

5.06 ml of IN. aqueous sodium hydroxide solution (5.06 mmoles) is added to a solution of 2.0 g (5.52 mmoles) of Compound 10 in 160 ml of absolute ethanol, and the reaction mixture is stirred at 20°-25° for 2 hours. 50 g of sodium sulfate is added, and the mixture is stirred for 1 hour and filtered. The salt is washed three times with 100 ml portions of diethyl ether, and the washings are combined with the ethanolic filtrate. The combined washings and filtrate are evaporated to dryness at reduced pressure, and the crystalline residue is dissolved in chloroform. The chloroform is evaporated at reduced pressure

300 ml of petroleum ether is added to the residue, and the mixture is stirred for about 60 hours. The solid product is collected by filtration and washed twice with petroleum ether, m.p. 215°-220° (dec.)

$$[\alpha]_D^{28} = +26.283° \ (CH_3OH, \ c = 0.0047).$$

**Example 10: Ethyl erythro-(E)-3,5-dihydroxy-7-(1'-[4''-fluorophenyl]-3'-[1''-methylethyl]naphth-2'-yl)hept-6-enoate (Compound no. 13)**

**Step 1: 1,3-Dimethoxynaphthalene (Reaction BA; compound CXVIa)**

A solution of 32.1 g (0.801 mole) of sodium hydroxide in 80.3 ml of water and 96 g (0.763 mole) of dimethyl sulfate are simultaneously added over a period of 30-45 minutes to 50 g (0.312 mole) of 1,3-dihydroxynaphthalene in 250 ml of absolute ethanol stirred at -5° - 0°C, the former being added slightly faster than the latter. The reaction mixture is allowed to gradually warm to 20° - 25°C with stirring over a 16 hour period. Most of the ethanol is evaporated at reduced pressure, water is added, and the mixture is extracted three times with methyl t-butyl ether. The extracts are combined, washed with 2N. aqueous sodium carbonate solution, dried over anhydrous sodium sulfate and evaporated to dryness at reduced pressure to obtain an oil. The oil is chromatographed on a Waters Prep-500 high pressure liquid chromatography apparatus having a silica gel column and utilizing 5% ethyl acetate/n-hexane as the eluant. The fractions containing the product are combined and evaporated at reduced pressure to obtain the product as a yellow oil.

**Step 2: 1,3-Dimethoxy-2-naphthoic acid (Reaction BC; compound CXVIIa)**

62 ml of 1.55M. n-butyllithium/n-hexane (96 mmoles) is slowly added to 15.04 g (80 mmoles) of 1,3-dimethoxynaphthalene in 250 ml of anhydrous diethyl ether stirred at 0°C under nitrogen. The reaction mixture is allowed to warm to 20°-25°C and is stirred at this temperature for 20 hours, the reaction mixture being stirred under nitrogen throughout. Excess anhydrous carbon dioxide is bubbled in for 30 minutes. The reaction mixture is stirred at 20°-25°C for 4 hours, quenched with water and extracted thoroughly with ethyl acetate. The alkaline aqueous phase is acidified with 2N. hydrochloric acid (to a pH of 1-2) and extracted with ethyl acetate. This ethyl acetate extract is dried over anhydrous sodium sulfate and evaporated to dryness. The residue is dried under high vacuum to obtain the product, m.p. 119°-123°C.

**Step 3: 1,3-Dimethoxy-2-naphthoyl chloride (Reaction BD; compound CXVIIIa)**

Analogous to Example 1, Step 1.

**Step 4: 1,3-Dimethoxy-2-naphthoic acid N-1,1-dimethyl-2-hydroxyethylamide (Reaction BE; compound CXIXa)**

Analogous to Example 1, Step 2.

**Steps 5 and 6: 2-(1',3'-dimethoxynaphth-2'-yl)-4,4-dimethyl-2-oxazoline and its hydrochloride salt (Reactions BF and BG; compound CXXIa + HCl salt**

Analogous to Example 1, Steps 3 and 4.

**Step 7: 4,4-Dimethyl-2-(1'-[4''-fluorophenyl]-3'-methoxynaphth-2'-yl)-2-oxazoline (Reaction BH; compound CXXIIa)**

A Grignard reagent is prepared by the dropwise addition of 7.7 g (0.044 mole) of p-bromofluorobenzene to a mixture of 1.1 g (0.045 mole) of magnesium turnings, one crystal of iodine and 40 ml of dry tetrahydrofuran stirred at 65°C under nitrogen, the addition being at a rate sufficient to maintain reflux without external heating. Upon completion of the addition (30-45 minutes), the reaction mixture is refluxed under nitrogen for 1.5 hours and cooled to obtain a solution of the Grignard reagent.

18 ml of a lM. solution of the Grignard reagent is slowly added to 4.25 g (14.9 mmoles) of Compound CXXIa in 80 ml of dry tetrahydrofuran (distilled from sodium) stirred at 20°-25°C under nitrogen, and the reaction mixture is stirred at 20°-25°C under nitrogen for 16 hours and quenched with ice and saturated aqueous ammonium chloride solution. The mixture is extracted with ethyl acetate, and the ethyl acetate extract is dried over anhydrous sodium sulfate and evaporated to a small volume at reduced pressure to obtain the product. The product is collected by filtration, washed with a small amount of diethyl ether, washed with a small amount of petroleum ether and dried under high vacuum, m.p. 169°-171°C.

**Step 8: 4,4-Dimethyl-2-(1'-[4''-fluorophenyl]-3'-[1''-methylethyl]naphth2'-yl)-2-oxazoline (Reaction BI; compound CXXIVa)**

13.25 ml of 2M. isopropylmagnesium chloride/diethyl ether (26.5 mmoles) is slowly added to 1.54 g (4.41 mmoles) of Compound CXXIIa in 90 ml of dry tetrahydrofuran (distilled from sodium) and 22 ml of dry toluene (dried over molecular sieves) stirred at 20°-25°C, the reaction mixture is stirred at 20°-25°C for 30 minutes and at 70°-80°C for 18 hours, an additional 13.25 ml of 2M. isopropylmagnesium chloride/diethyl ether (26.5 mmoles) is added, and the reaction mixture is stirred at 70°-80°C for an additional 20 hours, the reaction mixture being maintained under nitrogen throughout. The reaction mixture is quenched with ice and saturated aqueous ammonium chloride solution and extracted with ethyl acetate. The ethyl acetate extract is dried over anhydrous sodium sulfate and evaporated to dryness at reduced pressure. The residue is dissolved in methylene chloride, and the solution is dried over anhydrous sodium sulfate and evaporated to dryness at reduced pressure. The obtained gum is dissolved in about 10 ml of methylene chloride, a small amount of charcoal is added, and the solution is filtered through a silica gel column utilizing methylene chloride as the eluant. The fractions containing the product as determined by thin layer chromatography are combined and evaporated to dryness at reduced pressure to obtain the product as an amber-green gum.

**Step 9: 2-(1'-[4''-Fluorophenyl]-3-'-[1''-methylethyl]naphth-2'-yl)-3,4,4-trimethyl-2-oxazolinium iodide (Reaction R; cmpd. XXXIIIc)**

Analogous to Example 1, Step 6: m.p. 233°-243°C (dec.).

**Step 10: 1-(4-Fluorophenyl)-3-(1'-methylethyl)-2-naphthaldehyde (Reaction S; compound IVe)**

205 mg (9.37 mmoles) of lithium borohydride is added to 2.35 g (4.67 mmoles) of Compound XXXIIIc stirred in 105 ml of dry tetrahydrofuran (distilled from sodium) and 42 ml of absolute ethanol (dried over molecular sieves) at -30°C under nitrogen. The reaction mixture is stirred at -40° to -30°C under nitrogen for 2 hours and allowed to warm to 0° to 5°C, and 62 ml of 2N. hydrochloric acid is slowly added. The reaction mixture is stirred at 70°-80°C for 2 hours, cooled to 20°-25°C, quenched with water and thoroughly extracted with diethyl ether. The diethyl ether extract is washed with saturated aqueous sodium chloride solution, dried over anhydrous sodium sulfate and evaporated to dryness at reduced pressure. The residue is triturated with diethyl ether and then petroleum ether. The insoluble solids are removed by filtration, and the filtrate is evaporated to dryness at reduced pressure to obtain the crude product as a light orange-yellow sticky solid, m.p. 80°-90°C.

**Step 11: (E)-3-(1'-[4''-Fluorophenyl]-3'-(1''-methylethyl)naphth-2'-yl)-prop-2-enal(Reaction W; compound IVF)**

Analogous to Example 1, Step 8: m.p. 102°-105°C.

**Step 12: Ethyl (E)-7-(1'-[4''-fluorophenyl]-3'-[1''-methylethyl]naphth1'-yl)-5-hydroxy-3-oxohept-6-enoate (Reaction A; cmpd. VId)**

Analogous to Example 1, Step 9: m.p. 73-76°C.

**Step 13: Ethyl erythro-(E)-3,5-dihydroxy-7-(1'-[4''-fluorophenyl]-3'-[1''methylethyl]naphth-2'-yl)hept-6-enoate (Process a); cmpd. no. 13)**

Analogous to Example 1, Step 10: yellow gummy foam.

The principal (erythro) product is a racemate that may be resolved into two optically pure enantiomers, the 3R,5S and 3S,5R isomers, of which the former is preferred. The threo minor product (about 8%) is a racemate that may be resolved into the 3R,5R and 3S,5S isomers, of which the former is preferred. The use of a non-stereoselective reduction would afford all four stereoisomers in approximately equal amounts.

**Example 11: Erythro-(E)-3,5-dihydroxy-7-(1'-[4''-fluorophenyl]-3'-[1''methylethyl]naphth-2'-yl)hept-6-enoic acid and its sodium salt (Process d, cmpds. nos. 14 (acid) and 15 (salt)**

Analogous to Example 2 (without isolation of intermediate sodium salt). The principal (erythro) product is a racemate which may be resolved into two optically pure enantiomers, the 3R,5S and 3S,5R isomers, of which the former is preferred. The minor (threo) product is also a resolvable racemate, the two enantiomers being the 3R,5R and 3S,5S isomers, of which the former is preferred.

**Example 12: (E)-Trans-6-(2'-(1''-[4''-fluorophenyl)-3''-(1''-methylethyl)naphth-2''-yl]ethenyl)-4-hydroxy-3,4,5,6-tetrahydro-2H-pyran-2-one (Process e); compound no. 16)**

Analogous to Example 3.

The crude product is purified by preparative thin layer chromatography on silica gel plates utilizing 2% methanol/methylene chloride as the solvent and ethyl acetate to elute the product from the plates. Evaporation of the ethyl acetate at reduced pressure yields the product as a pale yellow foam.

Separation of isomers cf. Example 3.

**Example 13: Sodium erythro-(E)-3,5-dihydroxy-7-(1'-[4''-fluorophenyl]-3'[1''-methylethyl]naphth-2'-yl)hept-6-enoate (Process d); compound no. 15)**

0.19 ml of 1N. aqueous sodium hydroxide solution is added to 80 mg (0.2 mmole) of Compound no. 16 in 5 ml of absolute ethanol. The reaction mixture is stirred at 20°-25°C for 1 hour and evaporated to dryness at reduced pressure. The residue is dissolved in chloroform, the obtained solution is dried over anhydrous sodium sulfate and evaporated to dryness, and the residue is dried under high vacuum to obtain the product as a pale yellow foam.

The principal product is the erythro racemate which may be resolved to obtain the 3R,5S and 3S,5R enantiomers, of which the former is preferred. A very small amount of the threo racemate is present; it may be resolved to obtain the 3R,5R and 3S,5S enantiomers, of which the former is preferred.

**Example 14: Ethyl erythro-(E)-3,5-dihydroxy-7-(1'-[4''-fluorophenyl]-3'methylnaphth-2'-yl)hept-6-enoate (Compound no. 17)**

**Steps 1/2: 1-Methoxy-2-naphthoic acid (Compound no. XXIIIb)**

1-Hydroxy-2-naphthoic acid is dimethylated substantially as described in Example 5, Step 2 and the resulting methyl 1-methoxy-2-naphthoate hydrolysed substantially according to Example 2: m.p. of product 126°-128°C.

**Steps 3 to 7: 4,4-Dimethyl-2-(1'-[4''-fluorophenyl]naphth-2'-yl)-2oxazoline (Reactions M - Q; compound XXXIIc)**

Analogous to to Example 1, Steps 1 to 5:
m.p. Step 4 product = 95°-97°
m.p. Step 6 product = 75°-78°
m.p. Step 7 product (XXXIIc) = 96°-98°

**Step 8: 4,4-Dimethyl-2-(1'-[4''-fluorophenyl]-3'-methylnaphth-2'-yl)-2-oxazoline (Reaction CA; compound XXXIId)**

16.16 ml of 1.7M. n-butyllithium/n-hexane (27.5 mmoles) is added dropwise to 7.9 g (24.8 mmoles) of Compound XXXIIc in 185 ml of anhydrous diethyl ether stirred at 0°C under nitrogen. The reaction mixture is stirred at 0°C for 30 minutes, 3.9. g (27.5 mmoles) of methyl iodide is added and the reaction mixture is allowed to warm to 20°-25°C and stirred at this temperature for 20 hours, the reaction mixture being maintained under nitrogen throughout. Saturated aqueous sodium chloride solution and additional diethyl ether are added. The diethyl ether phase is separated, dried over anhydrous sodium sulfate and evaporated at reduced pressure until the onset of turbidity, and petroleum ether is added. The precipitate is washed with petroleum ether and dried under high vacuum, m.p. 125°-129°C.

**Steps 9 to 13: Ethyl erythro-(E)-3,5-dihydroxy-7-(1'-[4''-fluorophenyl]-3'methylnaphth-2'-yl)hept-6-enoate (Compound no. 17)**

Analogous to Example 1, Step 6; Example 10, Step 10; Example 1, Steps 8 to 10.
m.p. product of Step 10 first reaction isolated, 115°-118°
m.p. product of step 10 second reaction, 104-107°
m.p. product of Step 11. 143°-147°
m.p. product of Step 12, 89°-92°
m.p. product of Step 13 (compound no. 17), 121°-124°.

The product, the erythro racemate, may be resolved into two optically pure enantiomers, the 3R,5S and 3S,5R isomers, of which the former is preferred. It contains a small amount (less than about 5%) of the corresponding threo compound, a racemate that may be resolved into the 3R,5R and 3S,5S isomers, of which the former is preferred. The use of a non-stereoselective reduction would afford all four stereoisomers in approximately equal amounts.

**Example 15: Sodium erythro-(E)-3,5-dihydroxy-7-(1'-[4''-fluorophenyl]-3'methylnaphth-2'-yl)hept-6-enoate (Process d): compound no. 18)**

Analogous to Example 2 with isolation of sodium salt;
m.p. 222°-226° (dec.).

The erythro component of the product, the erythro racemate, may be resolved into two optically pure enantiomers, the 3R,5S and 3S,5R isomers, of which the former is preferred. The product contains a small amount (less than about 5%) of the corresponding threo compound also a resolvable racemate, the two enantiomers being the 3R,5R and 3S,5S isomers, of which the former is preferred.

**Example 16: Ethyl erythro-(E)-3,5-dihydroxy-7-(3'-ethyl-1'-[4''-fluorophenyl]naphth-2'-yl)hept-6-enoate (Compound no. 19)**

**Step 1: 2-(3'-Ethyl-1'-[4''-fluorophenyl]naphth-2'-yl)-4,4-dimethyl-2-oxazoline (Reaction CB; compound XXXIIe)**

Analogous to Example 14, Step 8 starting from XXXIId; m.p. product 106°-109°.

**Steps 2 to 6: Ethyl erythro-(E)-3,5-dihydroxy-7-(3'-ethyl-1'-[4''-fluorophenyl]naphth-2'-yl)hept-6-enoate (Compound no. 19)**

Analogous to Example 1, Step 6; Example 10, Step 10; Example 1, Steps 8 to 10.
m.p. product of Step 2 = 200° (dec.)
m.p. product of Step 5 = 92°-98°
m.p. product of Step 6 = (compound no. 19) = 101°-106°.

The principal component of the product, the erythro racemate, may be resolved into two optically pure enantiomers, the 3R,5S and 3S,5R isomers, of which the former is preferred. The use of a non-stereoselective reduction would afford all four stereoisomers in approximately equal amounts.

**Example 17: Erythro-(E)-3,5-dihydroxy-7-(3'-ethyl-1'-[4''-fluorophenyl]naphth-2'-yl)hept-6-enoic acid and its sodium salt (Process d); compound nos. 20 and 21)**

Analogous to Example 2 (without isolation of intermediate sodium salt); separation of isomers as Example 16.

**Example 18: (E)-Trans-6-(2'-[3''-ethyl-1''-(4''-fluorophenyl)naphth-2''-yl)ethenyl)-4-hydroxy-3,4,5,6-tetrahydropyran-2-one (process e); compound no. 22)**

Analogous to Examples 3 and 12; m.p. 118°-122°; separation of isomers cf. Example 3 (about 9% of cis racemate).

**Example 19: Sodium erythro-(E)-3,5-dihydroxy-7-(3'-ethyl-1'-[4''-fluorophenyl]naphth-2'-yl)hept-6-enoate (process d) compound no. 21)**

Analogous to Example 13 starting from compound no. 22.

The principal component of the product is the erythro racemate which may be resolved to obtain the 3R,5S and 3S,5R enantiomers, of which the former is preferred. The product contains a small amount of the corresponding threo racemate, which may be resolved to obtain the 3R,5R and 3S,5S enantiomers, of which the former is preferred.

TABLE I

The following compounds of Groups IAa1-IAa3 may be synthesized by the processes set forth above:

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|---|---|
| 1 | H | H | H | 4-F | H | 2 | (E)CH=CH | 1 | H | $C_2H_5$ | E | 114-116° |
| 12 | H | H | H | 4-F | H | 2 | " | 1 | H | $C_2H_5$ | 4 enantiomers | gum |
| 2 | H | H | H | 4-F | H | 2 | " | 1 | H | H | E | 43-102° |
| 3 | H | H | H | 4-F | H | 2 | " | 1 | H | Na | E | 210-220° (dec.) |
| 6 | 4-Cl | H | H | 4-F | H | 2 | " | 1 | H | $C_2H_5$ | E | 121-124° |

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|---|---|
| 7 | 4-Cl | H | H | 4-F | H | 2 | (E)CH=CH | 1 | H | H | E | oil |
| 8 | 4-Cl | H | H | 4-F | H | 2 | " | 1 | H | Na | E | 201-204°(dec.) |
| 11 | H | H | H | 4-F | H | 2 | " | 1 | H | Na | E(3R,5S) | 215-220°(dec.) |
| 13 | 3-i$C_3H_7$ | H | H | 4-F | H | 1 | " | 2 | H | $C_2H_5$ | E | gummy foam |
| 14 | 3-i$C_3H_7$ | H | H | 4-F | H | 1 | " | 2 | H | H | E | |

| cmpd. no. | $R_1$ | $R_2$ | $R_3$ | $R_4$ | $R_5$, $R_{5a}$ | Position of $R_4$-bearing Phenyl Group | -X- | Posi'ion of -^-Z Group | $R_6$ | $R_7$ | Isomer | m.p. |
|---|---|---|---|---|---|---|---|---|---|---|---|---|
| 15 | 3-i$C_3H_7$ | H | H | 4-F | H | 1 | (E)CH=CH | 2 | H | Na | E | foam |
| 17 | 3-$CH_3$ | H | H | 4-F | H | 1 | " | 2 | H | $C_2H_5$ | E | 121-124° |
| 18 | 3-$CH_3$ | H | H | 4-F | H | 1 | " | 2 | H | Na | E | 222-226°(dec.) |
| 19 | 3-$C_2H_5$ | H | H | 4-F | H | 1 | " | 2 | H | $C_2H_5$ | E | 101-106° |
| 20 | 3-$C_2H_5$ | H | H | 4-F | H | 1 | " | 2 | H | H | E | oil |

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|---|---|
| 21 | 3-$C_2H_5$ | H | H | 4-F | H | 1 | (E)CH=CH | 2 | H | Na | E | > 230°(dec.) |
| 23 | H | H | H | 4-F | H | 2 | $-CH_2CH_2-$ | 1 | H | $C_2H_5$ | E | Gum |
| 24 | H | H | H | 4-F | H | 2 | " | 1 | H | Na | E | 180-190° |
| 25 | H | H | H | 4-F | H | 2 | " | 1 | H | H | E | Solid foam |
| 26 | 4-Cl | H | H | 4-F | H | 2 | (E)CH=CH | 1 | H | K | E | |
| 27 | 4-$CH_3$ | H | H | 4-F | H | 2 | " | 1 | H | $C_2H_5$ | E | |

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|---|---|
| 28 | 4-$CH_3$ | H | H | 4-F | H | 2 | (E)CH=CH | 1 | H | Na | E | |
| 29 | 4-$CH_3$ | H | H | 4-F | H | 2 | " | 1 | H | H | E | |
| 30 | 3-$CH_3$ | H | H | 4-F | H | 1 | " | 2 | H | K | E | |
| 31 | 3-$CH_3$ | H | H | 4-F | H | 1 | " | 2 | H | H | E | |
| 32 | 3-$CH_3$ | H | H | 4-F | H | 1 | $-CH_2CH_2-$ | 2 | H | $C_2H_5$ | E | |

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|---|---|
| 33 | 3-$CH_3$ | H | H | 4-F | H | 1 | $-CH_2CH_2-$ | 2 | H | Na | E | |
| 34 | 3-$CH_3$ | H | H | 4-F | H | 1 | $-CH_2CH_2-$ | 2 | H | H | E | |
| 35 | 1-$CH_3$ | H | H | 4-F | H | 3 | (E)CH=CH | 2 | H | $C_2H_5$ | E | |
| 36 | 1-$CH_3$ | H | H | 4-F | H | 3 | " | 2 | H | K | E | |
| 37 | 1-$CH_3$ | H | H | 4-F | H | 3 | " | 2 | H | H | E | |
| 38 | 1-$CH_3$ | H | H | 4-F | H | 3 | $-CH_2CH_2-$ | 2 | H | $CH_3$ | E | |

| Cmpd. No. | $R_1$ | $R_2$ | $R_3$ | $R_4$ | $R_5$, $R_{5a}$ | Position of $R_1$-bearing Phenyl Group | -X- | Pos. .ion of -X-Z Group | $R_6$ | $R_7$ | Isomer | m.p. |
|---|---|---|---|---|---|---|---|---|---|---|---|---|
| 39 | 1-$CH_3$ | H | H | 4-F | H | 3 | $-CH_2CH_2-$ | 2 | H | Na | E | |
| 40 | 1-$CH_3$ | H | H | 4-F | H | 3 | $-CH_2CH_2-$ | 2 | H | H | E | |
| 41 | H | H | H | H | H | 2 | (E)CH=CH | 1 | H | $C_2H_5$ | E | |
| 42 | H | H | H | H | H | 2 | " | 1 | H | K | E | |
| 43 | H | H | H | H | H | 2 | " | 1 | H | H | E | |

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|---|---|
| 44 | H | $6-OC_6H_5$ | $7-CH_3$ | $3-CF_3$ | H | 2 | $-CH_2-$ | 1 | $CH_3$ | $CH_3$ | E | |
| 45 | H | $6-OC_6H_5$ | $7-CH_3$ | $3-CF_3$ | H | 2 | $-CH_2-$ | 1 | $CH_3$ | Na | E | |
| 46 | H | $6-OC_6H_5$ | $7-CH_3$ | $3-CF_3$ | H | 2 | $-CH_2-$ | 1 | $CH_3$ | H | E | |
| 47 | H | H | H | 4-F | H | 1 | (E)CH=CH | 2 | H | $C_2H_5$ | E | Oil |
| 48 | H | H | H | 4-F | H | 1 | " | 2 | H | Na | E | >220°C. (dec.) |
| 49 | H | H | H | 4-F | H | 1 | $-CH_2CH_2-$ | 2 | H | $C_2H_5$ | E | Oil |

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|---|---|
| 50 | H | H | H | 4-F | H | 1 | $-CH_2CH_2-$ | 2 | H | Na | E | >190°C. (dec.) |
| 51 | H | H | H | 4-F | H | 3 | (E)CH=CH | 2 | H | $C_2H_5$ | E | Gum |
| 52 | H | H | H | 4-F | H | 3 | " | 2 | H | H | E | Solid foam |
| 53 | H | H | H | 4-F | H | 3 | " | 2 | H | Na | E | 225°-230°C (dec. |

TABLE II

The following compounds of Groups IAb1-IAb3 may be synthesized by the processes set forth above:

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | Isomer | m.p. |
|---|---|---|---|---|---|---|---|---|---|---|---|
| 4 | H | H | H | 4-F | H | 2 | (E)CH=CH | 1 | H | trans | 152-154° (no detectable cis 153-156°) |
| 9 | 4-Cl | H | H | 4-F | H | 2 | " | 1 | H | trans | 131-134° |
| 10 | H | H | H | 4-F | H | 2 | " | 1 | H | trans (6S,4R) | 184-186° |
| 16 | 3-i$C_3H_7$ | H | H | 4-F | H | 1 | " | 2 | H | trans | foam |
| 22 | 3-$C_2H_5$ | H | H | 4-F | H | 1 | " | 2 | H | trans | 118-122° |

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | Isomer | m.p. |
|---|---|---|---|---|---|---|---|---|---|---|---|
| 54 | H | H | H | 4-F | H | 2 | $-CH_2CH_2-$ | 1 | H | trans | Solid foam |
| 55 | $4-CH_3$ | H | H | 4-F | H | 2 | (E)CH=CH | 1 | H | trans | |
| 56 | $3-CH_3$ | H | H | 4-F | H | 1 | " | 2 | H | trans | |
| 57 | $3-CH_3$ | H | H | 4-F | H | 1 | $-CH_2CH_2-$ | 2 | H | trans | |
| 58 | $1-CH_3$ | H | H | 4-F | H | 3 | (E)CH=CH | 2 | H | trans | |
| 59 | $1-CH_3$ | H | H | 4-F | H | 3 | $-CH_2CH_2-$ | 2 | H | trans | |

| Cmpd. No. | $R_1$ | $R_2$ | $R_3$ | $R_4$ | $R_5$, $R_{5a}$ | Position of $R_4$-bearing Phenyl Group | -X- | Position of -X-Z Group | $R_6$ | Isomer | m.p. |
|---|---|---|---|---|---|---|---|---|---|---|---|
| 60 | H | H | H | H | H | 2 | (E)CH=CH | 1 | H | trans | |
| 61 | H | 6-$OC_6H_5$ | 7-$CH_3$ | 3-$CF_3$ | H | 2 | $-CH_2-$ | 1 | H | trans | |
| 62 | H | H | H | 4-F | H | 2 | (E)CH=CH | 1 | H | cis | 143°-146°C. |
| 63 | H | H | H | 4-F | H | 2 | " | 1 | H | mixture: ~53% cis ~47% trans | 134°-137°C. |
| 64 | H | H | H | 4-F | H | 1 | $-CH_2CH_2-$ | 2 | H | trans | Oil |
| 65 | H | H | H | 4-F | H | 3 | (E)CH=CH | 2 | H | trans | Solid foam |

## Table III

The following compounds of Groups IBa1-IBa3 may be synthesized by the processes set forth above:

| Cmpd. No. | $R_1$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|
| 5 | H | 4-F | H | 2 | $-CH_2CH_2-$ | 1 | H | $C_2H_5$ | E | Gum |
| 66 | H | 4-F | H | 2 | $-CH_2CH_2-$ | 1 | H | K | E | |
| 67 | H | 4-F | H | 2 | $-CH_2CH_2-$ | 1 | H | H | E | |
| 68 | H | 4-F | H | 2 | (E)CH=CH | 1 | H | $C_2H_5$ | E | Gum |
| 69 | H | 4-F | H | 2 | " | 1 | H | Na | E | 220°-225°C. (dec.) |
| 70 | H | 4-F | H | 2 | " | 1 | H | H | E | |
| 71 | 4-Cl | 4-F | H | 2 | " | 1 | H | $C_2H_5$ | E | |

| Cmpd. No. | $R_1$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|
| 72 | 4-Cl | 4-F | H | 2 | (E)CH=CH | 1 | H | K | E | |
| 73 | 4-Cl | 4-F | H | 2 | " | 1 | H | H | E | |
| 74 | 4-$CH_3$ | 4-F | H | 2 | " | 1 | H | $C_2H_5$ | E | |
| 75 | 4-$CH_3$ | 4-F | H | 2 | " | 1 | H | Na | E | |
| 76 | 4-$CH_3$ | 4-F | H | 2 | " | 1 | H | H | E | |
| 77 | 3-$CH_3$ | 4-F | H | 1 | " | 2 | H | $C_2H_5$ | E | |

| Cmpd. No. | $R_1$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|
| 78 | 3-$CH_3$ | 4-F | H | 1 | (E)CH=CH | 2 | H | K | E | |
| 79 | 3-$CH_3$ | 4-F | H | 1 | " | 2 | H | H | E | |
| 80 | 3-$CH_3$ | 4-F | H | 1 | $-CH_2CH_2-$ | 2 | H | $C_2H_5$ | E | |
| 81 | 3-$CH_3$ | 4-F | H | 1 | $-CH_2CH_2-$ | 2 | H | Na | E | |
| 82 | 3-$CH_3$ | 4-F | H | 1 | $-CH_2CH_2-$ | 2 | H | H | E | |
| 83 | 1-$CH_3$ | 4-F | H | 3 | (E)CH=CH | 2 | H | $C_2H_5$ | E | |
| 84 | 1-$CH_3$ | 4-F | H | 3 | " | 2 | H | K | E | |

| Cmpd. No. | $R_1$ | $R_4$ | $R_5$ $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|
| 85 | 1-$CH_3$ | 4-F | H | 3 | 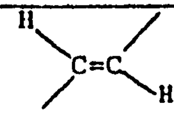 | 2 | H | H | E | |
| 86 | 1-$CH_3$ | 4-F | H | 3 | $-CH_2CH_2-$ | 2 | H | $C_2H_5$ | E | |
| 87 | 1-$CH_3$ | 4-F | H | 3 | $-CH_2CH_2-$ | 2 | H | Na | E | |
| 88 | 1-$CH_3$ | 4-F | H | 3 | $-CH_2CH_2-$ | 2 | H | H | E | |
| 89 | H | H | H | 2 | 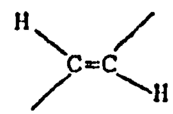 | 1 | H | $C_2H_5$ | E | |
| 90 | H | H | H | 2 | 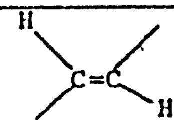 | 1 | H | K | E | |
| 91 | H | H | H | 2 | 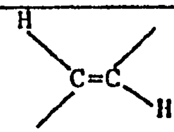 | 1 | H | H | E | |

| Cmpd. No. | $R_1$ | $R_4$ | $R_5$, $R_{5a}$ | Position of $R_4$-bearing Phenyl Group | -X- | Position of -X-Z Group | $R_6$ | $R_7$ | Isomer | m.p. |
|---|---|---|---|---|---|---|---|---|---|---|
| 92 | H | 3-$CH_3$ | 4-$OCH_3$ | 2 | -$CH_2$- | 1 | $CH_3$ | $CH_3$ | E | |
| 93 | H | 3-$CH_3$ | 4-$OCH_3$ | 2 | -$CH_2$- | 1 | $CH_3$ | Na | E | |
| 94 | H | 3-$CH_3$ | 4-$OCH_3$ | 2 | -$CH_2$- | 1 | $CH_3$ | H | E | |
| 95 | H | 4-F | H | 3 | (E)CH=CH | 2 | H | $C_2H_5$ | E | Gum |
| 96 | H | 4-F | H | 3 | " | 2 | H | H | E | Solid foam |
| 97 | H | 4-F | H | 3 | " | 2 | H | Na | E | 215°-230°C. (dec.) |

~~600-6952~~

Table IV

The following compounds of Groups IBb1-IBb3 may be synthesized by the processes set forth above:

| Cmpd. No. | $R_1$ | $R_4$ | $R_5$, $R_{5a}$ | Position of $R_4$-bearing Phenyl Group | -X- | Position of -X-Z Group | $R_6$ | Isomer | m.p. |
|---|---|---|---|---|---|---|---|---|---|
| 98 | H | 4-F | H | 2 | $-CH_2CH_2-$ | 1 | H | trans | 188°-191°C. |
| 99 | H | 4-F | H | 2 | 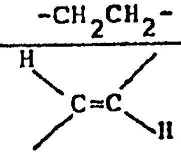 | 1 | H | trans | |
| 100 | 4-Cl | 4-F | H | 2 | 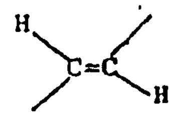 | 1 | H | trans | |
| 101 | $4-CH_3$ | 4-F | H | 2 | 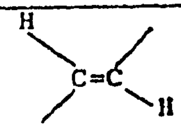 | 1 | H | trans | |
| 102 | $3-CH_3$ | 4-F | H | 1 | 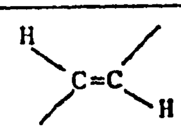 | 2 | H | trans | |
| 103 | $3-CH_3$ | 4-F | H | 1 | $-CH_2CH_2-$ | 2 | H | trans | |
| 104 | $1-CH_3$ | 4-F | H | 3 | 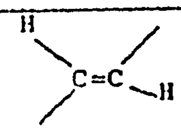 | 2 | H | trans | |

| Cmpd. No. | $R_1$ | $R_4$ | $R_5$, $R_{5a}$ | Position of $R_4$-bearing Phenyl Group | -X- | Position of -X-Z Group | $R_6$ | Isomer | m.p. |
|---|---|---|---|---|---|---|---|---|---|
| 105 | 1-$CH_3$ | 4-F | H | 3 | -$CH_2CH_2$- | 2 | H | trans | |
| 106 | H | H | H | 2 | (E)CH=CH | 1 | H | trans | |
| 107 | H | 3-$CH_3$ | 4-$OCH_3$ | 2 | -$CH_2$- | 1 | $CH_3$ | trans | |
| 108 | H | 4-F | H | 2 | (E)CH=CH | 1 | H | cis | |
| 109 | H | 4-F | H | 3 | " | 2 | H | trans | Solid foam |
| 110 | H | 4-F | H | 2 | " | 1 | H | trans | 168°-172°C. |

In Tables I and III,

E = erythro racemate (two stereoisomers unless otherwise stated)

In Tables II and IV,

cis and trans refer to the relative positions of the $R_6$ group in the 4-position and the hydrogen atom in the 6-position of the lactone ring (racemates unless otherwise stated)

The principal component of each of Compounds 23-25, 32-34, 38-40, 44-46, 49, 50, 66, 67, 80-82, 86-88 and 92-94 is the erythro racemate which may be resolved, the two enantiomers being the 3R,5R and 3S,5S isomers. The minor component (usually about 1-15%) of each example is the corresponding threo racemate which may be separated therefrom and resolved to obtain the 3R,5S and 3S,5R isomers. If, however, a nonstereoselective process were utilized in process a) to reduce the 3-oxo group to the 3-hydroxy group, a resolvable mixture containing approximately equal amounts of the four stereoisomers would be obtained. Preferred are the 3R,5R and 3R,5S isomers and the racemate of which each is a constituent, viz., the 3R,5R-3S,5S and 3R,5S-3S,5R racemates, with the 3R,5R isomer and the 3R,5R-3S,5S racemate being more preferred.

The principal component of each of Compounds 26-31, 35-37, 41-43, 47, 48, 51-53, 68-79, 83-85, 89-91 and 95-97 is likewise the erythro racemate which may be resolved into two optically pure enantiomers, viz., the 3R,5S and 3S,5R isomers. The minor component (usually about 1-15%) of each example is the corresponding threo racemate which may be separated therefrom and resolved to obtain the 3R,5R and 3S,5S isomers. If, however, a non-stereoselective process were utilized in process a) to reduce the 3-oxo group to the 3-hydroxy group, a resolvable mixture containing approximately equal amounts of the four stereoisomers would be obtained. Preferred are the 3R,5R and 3R,5S isomers and the racemate of which each is a constituent, viz., the 3R,5R-3S,5S and 3R,5S-3S,5R racemates, with the 3R,5S isomer and the 3R,5S-3S,5R racemate being more preferred.

The principal component of each of Compounds 54, 57, 59, 61, 64, 98, 103, 105 and 107 is the trans racemate which may be resolved into two optically pure enantiomers, viz., the 4R,6R and 4S,6S isomers. The minor component (usually about 1-15%) of each example is the corresponding cis racemate which may be separated therefrom and resolved to obtain the 4R,6S and 4S,6R isomers. The use in process e) of a mixture containing approximately equal amounts of the four stereoisomeric carboxylic acids would yield a mixture containing approximately equal amounts of the four stereoisomeric lactones. Preferred are the 4R,6R and 4R 6S isomers and the racemate of which each is a constituent, with the 4R 6R isomer and the 4R,6R-4S,6S racemate being more preferred.

The principal component of each of Compounds 55, 56, 58, 60, 65, 99-102, 104, 106, 109 and 110 is the trans racemate which may be resolved into two optically pure enantiomers, viz., the 4R,6S and 4S,6R isomers. The minor component (usually about 1-15%) of each example is the corresponding cis racemate which may be separated therefrom and resolved to obtain the 4R,6R and 4S,6S isomers. The use in process e) of a mixture containing approximately equal amounts of the four stereoisomeric carboxylic acids would yield a mixture containing approximately equal amounts of the four stereoisomeric lactones as in Compound 63. Preferred are the 4R,6R and 4R,6S isomers and the racemate of which each is a constituent, with the 4R,6S isomer and the 4R,6S-4S,6R racemate being more preferred.

The principal component of each of Compounds 62 and 108 is the cis racemate which may be resolved into two optically pure enantiomers, viz., the 4R,6R and 4S,6S isomers. The minor component (usually about 1-15%) of each example is the corresponding trans racemate which may be separated therefrom and resolved to obtain the 4R,6S and 4S,6R isomers. The preferred isomers are as indicated in the preceding paragraph.

Each of the compounds of the examples wherein $R_7$ is a cation may be converted into the corresponding free acid and into the corresponding compounds wherein $R_7$ is a different M by conventional means.

Throughout the examples, the term "reduced pressure" denotes aspirator pressure, and where no solvent is specified in connection with a solution, the solvent is water. All solvent mixtures are by volume.

The following data were obtained for the preceding compounds. Unless otherwise stated the data are NMR spectra measured at 200 mHz. Shifts are in ppm. relative to tetramethylsilane.

**Abbreviations**

s = singlet
d = doublet
dd = doublet of a doublet
t = triplet
q = quartet
m = multiplet
br = broad
bm = broad multiplet
bs = broad singlet

Cmpd. No.

2 $CDCl_3$ - (90mHz); 1.59(m,2H); 2.53(d,2H,J=2.5Hz); 4.24(m,1H); 4.52(m,1H); 5.62(m,1H); 6.83(m,1H); 7.23(m,7H); 7.79(m,2H); 8.13(m,1H).

5 $CDCl_3$: 1.23(t,3H,J=1.5Hz); 1.42(m,6H); 1.8(m,2H); 2.4(m,2H); 2.8(m,4H); 3.12(m,2H): 3.78(m,2H); 4.16(q,2H,J=1.5Hz); 7.15(m,4H); 7.52(m,1H); 7.82(m,1H).

12 $CDCl_3$: 1.28(t,3H,J=1.5Hz); 1.6(m,2H); 2.43(m,2H); 2.95(br,2H); 4.2(q,2H,J=1.5Hz); 4.2(br,1H); 4.51(br,1H); 5.65(m,1H); 6.9(dd,1H,J=3 and 3 Hz); 7.07 (t,2H,J=1.5Hz); 7.41(m,5H); 7.82 (m,2H); 8.17(m,1H).

13 $CDCl_3$: 1.31(m,11H); 2.41(m,2H); 2.88(s,1H); 3.32(m,1H); 3.61(s,1H); 4.09(m,1H); 4.19(q,2H,J=1Hz); 4.33 (m,1H); 5.28(m,1H); 6.54(d,1H,J=2Hz); 7.23(m,7H); 7.77(m,2H).

14 $CDCl_3$: 1.3 (m,8H); 2.49(d,2H); 3.3(m,1H); 3.57(bm,1H); 4.12(bm,2H); 4.36(m,1H); 5.3(dd,1H,J=1.5Hz); 6.56(d,1H,J=3Hz); 7.25(m,8H); 7.78(m,2H).

15 $D_2O$: 0.9(m,7H); 1.27(m,1H); 2.03(m,2H); 3.0(m,1H); 3.43(m,1H); 3.97(m,1H); 4.9(m,1H); 6.29(d,1H,J=2Hz); 6.72(m,7H); 7.26(m,2H);

16 $CDCl_3$: 1.3(m,6H); 1.45(bm,2H); 1.82(bs,1H); 2.61(m,2H); 3.3(m,1H); 4.12(m,1H); 5.1(m,1H); 5.31(dd.1H,J=1Hz); 6.64(d,1H,J=2.5Hz); 7.25(m,7H); 7.78(m,2H).

18 $CD_3SOCD_3$: 1.1(m,2H); 1.85(m,2H); 2.5(s,3H); 3.5(m,1H); 4.1(m,1H); 5.4(q,1H,J=1.25Hz); 6.3(d,1H,J=3.5Hz); 7.3(m,7H); 7.83(m,2H).

21 $CDCl_3$ + $CD_3OD$: 1.25(m,2H); 1.35(t,3H,J=1.5Hz); 2.25(m,2H); 2.89(q,2H,J=1.5Hz); 3.88(m,1H); 4.27(m,1H); 5.39(q,1H,J=1.5Hz); 6.52(d,1H,J=3Hz); 7.25(m,5H); 7.72(m,4H).

Cmpd. No.

23 $CDCl_3$: 1.29(t,3H,J=1.5Hz); 1.80(m,4H); 2.43(m,2H); 3.13(m,2H); 3.92(m,2H); 4.20(q,2,J=1.5Hz); 7.25(m,5H); 7.56(t,2H,J=1.5Hz); 7.75(d,1H,J=1.5Hz); 7.90(d,1H,J=1.5Hz); 8.17(m,1H).

25 $CDCl_3$: 1.45(m,2H); 1.80(m,2H); 2.45(m,2H); 3.10(m,2H); 3.95(bm,2H); 7.6(bm,10H).

47 $CDCl_3$: 1.26(t,3H,J=1.5Hz); 1.75(m,2H); 2.49(d,2H,J=1.33Hz); 3.13(d,1H,J=0.5Hz); 3.66(d,1H,J=0.5Hz); 4.17(q,2H,J=1.5Hz); 4.25(m,1H); 4.45(m,1H); 6.23(dd,1H,J=1.5 and 2 Hz), 6.47(d,1H,J=3.5Hz); 7.25(m,4H); 7.4(m,3H); 7.8(m,3H).

49 $CDCl_3$: 1.23(t,3H,J=1.5Hz); 1.40(m,2H); 1.65(m,2H); 2.41(d,2H, J=1.5Hz); 2.60(m,2H); 3.25(d,1H,J=0.5Hz); 3.70(d,1H,J=0.5Hz); 3.75(m,1H); 4.15(q,2H,J=1.5Hz); 4.2(m,1H); 7.3(m,8H); 7.83(m,2H).

51 $CDCl_3$; 1.27(t,3H,J=1.5Hz); 1.72(m,2H); 2.5(d,2H,J=1.5Hz); 3.22(s,1H); 3.69(s,1H); 4.15(q,2H,J=1.5Hz); 4.27(m,1H); 4.51(m,1H); 6.22(dd,1H,J=3 and 1.5 Hz); 6.61(d,1H,J=3Hz); 7.11(t,2H,J=2Hz); 7.38(m,4H); 7.67(s,1H); 7.78(m,2H); 7.97(s,1H).

52 $CDCl_3$: 1.73(m,2H); 2.55(d,2H,J=1.5Hz); 4.3(m,1H); 4.5(m,1H); 6.2(dd,1H,J=3 and 1.5 Hz); 6.6(d,1H,J=3Hz); 7.1(m,2H); 7.4(m,4H); 7.66(s,1H); 7.78(m,2H); 7.97(s,1H).

54 $CDCl_3$: 1.82(m,4H); 2.67(m,2H); 3.05(m,1H); 3.26(m,1H); 4.32(m,1H); 4.61(m,1H); 7.10(m,2H); 7.28(m,3H); 7.51(m,2H); 7.72(d,1H,J=1.5Hz); 7.86(m,1H); 8.11(d,1H,J=1.5Hz).

64 $CDCl_3$: 1.7(m,2H); 1.7(m,2H); 1.75(s,1H); 2.7(m,2H); 2,7(m,2H); 4.35(m,1H); 4.56(m,1H); 7.3(m,8H); 7.85(d,2H).

Cmpd.No.

65 $CDCl_3$: 2.0(m,2H); 2.71(m,2H); 4.41(m,1H); 5.29(m,1H); 6.23(dd,1H,J=3 and 1.5 Hz); 6.7(d,1H,J=3Hz); 7.13(m,2H); 7.41(m,4H); 7.68(s,1H); 7.79(m,2H); 7.98(s,1H).

68 $CDCl_3$: 1.3(t,3H,J=1.5Hz); 1.75(m,6H); 2.43(m,2H); 2.78(m,4H); 4.18(m,3H); 4.37(m,1H); 5.25(dd,1H,J=3 and 1.5 Hz); 6.5(d,1H,J=3Hz); 7.02(m,3H); 7.22(m,3H).

95 $CDCl_3$: 1.24(t,3H,J=1.5Hz); 1.76(m,6H); 2.5(m,2H); 2.8(m,4H); 4.17(m,3H); 4.14(m,1H); 6.06(dd,1H,J=3 and 1.5 Hz); 6.5(d,1H,J=3Hz); 7.1(m,6H).

96 $CDCl_3$: 1.83(m,6H); 2.69(m,6H); 4.26(m,1H); 4.43(m,1H); 6.06(m,1H); 6.52(m,1H); 7.12(m,6H).

109 $CDCl_3$: 1.96(m,6H); 2.73(m,6H); 4.4(m,1H); 5.22(m,1H); 6.1(dd,1H,J=3 and 1.5 Hz); 6.57(d,1H,J=3Hz); 7.17(m,6H).

**Claims**

for the Contracting States: BE, CH, DE, FR, GB, IT, LI, LU, NL, SE.

1. A compound of formula I

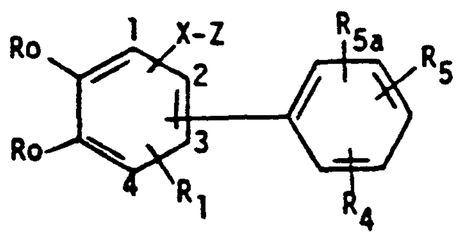

I

wherein the two groups Ro together form a radical of formula

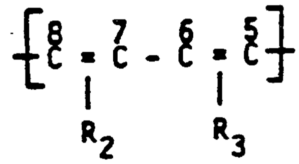 or $-(CH_2)_4-$ ,

wherein $R_2$ is hydrogen, $C_{1-4}$alkyl, $C_{1-3}$alkoxy, n-butoxy, i-butoxy, s-butoxy, trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the provisos that not more than one of $R_2$ and $R_3$ is trifluoromethyl, not more than one of $R_2$ and $R_3$ is phenoxy, and not more than one of $R_2$ and $R_3$ is benzyloxy,

$R_1$ is hydrogen, $C_{1-6}$alkyl, fluoro, chloro or benzyloxy,

$R_4$ is hydrogen, $C_{1-4}$alkyl, $C_{1-3}$alkoxy, n-butoxy, i-butoxy, s-butoxy, trifluoromethyl, fluoro, chloro, phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_{5a}$ is hydrogen, $C_{1-2}$alkyl, $C_{1-2}$alkoxy, fluoro or chloro, with the provisos that not more than one of $R_4$ and $R_5$ is trifluoromethyl, not more than one of $R_4$ and $R_5$ is phenoxy and not more than one of $R_4$ and $R_5$ is benzyloxy,

X is $-(CH_2)_n-$ or 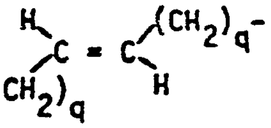

wherein n is 0, 1, 2 or 3 and
both q's are 0 or one is 0 and the other is 1, and

Z is 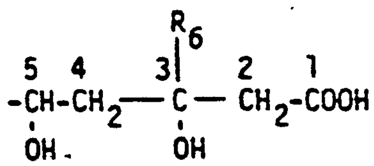 II

wherein $R_6$ is hydrogen or $C_{1-3}$alkyl, with the general proviso that -X-Z and the $R_4$-bearing phenyl group are ortho to each other;

in free acid form or in the form of a physiologically-hydrolysable and -acceptable ester or a δ lactone thereof or in salt form.

2. A compound according to Claim 1, wherein the two Ro groups together form a radical of the formula

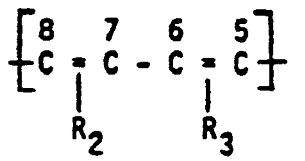 or $-CH_2CH_2CH_2CH_2-$,

wherein $R_2$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy,

with the provisos that not more than one of $R_2$ and $R_3$ is trifluoromethyl, not more than one of $R_2$ and $R_3$ is phenoxy, and not more than one of $R_2$ and $R_3$ is benzyloxy,

$R_1$ is hydrogen, $C_{1-3}$alkyl, fluoro, chloro or benzyloxy,

$R_4$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_{5a}$ is hydrogen,

with the proviso that not more than one of $R_4$ and $R_5$ is trifluoromethyl, not more than one of $R_4$ and $R_5$ is phenoxy, and not more than one of $R_4$ and $R_5$ is benzyloxy,

X is $-(CH_2)_n-$, 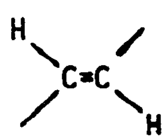 , 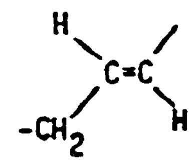 or 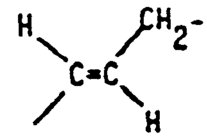 ,

wherein n is 0, 1, 2 or 3, and

Z is 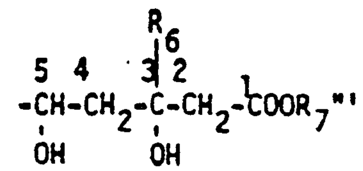 or

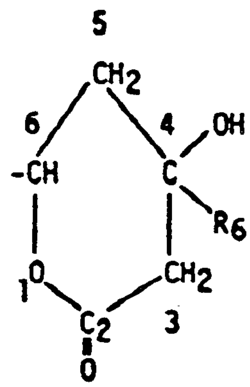

IIb .

wherein $R_6$ is hydrogen or $C_{1-3}$alkyl, and

$R_7'''$ is hydrogen, $C_{1-3}$alkyl, n-butyl, i-butyl, t-butyl, benzyl or M, wherein M is a pharmaceutically acceptable cation,

with the proviso that the -X-Z group and the $R_4$-bearing phenyl group are ortho to each other.

3. A compound according to Claim 1 wherein Ro + Ro is as defined in Claim 1,

$R_1$ is hydrogen or $C_{1-3}$alkyl,

$R_2$, $R_3$, $R_4$ and $R_5$ are independently hydrogen, methyl, methoxy, fluoro or chloro,

$R_{5a}$ is hydrogen,

X-Z is in 1-position and represents

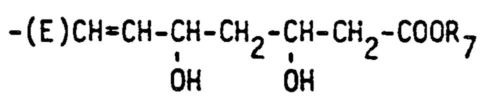

wherein $R_7$ is hydrogen, $C_{1-2}$alkyl or a pharmaceutically acceptable cation, and the $R_4$ bearing group is in 2-position.

4. A compound according to Claim 3, wherein X-Z is in 2-position and the $R_4$ bearing group is in 1-position.

5. A compound according to Claim 1, wherein the substituents have the following meanings

a) Z is a group IIa in erythro form, Ro + Ro =

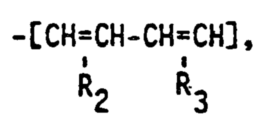 ,

$R_2$, $R_3$, $R_5$, $R_{5a}$ and $R_6$ are hydrogen, X-Z is in 1-position, the $R_4$ bearing group is in 2-position and

(i) X is (E)CH=CH, $R_4$ is 4-F and

$R_1$ is hydrogen or 4-methyl and $R_7$ is ethyl, sodium or hydrogen; or
$R_1$ is 4-chloro and $R_7$ is ethyl, potassium or hydrogen;
or (ii) X is (E)CH=CH, $R_4$ and $R_1$ are hydrogen and $R_7$ is ethyl, potassium or hydrogen;
or (iii) X is $CH_2CH_2$, $R_4$ is 4-F,
$R_1$ is hydrogen and $R_7$ is ethyl, sodium or hydrogen:
b) as a) except X-Z is in 2-position, the $R_4$ bearing group is in 1-position and
(i) X is (E)CH=CH, $R_4$ is 4-F, $R_1$ is 3-methyl and $R_7$ is ethyl, potassium or hydrogen;
or (ii) X is $CH_2CH_2$, $R_4$ is 4-F, $R_1$ is 3-methyl and $R_7$ is ethyl, sodium or hydrogen:
c) as a) except X-Z is in 2-position, the $R_4$ bearing group is in 3-position and
(i) X is (E)CH=CH, $R_4$ is 4-F,
$R_1$ is 1-methyl and $R_7$ is ethyl, potassium or hydrogen;
or (ii) X is $CH_2CH_2$, $R_4$ is 4-F,
$R_1$ is hydrogen and $R_7$ is methyl, sodium or hydrogen:
d) as a) except that Z is a group IIb in trans form and
(i) X is (E)CH=CH, $R_4$ is 4-F and
$R_1$ is hydrogen, 4-fluoro or 4-methyl;
or (ii) X is (E)CH=CH and $R_4$ and $R_1$ are hydrogen;
or (iii) X is $CH_2CH_2$, $R_4$ is 4-F and $R_1$ is hydrogen:
e) as b) except that Z is a group IIb in trans form and $R_4$ is 4-F, $R_1$ is 3-methyl and X is (E)CH=CH or $CH_2CH_2$:
f) as c) except that Z is a group IIb in trans form and $R_4$ is 4-F,
$R_1$ is 1-methyl and X is (E)CH=CH or $CH_2CH_2$:
g) as a) except that Z is a group IIb in cis form, X is (E)CH=CH,
$R_4$ is 4-F and $R_1$ is hydrogen:
h) as a) except that Ro + Ro = $(CH_2)_4$ and
(i) X is (E)CH=CH, $R_4$ is 4-F and
$R_1$ is hydrogen or 4-methyl and $R_7$ is ethyl, sodium or hydrogen;
or $R_1$ is 4-chloro and $R_7$ is ethyl, potassium or hydrogen;
or (ii) X is $CH_2CH_2$, $R_4$ is 4-F, $R_1$ is hydrogen and $R_7$ is ethyl, potassium or hydrogen;
or (iii) X is (E)CH=CH, $R_4$ and $R_1$ are hydrogen and $R_7$ is ethyl, potassium or hydrogen:
i) as b) except that Ro + Ro = $(CH_2)_4$ and
(i) X is (E)CH=CH, $R_4$ is 4-F,
$R_1$ is 3-methyl and $R_7$ is ethyl, potassium or hydrogen;
or (ii) X is $CH_2CH_2$, $R_4$ is 4-F,
$R_1$ is 3-methyl and $R_7$ is ethyl, sodium or hydrogen:
j) as c) except that Ro + Ro = $(CH_2)_4$ and
(i) X is (E)CH=CH, $R_4$ is 4-F,
$R_1$ is 1-methyl and $R_7$ is ethyl, potassium or hydrogen;
or (ii) X is $CH_2CH_2$, $R_4$ is 4=F,
$R_1$ is 1-methyl and $R_7$ is ethyl, sodium or hydrogen:
k) as d) except that Ro + Ro = $(CH_2)_4$ and
(i) X is (E)CH=CH, $R_4$ is 4-F and $R_1$ is hydrogen; 4-chloro or 4-methyl;
or (ii) X is (E)CH=CH and $R_4$ and $R_1$ are hydrogen,
or (iii) X is $CH_2CH_2$, $R_4$ is 4-F and $R_1$ is hydrogen:
(l) as e) except that Ro + Ro = $(CH_2)_4$, X is (E)CH=CH or $CH_2CH_2$, $R_4$ is 4-F and $R_1$ is 3-methyl:
(m) as f) except that Ro + Ro = $(CH_2)_4$, X is (E)CH=CH or $CH_2CH_2$, $R_4$ is 4-F and $R_1$ is 1-methyl:
(n) as g) except that Ro + Ro = $(CH_2)_4$, X is (E)CH=CH, $R_4$ is 4-fluoro and $R_1$ is hydrogen:
(o) $R_1$, $R_5$ and $R_{5a}$ are hydrogen, $R_2$ is phenoxy, $R_3$ is 7-methyl, $R_4$ is 3-$CF_3$, X is $CH_2$, Ro + Ro is

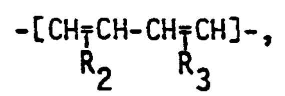

X-Z is in 1-position,
the $R_4$ bearing group is in 2-position and
(i) $R_6$ is methyl, Z is a group IIa in erythro form and $R_7$ is methyl, sodium or hydrogen;
or (ii) $R_6$ is hydrogen and Z is a group IIb in trans form:
(p) $R_1$ and $R_{5a}$ are hydrogen, $R_4$ is 3-methyl, $R_5$ is 4-methoxy, Ro + Ro = $(CH_2)_4$, $R_6$ is methyl, X is $CH_2$, X-Z is in 1-position, the $R_4$ bearing group is in 2-position and
(i) Z is a group IIa in erythro form and $R_7$ is methyl, sodium or hydrogen;
or (ii) Z is a group IIb in trans form.
6. A compound according to Claim 1 wherein
a) Z is a group IIa in erythro form, Ro + Ro =

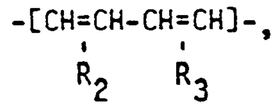

$R_2$, $R_3$, $R_4$, $R_5$, $R_{5a}$ and $R_6$ are hydrogen, $R_4$ is 4-F, X-Z is in 2-position, the $R_4$ bearing group is in 1-position and
(i) X is (E)CH = CH, $R_1$ is 3-ethyl or 3-i-propyl and $R_7$ is ethyl, sodium or hydrogen;
or (ii) X is (E)CH = CH or $CH_2CH_2$, $R_1$ is hydrogen and $R_7$ is ethyl or sodium;
or (iii) X is (E)CH = CH, $R_1$ is 4-chloro or 3-methyl and $R_7$ is sodium:
b) as a) except that X-Z is in 2-position, the $R_4$ bearing group is in 3-position, $R_1$ is hydrogen, X is (E)CH = CH and $R_7$ is ethyl sodium or hydrogen:
c) Z is a group IIb in trans form, Ro + Ro =

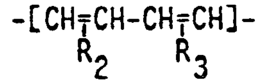 , $R_2$, $R_3$,

$R_2$, $R_3$, $R_5$, $R_{5a}$ and $R_6$ are hydrogen, $R_4$ is 4-F and
(i) X-Z is in 2-position, the $R_4$ bearing group is in 1-position, X is (E)CH = CH and $R_1$ is 3-ethyl; 3-i-propyl,
or (ii) X-Z is in 2-position, the $R_4$ bearing group is in 3-position, X = (E)CH = CH and $R_1$ is hydrogen;
or (iii) X-Z is in 2-position, the $R_4$ bearing group is in 1-position, X is $CH_2CH_2$ and $R_1$ is hydrogen:
d) Z is a group IIa in erythro form, Ro + Ro = $(CH_2)_4$, $R_1$, $R_5$,$R_{5a}$ and $R_6$ are hydrogen, $R_4$ is 4-F, X is (E)CH = CH, X-Z is in 2-position, the $R_4$ bearing group is in 3-position and $R_7$ is ethyl, sodium or hydrogen:
e) Z is a group IIb in trans form Ro + Ro = $(CH_2)_4$, $R_1$, $R_5$, $R_{5a}$ and $R_6$ are hydrogen, $R_4$ is 4-F, X is (E)CH = CH and X-Z is in 1- or 2-position when the $R_4$ bearing group is in 2- or 3-position respectively.

7. Erythro-(E)-3R,5S,dihydroxy-7-(2'-[4"-fluorophenyl]naphth-1'-yl)hept-6-enoate or a salt thereof.

8. Erythro-(E)-3,5-dihydroxy-7-(1'-[4"-fluorophenyl]-3'-[1'-methyl-ethyl]naphth-2'-yl)hept-6-enoate or a salt thereof.

9. (E)-Trans-6-(2'-[3"-ethyl-1"-(4"-fluorophenyl)naphth-2"-yl]-ethenyl)-4-hydroxy-3,4,5,6-tetrahydropyran-2-one.

10. A compound according to Claim 7 or 8 in sodium salt form.

11. A pharmaceutical composition comprising a compound according to any one of Claims 1 to 10 as appropriate in free acid form or in the form of a physiologically-hydrolysable and -acceptable ester or a lactone thereof or in pharmaceutically acceptable salt form, together with a pharmaceutically acceptable diluent or carrier.

12. A compound according to any one of Claims 1 to 10 as appropriate in free acid form or in the form of a physiologically-hydrolysable and -acceptable ester or a lactone thereof or in pharmaceutically acceptable salt form for use in inhibiting cholesterol biosynthesis or treating atherosclerosis.

13. A process for preparing a compound according to Claim 1 which comprises
a) When $R_6$ is hydrogen reducing a compound of formula VI

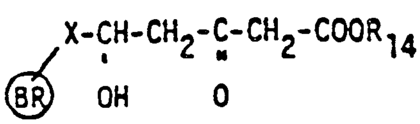 VI

wherein $R_{14}$ is a radical forming a physiologically-hydrolysable and acceptable ester and X, is as defined above,
b) when $R_6$ = $C_{1-3}$alkyl, hydrolysing a compound of formula XVII

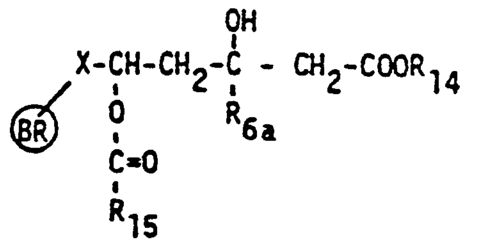

XVII

wherein $R_{6a}$ is $C_{1-3}$alkyl, $R_{15}$ is $C_{1-2}$alkyl and X and $R_{14}$ are as defined above,

c) when X is 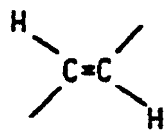 deprotecting a compound of formula LIX

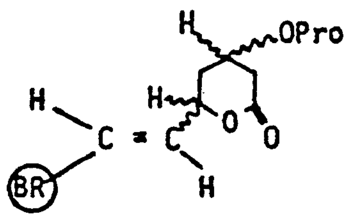

LVIII

wherein Pro is a protecting group
d) hydrolysing a compound of formula I in the form of a physiologicallyhydrolysable ester or a lactone or
e) esterifying or lactonising a compound of formula I in free acid form, and when a free carboxyl group is present, recovering the compound obtained in free acid form or in the form of a salt, whereby

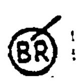 represents the basic ring structure

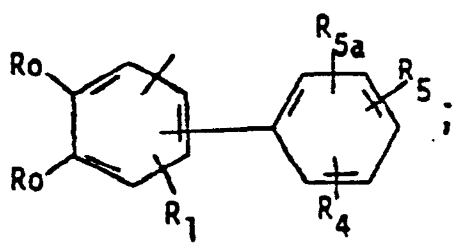

Ro, $R_1$, $R_4$, $R_{5a}$, $R_5$, X and Z as defined in Claim 1.

**Claims**

for the contracting state AT

1. Process for preparing a compound of formula I

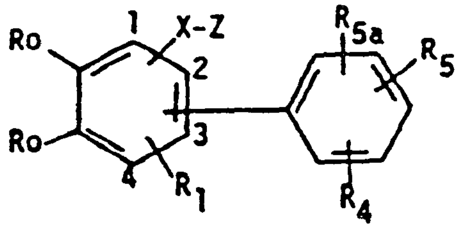

I

wherein the two groups Ro together form a radical of formula

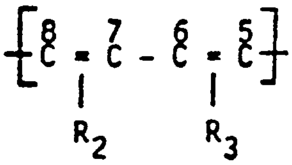

or $-(CH_2)_4-$ ,

wherein $R_2$ is hydrogen, $C_{1-4}$alkyl, $C_{1-3}$alkoxy, n-butoxy, i-butoxy, s-butoxy, trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the provisos that not more than one of $R_2$ and $R_3$ is trifluoromethyl, not more than one of $R_2$ and $R_3$ is phenoxy, and not more than one of $R_2$ and $R_3$ is benzyloxy,

$R_1$ is hydrogen, $C_{1-6}$alkyl, fluoro, chloro or benzyloxy,

$R_4$ is hydrogen, $C_{1-4}$alkyl, $C_{1-3}$alkoxy, n-butoxy, i-butoxy, s-butoxy, trifluoromethyl, fluoro, chloro, phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_{5a}$ is hydrogen, $C_{1-2}$alkyl, $C_{1-2}$alkoxy, fluoro or chloro, with the provisos that not more than one of $R_4$ and $R_5$ is trifluoromethyl, not more than one of $R_4$ and $R_5$ is phenoxy and not more than one of $R_4$ and $R_5$ is benzyloxy,

X is $-(CH_2)_n-$ or

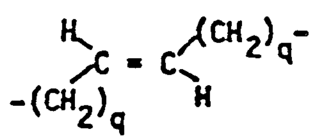

wherein n is 0, 1, 2 or 3 and

both q's are 0 or one is 0 and the other is 1, and

Z is

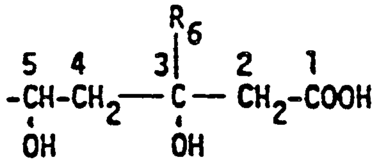

II

wherein $R_6$ is hydrogen or $C_{1-3}$alkyl, with the general proviso that -X-Z and the $R_4$-bearing phenyl group are ortho to each other;

in free acid form or in the form of a physiologically-hydrolysable and -acceptable ester or a δ lactone thereof or in salt form, which comprises

a) When $R_6$ is hydrogen reducing a compound of formula VI

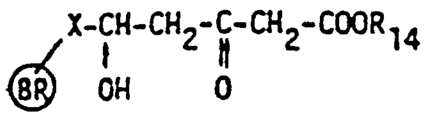

VI

wherein $R_{14}$ is a radical forming a physiologically-hydrolysable and acceptable ester and X, is as defined above,

b) when $R_6 = C_{1-3}$alkyl, hydrolysing a compound of formula XVII

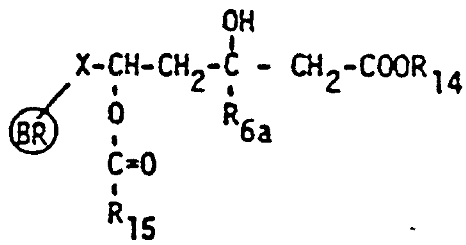

XVII

wherein $R_{6a}$ is $C_{1-3}$alkyl, $R_{15}$ is $C_{1-2}$alkyl and X and $R_{14}$ are as defined above,

c) when X is 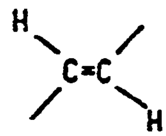 deprotecting a compound of formula LIX

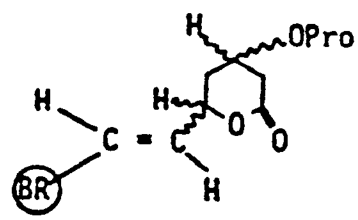

LVIII

wherein Pro is a protecting group

d) hydrolysing a compound of formula I in the form of a physiologicallyhydrolysable ester or a lactone or

e) esterifying or lactonising a compound of formula I in free acid form, and when a free carboxyl group is present, recovering the compound obtained in free acid form or in the form of a salt, whereby

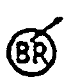 represents the basic ring structure

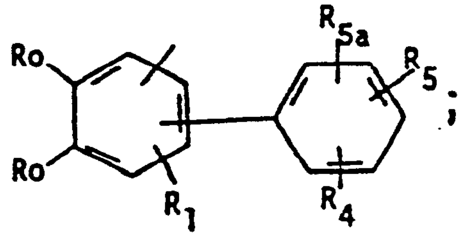

Ro, $R_1$, $R_4$, $R_{5a}$, $R_5$, X and Z as defined in Claim 1.

2. A process according to Claim 1, wherein the two Ro groups together form a radical of the formula

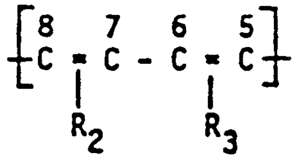 or $-CH_2CH_2CH_2CH_2-$,

wherein $R_2$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the provisos that not more than one of $R_2$ and $R_3$ is trifluoromethyl, not more than one of $R_2$ and $R_3$ is phenoxy, and not more than one of $R_2$ and $R_3$ is benzyloxy,

$R_1$ is hydrogen, $C_{1-3}$alkyl, fluoro, chloro or benzyloxy,

$R_4$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_{5a}$ is hydrogen,

with the proviso that not more than one of $R_4$ and $R_5$ is trifluoromethyl, not more than one of $R_4$ and $R_5$ is phenoxy, and not more than one of $R_4$ and $R_5$ is benzyloxy,

X is $-(CH_2)_n-$,

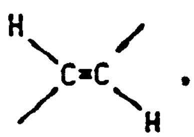 , 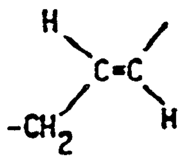 or 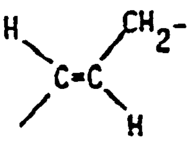 ,

wherein n is 0, 1, 2 or 3, and

Z is

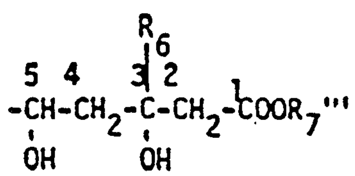 or

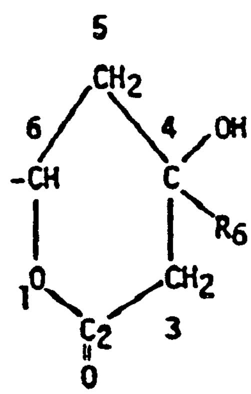 IIb ,

wherein $R_6$ is hydrogen or $C_{1-3}$alkyl, and
$R_7'''$ is hydrogen, $C_{1-3}$alkyl, n-butyl, i-butyl, t-butyl, benzyl or M, wherein M is a pharmaceutically acceptable cation,
with the proviso that the -X-Z group and the $R_4$-bearing phenyl group are ortho to each other.

3. A process according to Claim 1 wherein Ro + Ro is as defined in Claim 1,
$R_1$ is hydrogen or $C_{1-3}$alkyl,
$R_2$, $R_3$, $R_4$ and $R_5$ are independently hydrogen, methyl, methoxy,fluoro or chloro,
$R_{5a}$ is hydrogen,
X-Z is in 1-position and represents

$$-(E)CH{=}CH-\underset{OH}{CH}-CH_2-\underset{OH}{CH}-CH_2-COOR_7$$

wherein $R_7$ is hydrogen, $C_{1-2}$alkyl or a pharmaceutically acceptable cation, and the $R_4$ bearing group is in 2-position.

4. A process according to Claim 3, wherein X-Z is in 2-position and the $R_4$ bearing group is in 1-position.

5. A process according to Claim 1, wherein the substituents have the following meanings
a) Z is a group IIa in erythro form, Ro + Ro =

$$-[\underset{R_2}{CH}{=}CH-\underset{R_3}{CH}{=}CH],$$

$R_2$, $R_3$, $R_5$, $R_{5a}$ and $R_6$ are hydrogen, X-Z is in 1-position,the $R_4$ bearing group is in 2-position and
(i) X is (E)CH=CH, $R_4$ is 4-F and
$R_1$ is hydrogen or 4-methyl and $R_7$ is ethyl, sodium or hydrogen;
or
$R_1$ is 4-chloro and $R_7$ is ethyl, potassium or hydrogen;
or (ii) X is (E)CH=CH, $R_4$ and $R_1$ are hydrogen and $R_7$ is ethyl, potassium or hydrogen;
or (iii) X is $CH_2CH_2$, $R_4$ is 4-F,
$R_1$ is hydrogen and $R_7$ is ethyl, sodium or hydrogen:
b) as a) except X-Z is in 2-position, the $R_4$ bearing group is in 1-position and
(i) X is (E)CH=CH, $R_4$ is 4-F,
$R_1$ is 3-methyl and $R_7$ is ethyl, potassium or hydrogen;
or (ii) X is $CH_2CH_2$, $R_4$ is 4-F,
$R_1$ is 3-methyl and $R_7$ is ethyl, sodium or hydrogen:
c) as a) except X-Z is in 2-position, the $R_4$ bearing group is in 3-position and
(i) X is (E)CH=CH, $R_4$ is 4-F,
$R_1$ is 1-methyl and $R_7$ is ethyl, potassium or hydrogen;
or (ii) X is $CH_2CH_2$, $R_4$ is 4-F,
$R_1$ is hydrogen and $R_7$ is methyl, sodium or hydrogen:
d) as a) except that Z is a group IIb in trans form and
(i) X is (E)CH=CH, $R_4$ is 4-F and
$R_1$ is hydrogen, 4-fluoro or 4-methyl;
or (ii) X is (E)CH=CH and $R_4$ and $R_1$ are hydrogen;
or (iii) X is $CH_2CH_2$, $R_4$ is 4-F and $R_1$ is hydrogen:
e) as b) except that Z is a group IIb in trans form and $R_4$ is 4-F, $R_1$ is 3-methyl and X is (E)CH=CH or $CH_2CH_2$:
f) as c) except that Z is a group IIb in trans form and $R_4$ is 4-F,
$R_1$ is 1-methyl and X is (E)CH=CH or $CH_2CH_2$:
g) as a) except that Z is a group IIb in cis form, X is (E)CH=CH,
$R_4$ is 4-F and $R_1$ is hydrogen:
h) as a) except that Ro + Ro = $(CH_2)_4$ and
(i) X is (E)CH=CH, $R_4$ is 4-F and
$R_1$ is hydrogen or 4-methyl and $R_7$ is ethyl, sodium or hydrogen;
or $R_1$ is 4-chloro and $R_7$ is ethyl, potassium or hydrogen;
or (ii) X is $CH_2CH_2$, $R_4$ is 4-F,

$R_1$ is hydrogen and $R_7$ is ethyl, potassium or hydrogen;
or (iii) X is (E)CH=CH, $R_4$ and $R_1$ are hydrogen and $R_7$ is ethyl, potassium or hydrogen:
i) as b) except that Ro + Ro = $(CH_2)_4$ and
(i) X is (E)CH=CH, $R_4$ is 4-F,
$R_1$ is 3-methyl and $R_7$ is ethyl, potassium or hydrogen;
or (ii) X is $CH_2CH_2$, $R_4$ is 4-F,
$R_1$ is 3-methyl and $R_7$ is ethyl, sodium or hydrogen:
j) as c) except that Ro + Ro = $(CH_2)_4$ and
(i) X is (E)CH=CH, $R_4$ is 4-F,
$R_1$ is 1-methyl and $R_7$ is ethyl, potassium or hydrogen;
or (ii) X is $CH_2CH_2$, $R_4$ is 4-F,
$R_1$ is 1-methyl and $R_7$ is ethyl, sodium or hydrogen:
k) as d) except that Ro + Ro = $(CH_2)_4$ and
(i) X is (E)CH=CH, $R_4$ is 4-F and $R_1$ is hydrogen;
4-chloro or 4-methyl;
or (ii) X is (E)CH=CH and $R_4$ and $R_1$ are hydrogen,
or (iii) X is $CH_2CH_2$, $R_4$ is 4-F and $R_1$ is hydrogen:
(l) as e) except that Ro + Ro = $(CH_2)_4$, X is (E)CH=CH or $CH_2CH_2$, $R_4$ is 4-F and $R_1$ is 3-methyl:
(m) as f) except that Ro + Ro = $(CH_2)_4$, X is (E)CH=CH or $CH_2CH_2$, $R_4$ is 4-F and $R_1$ is 1-methyl:
(n) as g) except that Ro + Ro = $(CH_2)_4$, X is (E)CH=CH, $R_4$ is 4-fluoro and $R_1$ is hydrogen:
(o) $R_1$, $R_5$ and $R_{5a}$ are hydrogen, $R_2$ is phenoxy, $R_3$ is 7-methyl, $R_4$ is 3-$CF_3$, X is $CH_2$, Ro + Ro is

$$-[CH=\underset{R_2}{C}-CH=\underset{R_3}{C}H]-,$$

X-Z is in 1-position,
the $R_4$ bearing group is in 2-position and
(i) $R_6$ is methyl, Z is a group IIa in erythro form and $R_7$ is methyl, sodium or hydrogen;
or (ii) $R_6$ is hydrogen and Z is a group IIb in trans form:
(p) $R_1$ and $R_{5a}$ are hydrogen, $R_4$ is 3-methyl, $R_5$ is 4-methoxy, Ro + Ro = $(CH_2)_4$, $R_6$ is methyl, X is $CH_2$, X-Z is in 1-position, the $R_4$ bearing group is in 2-position and
(i) Z is a group IIa in erythro form and $R_7$ is methyl, sodium or hydrogen;
or (ii) Z is a group IIb in trans form.

6. A process according to Claim 1 wherein
a) Z is a group IIa in erythro form, Ro + Ro =

$$-[CH=\underset{R_2}{C}-CH=\underset{R_3}{C}H]-,$$

$R_2$, $R_3$, $R_4$, $R_5$, $R_{5a}$ and $R_6$ are hydrogen, $R_4$ is 4-F, X-Z is in 2-position, the $R_4$ bearing group is in 1-position and
(i) X is (E)CH=CH, $R_1$ is 3-ethyl or 3-i-propyl and $R_7$ is ethyl, sodium or hydrogen;
or (ii) X is (E)CH=CH or $CH_2CH_2$, $R_1$ is hydrogen and $R_7$ is ethyl or sodium;
or (iii) X is (E)CH=CH, $R_1$ is 4-chloro or 3-methyl and $R_7$ is sodium:
b) as a) except that X-Z is in 2-position, the $R_4$ bearing group is in 3-position, $R_1$ is hydrogen, X is (E)CH=CH and $R_7$ is ethyl sodium or hydrogen:
c) Z is a group IIb in trans form, Ro + Ro =

$$-[CH=\underset{R_2}{C}-CH=\underset{R_3}{C}H]-,$$

$R_2$, $R_3$, $R_5$, $R_{5a}$ and $R_6$ are hydrogen, $R_4$ is 4-F and
(i) X-Z is in 2-position, the $R_4$ bearing group is in 1-position, X is (E)CH=CH and $R_1$ is 3-ethyl; 3-i-propyl,
or (ii) X-Z is in 2-position, the $R_4$ bearing group is in 3-position, X=(E)CH=CH and $R_1$ is hydrogen;
or (iii) X-Z is in 2-position, the $R_4$ bearing group is in 1-position, X is $CH_2CH_2$ and $R_1$ is hydrogen:
d) Z is a group IIa in erythro form, Ro + Ro = $(CH_2)_4$, $R_1$, $R_5$,$R_{5a}$ and $R_6$ are hydrogen, $R_4$ is 4-F, X is (E)CH=CH, X-Z is in 2-position, the $R_4$ bearing group is in 3-position and $R_7$ is ethyl, sodium or hydrogen:
e) Z is a group IIb in trans form Ro + Ro = $(CH_2)_4$, $R_1$, $R_5$, $R_{5a}$ and $R_6$ are hydrogen, $R_4$ is 4-F, X is (E)CH=CH and X;Z is in 1- or 2-position when the $R_4$ bearing group is in 2- or 3-position respectively.

7. A process according to Claim 1 wherein the compound prepared is selected from
Erythro-(E)-3R,5S,dihydroxy-7-(2'-[4"-fluorophenyl]naphth-1'-yl)hept-6-enoate or a salt thereof;
Erythro-(E)-3,5-dihydroxy-7-(1'-[4"-fluorophenyl]-3'-[1'-methyl-ethyl]naphth-2'-yl)hept-6-enoate or a salt thereof;
(E)-Trans-6-(2'-[3"-ethyl-1"-(4'''-fluorophenyl)naphth-2"-yl]-ethenyl)-4-hydroxy-3,4,5,6-tetrahydropyran-2-one.

8. A pharmaceutical composition comprising a compound prepared according to any one of Claims 1 to 7 as appropriate in free acid form or in the form of a physiologically-hydrolysable and -acceptable ester or a lactone thereof or in pharmaceutically acceptable salt form, together with a pharmaceutically acceptable diluent or carrier.

9. A compound prepared according to any one of Claims 1 to 7 as appropriate in free acid form or in the form of a physiologically-hydrolysable and -acceptable ester or a lactone thereof or in pharmaceutcally salt form for use in inhibiting cholesterol biosynthesis or treating atherosclerosis.

10. A process for the preparation of a pharmaceutical composition comprising admixing a compound of formula I

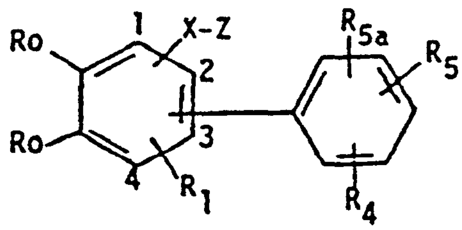

I

wherein the substituents are as defined in any one of Claims 1 to 6 as appropriate in free acid form or in the form of a physiologicallyhydrolysable and -acceptable ester or a lactone thereof or in pharmaceutically acceptable salt form, together with a pharmaceutically acceptable diluent or carrier.

**Patentansprüche**

für die Vertragsstaaten BE CH DE FR GB IT LI LU NL SE.

1. Eine Verbindung der Formel I,

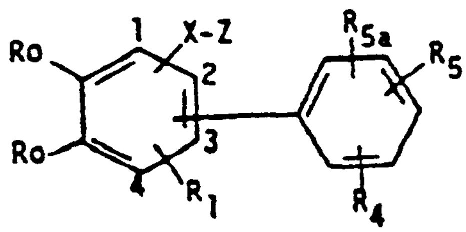

I

worin die zwei Ro-Gruppen zusammen ein Radikal der Formel

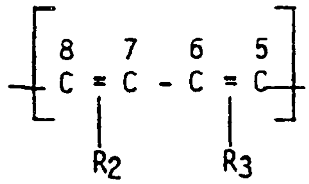 oder $-(CH_2)_4-$

bilden, worin $R_2$ Wasserstoff, $C_{1-4}$Alkyl, $C_{1-3}$Alkoxy, n-Butoxy, i-Butoxy, s-Butoxy, Trifluormethyl, Fluor, Chlor, Phenoxy oder Benzyloxy bedeutet,

$R_3$ Wasserstoff, $C_{1-3}$Alkyl, $C_{1-3}$Alkoxy, Trifluormethyl, Fluor, Chlor, Phenoxy oder Benzyloxy bedeutet, mit der Massgabe, dass nicht mehr als einer von $R_2$ und $R_3$ Trifluormethyl bedeutet, dass nicht mehr als einer von $R_2$ und $R_3$ Phenoxy bedeutet und nicht mehr als einer von $R_2$ und $R_3$ Benzyloxy bedeutet,

$R_1$ Wasserstoff, $C_{1-6}$Alkyl, Fluor, Chlor oder Benzyloxy bedeutet,

$R_4$ Wasserstoff, $C_{1-4}$Alkyl, $C_{1-3}$Alkoxy, n-Butoxy, i-Butoxy, s-Butoxy, Trifluormethyl, Fluor, Chlor, Phenoxy oder Benzyloxy bedeutet,

$R_5$ Wasserstoff, $C_{1-3}$Alkyl, $C_{1-3}$Alkoxy, Trifluormethyl, Fluor, Chlor, Phenoxy oder Benzyloxy bedeutet,

$R_{5a}$ Wasserstoff, $C_{1-2}$Alkyl, $C_{1-2}$Alkoxy, Fluor oder Chlor bedeutet,

mit der Massgabe, dass nicht mehr als einer von $R_4$ und $R_5$ Trifluormethyl bedeutet, dass nicht mehr als einer von $R_4$ und $R_5$ Phenoxy bedeutet und nicht mehr als einer von $R_4$ und $R_5$ Benzyloxy bedeutet,

X $-(CH_2)n-$ oder

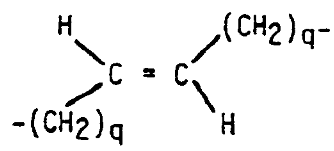

bedeutet, worin n 0, 1, 2 oder 3 bedeutet und beide q's 0 oder eines 0 und das andere 1 bedeuten, und

Z

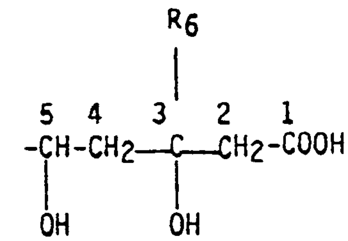

II

bedeutet, worin $R_6$ Wasserstoff oder $C_{1-3}$Alkyl bedeutet, mit der allgemeinen Massgabe, dass -X-Z und die $R_4$-tragende Phenylgruppe in ortho-Stellung zueinander stehen;

in Form der freien Säure oder in Form eines physiologischhydrolysierbaren und akzeptablen Esters oder ein δ-Lacton davon oder in Salzform.

2. Eine Verbindung nach Anspruch 1, worin die zwei Ro-Gruppen zusammen ein Radikal der Formel

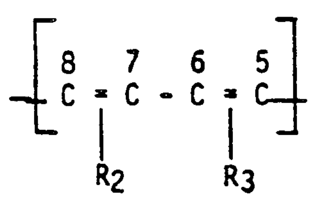 oder $-CH_2CH_2CH_2CH_2-$

bilden, worin $R_2$ Wasserstoff, $C_{1-3}$Alkyl, n-Butyl, i-Butyl, $C_{1-3}$Alkoxy, n-Butoxy, i-Butoxy, Trifluormethyl, Fluor, Chlor, Phenoxy oder Benzyloxy bedeutet,

$R_3$ Wasserstoff, $C_{1-3}$Alkyl, $C_{1-3}$Alkoxy, Trifluormethyl, Fluor, Chlor, Phenoxy oder Benzyloxy bedeutet,

mit der Massgabe, dass nicht mehr als einer von $R_2$ und $R_3$ Trifluormethyl bedeutet, dass nicht mehr als einer von $R_2$ und $R_3$ Phenoxy bedeutet und nicht mehr als einer von $R_2$ und $R_3$ Benzyloxy bedeutet,

$R_1$ Wasserstoff, $C_{1-3}$Alkyl, Fluor, Chlor oder Benzyloxy bedeutet,

$R_4$ Wasserstoff, $C_{1-3}$Alkyl, n-Butyl, i-Butyl, $C_{1-3}$Alkoxy, n-Butoxy, i-Butoxy, Trifluormethyl, Fluor, Chlor,

Phenoxy oder Benzyloxy bedeutet,

$R_5$ Wasserstoff, $C_{1-3}$Alkyl, $C_{1-3}$Alkoxy, Trifluormethyl, Fluor, Chlor, Phenoxy oder Benzyloxy bedeutet,

$R_{5a}$ Wasserstoff bedeutet,

mit der Massgabe, dass nicht mehr als einer von $R_4$ und $R_5$ Trifluormethyl bedeutet und nicht mehr als einer von $R_4$ und $R_5$ Benzyloxy und nicht mehr als einer von $R_4$ und $R_5$ Phenoxy bedeutet,

X

$-(CH_2)_n-$, 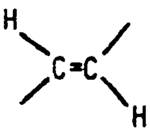 , 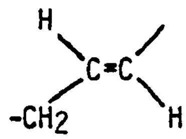 oder 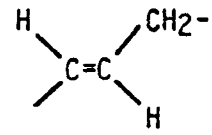

bedeutet, worin n 0, 1, 2 oder 3 bedeutet, und

Z

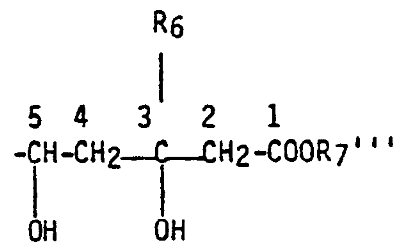

oder

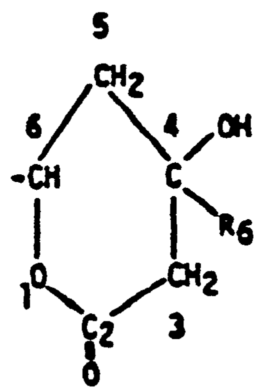

IIb

bedeutet, worin $R_6$ Wasserstoff oder $C_{1-3}$Alkyl bedeutet, und

$R_7'''$ Wasserstoff, $C_{1-3}$Alkyl, n-Butyl, i-Butyl, t-Butyl, Benzyl oder M bedeutet, worin M ein pharmazeutisch akzeptables Kation bedeutet,

mit der Massgabe, dass die -X-Z Gruppe und die R4-tragende Phenylgruppe in ortho-Stellung zueinander stehen.

3. Eine Verbindung nach Anspruch 1, worin Ro + Ro wie im Anspruch 1 definiert ist,

$R_1$ Wasserstoff oder $C_{1-3}$Alkyl bedeutet,

$R_2$, $R_3$, $R_4$ und $R_5$ unabhängig voneinander Wasserstoff, Methyl, Methoxy, Fluor oder Chlor bedeuten,

$R_{5a}$ Wasserstoff bedeutet,

X-Z sich in 1-Stellung befindet und stellt

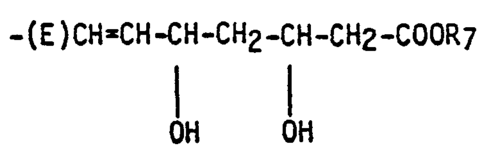

dar, worin $R_7$ Wasserstoff, $C_{1-2}$Alkyl oder ein pharmazeutisch akzeptables Kation bedeutet und die $R_4$-tragende Gruppe sich in 2-Stellung befindet.

4. Eine Verbindung nach Anspruch 3, worin X-Z sich in Stellung 2 und die $R_4$-tragende Gruppe sich in Stellung 1 befindet.

5. Eine Verbindung nach Anspruch 1, worin die Substituenten die folgende Bedeutung haben:

a) Z eine Gruppe IIa in Erythro-Form bedeutet, Ro + Ro =

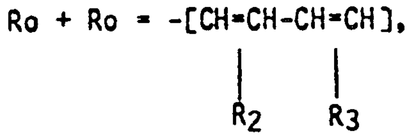

$R_2$, $R_3$, $R_5$, $R_{5a}$ und $R_6$ Wasserstoff bedeuten,
X-Z sich in 1-Stellung, die $R_4$-tragende Gruppe sich in 2-Stellung befindet und
(i) X (E)CH=CH bedeutet, $R_4$ 4-F bedeutet und
$R_1$ Wasserstoff oder 4-Methyl bedeutet und
$R_7$ Aethyl, Natrium oder Wasserstoff bedeutet;
oder
$R_1$ 4-Chlor bedeutet und $R_7$ Aethyl, Kalium oder Wasserstoff bedeutet;
oder (ii) X (E)CH=CH, $R_4$ und $R_1$ Wasserstoff und $R_7$ Aethyl, Kalium oder Wasserstoff bedeuten;
oder (iii) X $CH_2CH_2$, $R_4$ 4-F bedeutet,
$R_1$ Wasserstoff und $R_7$ Aethyl, Natrium oder Wasserstoff bedeutet:

b) wie a) ausgenommen X-Z befindet sich in 2-Stellung, so befindet sich die $R_4$-tragende Gruppe in 1-Stellung und
(i) X (E)CH=CH bedeutet, $R_4$ 4-F bedeutet,
$R_1$ 3-Methyl bedeutet und $R_7$ Aethyl, Kalium oder Wasserstoff bedeutet,
oder (ii) X $CH_2CH_2$ bedeutet, $R_4$ 4-F bedeutet,
$R_1$ 3-Methyl und $R_7$ Aethyl, Natrium oder Wasserstoff bedeutet;

c) wie a) ausgenommen X-Z befindet sich in 2-Stellung, so befindet sich die $R_4$ tragende Gruppe in 3-Stellung und
(i) X (E)CH=CH bedeutet, $R_4$ 4-F bedeutet,
$R_1$ 1-Methyl und $R_7$ Aethyl, Kalium oder Wasserstoff bedeutet;
oder (ii) X $CH_2$-$CH_2$, $R_4$ 4-F bedeutet;

d) wie a) ausgenommen Z bedeutet eine Gruppe IIb in trans-Form und
(i) X (E)CH=CH bedeutet, $R_4$ 4-F bedeutet und
$R_1$ Wasserstoff, 4-Fluor oder 4-Methyl bedeutet;
oder (ii) X (E)CH=CH bedeutet und $R_4$ und $R_1$ Wasserstoff bedeuten;
oder (iii) X $CH_2CH_2$, $R_4$ 4-F und $R_1$ Wasserstoff bedeuten;

e) wie b) ausgenommen, dass Z eine Gruppe IIb in trans-Form bedeutet und $R_4$ 4-F, $R_1$ 3-Methyl und X (E)CH=CH oder $CH_2CH_2$ bedeuten;

f) wie c) ausgenommen, dass Z eine Gruppe IIb in trans-Form bedeutet und $R_4$ 4-F bedeutet, $R_1$ 1-Methyl und X (E)CH=CH oder $CH_2CH_2$ bedeuten;

g) wie a) ausgenommen, dass Z eine Gruppe IIb in cis-Form bedeutet, X (E)CH=CH bedeutet, $R_4$ 4-F und $R_1$ Wasserstoff bedeuten;

h) wie a) ausgenommen, dass Ro + Ro = $(CH_2)_4$ und
(i) X (E)CH=CH, $R_4$ 4-F bedeuten und
$R_1$ Wasserstoff oder 4-Methyl bedeutet und $R_7$ Aethyl, Natrium oder Wasserstoff bedeutet;
oder
$R_1$ 4-Chlor und $R_7$ Aethyl, Kalium oder Wasserstoff bedeuten;
oder (ii) X $CH_2CH_2$, $R_4$ 4-F bedeutet,
$R_1$ Wasserstoff bedeutet und $R_7$ Aethyl, Kalium oder Wasserstoff bedeutet;
oder (iii) X (E)CH=CH bedeutet, $R_4$ und $R_1$ Wasserstoff bedeuten und $R_7$ Aethyl, Kalium oder Wasserstoff bedeutet;

i) wie b) ausgenommen, dass Ro + Ro = $(CH_2)_4$ und
(i) X (E)CH=CH, $R_4$ 4-F bedeuten,
$R_1$ 3-Methyl bedeutet und $R_7$ Aethyl, Kalium oder Wasserstoff bedeutet;
oder (ii) X $CH_2CH_2$, $R_4$ 4-F bedeutet,
$R_1$ 3-Methyl und $R_7$ Aethyl, Natrium oder Wasserstoff bedeutet;

j) wie c) ausgenommen, dass Ro + Ro = $(CH_2)_4$ und
(i) X (E)CH=CH, $R_4$ 4-F bedeutet,
$R_1$ 1-Methyl bedeutet und $R_7$ Aethyl, Kalium oder Wasserstoff bedeutet;
oder (ii) X $CH_2CH_2$, $R_4$ 4-F bedeutet, $R_1$ 1-Methyl und $R_7$ Aethyl, Natrium oder Wasserstoff bedeutet;

k) wie d) ausgenommen, dass Ro + Ro = $(CH_2)_4$ und
(i) X (E)CH=CH, $R_4$ 4-F und $R_1$ Wasserstoff, 4-Chlor oder 4-Methyl bedeuten;
oder (ii) X (E)CH=CH bedeutet und $R_4$ und $R_1$ Wasserstoff bedeuten;

oder (iii) X $CH_2CH_2$, $R_4$ 4-F und $R_1$ Wasserstoff bedeuten;

l) wie e) ausgenommen, dass Ro + Ro = $(CH_2)_4$, X (E)CH=CH oder $CH_2CH_2$ bedeutet, $R_4$ 4-F und $R_1$ 3-Methyl bedeuten;

m) wie f) ausgenommen, dass Ro + Ro = $(CH_2)_4$, X (E)CH=CH oder $CH_2CH_2$ bedeutet, $R_4$ 4-F und $R_1$ 1-Methyl bedeuten;

n) wie g) ausgenommen, dass Ro + Ro = $(CH_2)_4$, X (E)CH=CH bedeutet, $R_4$ 4-F und $R_1$ Wasserstoff bedeuten;

o) $R_1$, $R_5$ und $R_{5a}$ Wasserstoff bedeuten, $R_2$ Phenoxy bedeutet, $R_3$ 7-Methyl bedeutet, $R_4$ 3-$CF_3$ bedeutet, X $CH_2$ bedeutet,

Ro + Ro =

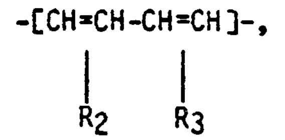

X-Z sich in 1-Stellung befindet, die $R_4$-tragende Gruppe sich in 2-Stellung befindet und

(i) $R_6$ Methyl bedeutet, Z eine Gruppe IIa in erythro-Form bedeutet und $R_7$ Methyl, Natrium oder Wasserstoff bedeutet;

oder (ii) $R_6$ Wasserstoff bedeutet und Z eine Gruppe IIb in trans-Form bedeutet:

p) $R_1$ und $R_{5a}$ Wasserstoff bedeuten, $R_4$ 3-Methyl bedeutet, $R_5$ 4-Methoxy bedeutet, Ro + Ro = $(CH_2)_4$, $R_6$ Methyl bedeutet, X $CH_2$ bedeutet, X-Z sich in 1-Stellung befindet, die $R_4$-tragende Gruppe sich in 2-Stellung befindet und

(i) Z eine Gruppe IIa in erythro-Form bedeutet und $R_7$ Methyl, Natrium oder Wasserstoff bedeutet;

oder (ii) Z eine Gruppe IIb in trans-Form bedeutet.

6. Eine Verbindung nach Anspruch 1, worin

a) Z eine Gruppe IIa in erythro-Form bedeutet, Ro + Ro =

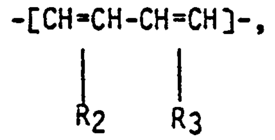

$R_2$, $R_3$, $R_4$, $R_5$, $R_{5a}$ und $R_6$ Wasserstoff bedeuten, $R_4$ 4-F bedeutet, X-Z sich in 2-Stellung befindet, die $R_4$-tragende Gruppe sich in 1-Stellung befindet und

(i) X (E)CH=CH bedeutet, $R_1$ 3-Aethyl oder 3-i-Propyl bedeutet und $R_7$ Aethyl, Natrium oder Wasserstoff bedeutet;

oder (ii) X (E)CH=CH oder $CH_2CH_2$ bedeutet, $R_1$ Wasserstoff bedeutet und $R_7$ Aethyl oder Natrium bedeutet;

oder (iii) X (E)CH=CH bedeutet, $R_1$ 4-Chlor oder 3-Methyl und $R_7$ Natrium bedeutet:

b) wie a) ausgenommen, dass X-Z sich in 2-Stellung befindet, die $R_4$-tragende Gruppe sich in 3-Stellung befindet, $R_1$ Wasserstoff bedeutet, X (E)CH=CH bedeutet und $R_7$ Aethyl, Natrium oder Wasserstoff bedeutet;

c) Z eine Gruppe IIb in trans-Form bedeutet, Ro + Ro =

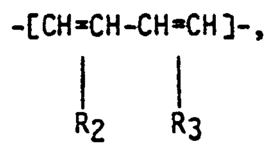

$R_2$, $R_3$, $R_5$, $R_{5a}$ und $R_6$ Wasserstoff bedeuten, $R_4$ 4-F bedeutet und

(i) X-Z sich in 2-Stellung befindet, die $R_4$-tragende Gruppe sich in 1-Stellung befindet, X (E)CH=CH bedeutet und $R_1$ 3-Aethyl oder 3-i-Propyl bedeutet;

oder (ii) X-Z sich in 2-Stellung befindet, die $R_4$-tragende Gruppe sich in Stellung 3 befindet, X (E)CH=CH und $R_1$ Wasserstoff bedeuten;

oder (iii) X-Z sich in 2-Stellung befindet, die $R_4$-tragende Gruppe sich in Stellung 1 befindet, X $CH_2CH_2$ und $R_1$ Wasserstoff bedeuten:

d) Z eine Gruppe IIa in erythro-Form bedeutet, Ro + Ro = $(CH_2)_4$, $R_1$, $R_5$, $R_{5a}$ und $R_6$ Wasserstoff bedeuten, $R_4$ 4-F bedeutet, X (E)CH=CH bedeutet, X-Z sich in 2-Stellung befindet, die $R_4$-tragende Gruppe sich in 3-Stellung befindet und $R_7$ Aethyl, Natrium oder Wasserstoff bedeutet;

e) Z eine Gruppe IIb in trans-Form bedeutet, Ro + Ro = $(CH_2)_4$, $R_1$, $R_5$, $R_{5a}$ und $R_6$ Wasserstoff bedeuten, $R_4$

4-F bedeutet, X (E)CH=CH bedeutet, X-Z sich in 1- oder 2-Stellung befindet, wenn die $R_4$-tragende Gruppe sich in 2- bzw. 3-Stellung befindet.

7. Erythro-(E)-3R,5S,dihydroxy-7-(2'-[4''-fluorphenyl]naphth-1'-yl)hept-6-enoat oder ein Salz davon.

8. Erythro-(E)-3,5-dihydroxy-7-(1'-[4''-fluorphenyl]-3'-[1',-methyl-aethyl]naphth-2'-yl)hept-6-enoat oder ein Salz davon.

9. (E)-Trans-6-(2'-[3''-aethyl-1''-(4'''-fluorphenyl)naphth-2''-yl]-aethenyl)-4-hydroxy-3,4,5,6-tetrahydropyran-2-on.

10. Eine Verbindung in Uebereinstimmung mit Anspruch 7 oder 8 in Form seines Natriumsalzes.

11. Eine pharmazeutische Zusammensetzung, umfassend eine Verbindung entsprechend einem der Ansprüche 1 bis 10, geeignet in Form der freien Säure oder in Form eines physiologisch hydrolysierbaren und akzeptablen Esters oder eines Laktones davon oder in Form eines pharmazeutisch akzeptablen Salzes, zusammen mit einem pharmazeutisch akzeptablen Verdünner oder Träger.

12. Eine Verbindung entsprechend einem der Ansprüche 1 bis 10, geeignet in Form der freien Säure oder in Form eines physiologisch-hydrolysierbaren und akzeptablen Esters oder eines Laktones davon oder in Form eines pharmazeutisch akzeptablen Salzes zur Anwendung bei der Hemmung der Cholesterin-Biosynthese oder zur Behandlung der Atherosklerose.

13. Ein Verfahren zur Herstellung einer Verbindung nach Anspruch 1, umfassend

a) Wenn $R_6$ Wasserstoff bedeutet, die Reduktion einer Verbindung der Formel VI

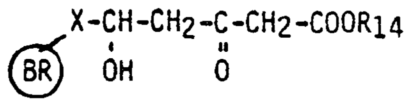

VI

worin $R_{14}$ ein Radikal bedeutet, das einen physiologisch-hydrolysierbaren und akzeptablen Ester bildet und X obige Bedeutung hat,

b) wenn $R_6$ $C_{1-3}$Alkyl bedeutet, eine Verbindung der Formel XVII hydrolysiert,

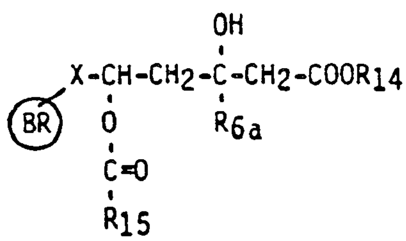

XVII

worin $R_{6a}$ $C_{1-3}$Alkyl bedeutet, $R_{15}$ $C_{1-2}$Alkyl bedeutet und X und $R_{14}$ obige Bedeutung haben,

c) wenn X die Bedeutung

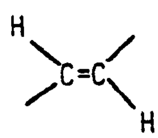

hat, durch Abspalten einer Schutzgruppe bei einer Verbindung der Formel LVIII

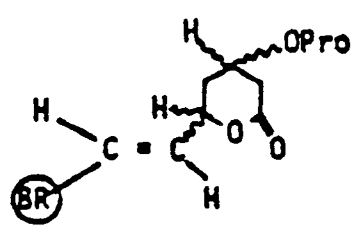

LVIII

worin Pro eine Schutzgruppe bedeutet,

d) durch Hydrolyse einer Verbindung der Formel I in Form eines physiologisch hydrolisierbaren Esters oder eines Lactones oder

e) durch Veresterung oder Lactonisierung einer Verbindung der Formel I in Form der freien Säure und falls eine freie Carboxylgruppe anwesend ist, die so in Form der freien Säure oder in Form eines Salzes wiedergewinnt, wobei

(BR) die Grundringstruktur

I

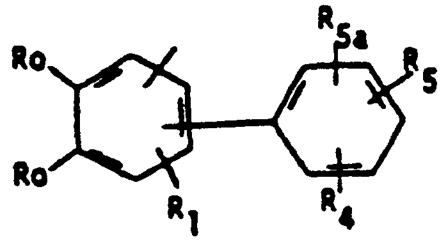

aufweist und Ro, $R_1$, $R_4$, $R_{5a}$, $R_5$, X und Z die im Anspruch 1 angegebene Bedeutung haben.

**Patentansprüche**

für den Vertragsstaat AT.

1. Verfahren zur Herstellung einer Verbindung der Formel I,

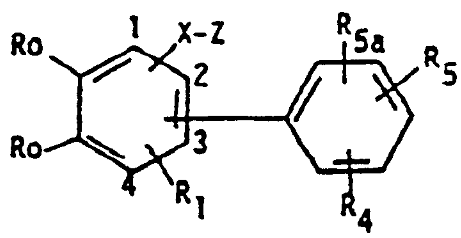

I

worin die zwei Ro-Gruppen zusammen ein Radikal der Formel

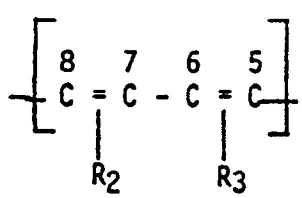

oder $-(CH_2)_4-$

bilden, worin $R_2$ Wasserstoff, $C_{1-4}$Alkyl, $C_{1-3}$Alkoxy, n-Butoxy, i-Butoxy, s-Butoxy, Trifluormethyl, Fluor, Chlor, Phenoxy oder Benzyloxy bedeutet,

$R_3$ Wasserstoff, $C_{1-3}$Alkyl, $C_{1-3}$Alkoxy, Trifluormethyl, Fluor, Chlor, Phenoxy oder Benzyloxy bedeutet, mit der Massgabe, dass nicht mehr als einer von $R_2$ und $R_3$ Trifluormethyl bedeutet, dass nicht mehr als einer von $R_2$ und $R_3$ Phenoxy bedeutet und nicht mehr als einer von $R_2$ und $R_3$ Benzyloxy bedeutet,

$R_1$ Wasserstoff, $C_{1-6}$Alkyl, Fluor, Chlor oder Benzyloxy bedeutet,

$R_4$ Wasserstoff, $C_{1-4}$Alkyl, $C_{1-3}$Alkoxy, n-Butoxy, i-Butoxy, s-Butoxy, Trifluormethyl, Fluor, Chlor, Phenoxy oder Benzyloxy bedeutet,

$R_5$ Wasserstoff, $C_{1-3}$ Alkyl, $C_{1-3}$Alkoxy, Trifluormethyl, Fluor, Chlor, Phenoxy oder Benzyloxy bedeutet,

$R_{5a}$ Wasserstoff, $C_{1-2}$Alkyl, $C_{1-2}$Alkoxy, Fluor oder Chlor bedeutet,

mit der Massgabe, dass nicht mehr als einer von $R_4$ und $R_5$ Trifluormethyl bedeutet, dass nicht mehr als einer von $R_4$ und $R_5$ Phenoxy bedeutet und nicht mehr als einer von $R_4$ und $R_5$ Benzyloxy bedeutet,

X $-(CH2)_n-$ oder

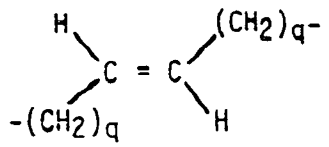

bedeutet, worin n 0, 1, 2 oder 3 bedeutet und beide q's 0 oder eines 0 und das andere 1 bedeuten, und

Z

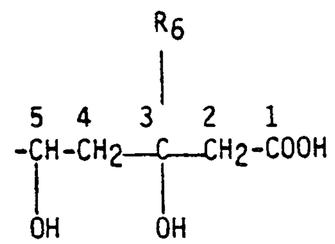

II

bedeutet, worin $R_6$ Wasserstoff oder $C_{1-3}$Alkyl bedeutet, mit der allgemeinen Massgabe, dass -X-Z und die $R_4$-tragende Phenylgruppe in ortho-Stellung zueinander stehen;

in Form der freien Säure oder in Form eines physiologischhydrolysierbaren und akzeptablen Esters oder ein δ-Lacton davon oder in Salzform, dadurch gekennzeichnet, dass

a) Wenn $R_6$ Wasserstoff bedeutet, die Reduktion einer Verbindung der Formel VI

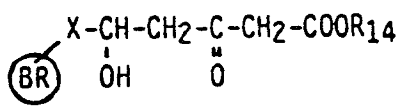

VI

worin $R_{14}$ ein Radikal bedeutet, das einen physiologisch-hydrolysierbaren und akzeptablen Ester bildet und X obige Bedeutung hat,

b) wenn $R_6$ $C_{1-3}$Alkyl bedeutet, eine Verbindung der Formel XVII hydrolysiert,

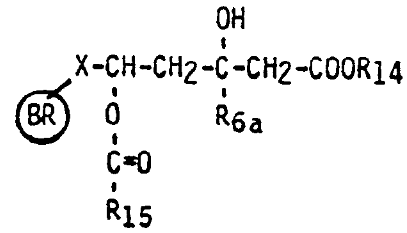

XVII

worin $R_{6a}$ $C_{1\text{-}3}$Alkyl bedeutet, $R_{15}$ $C_{1\text{-}2}$Alkyl bedeutet und X und $R_{14}$ obige Bedeutung haben,
c) wenn X die Bedeutung

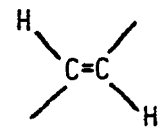

hat, durch Abspalten einer Schutzgruppe bei einer Verbindung der Formel LVIII

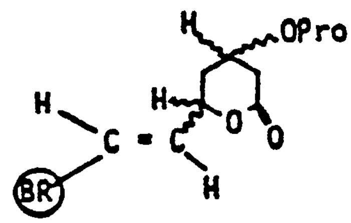

LVIII

worin Pro eine Schutzgruppe bedeutet,
d) durch Hydrolyse einer Verbindung der Formel I in Form eines physiologisch hydrolisierbaren Esters oder eines Lactones oder
e) durch Veresterung oder Lactonisierung einer Verbindung der Formel I in Form der freien Säure und falls eine freie Carboxylgruppe anwesend ist, die so in Form der freien Säure oder in Form eines Salzes wiedergewinnt, wobei

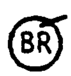 die Grundringstruktur

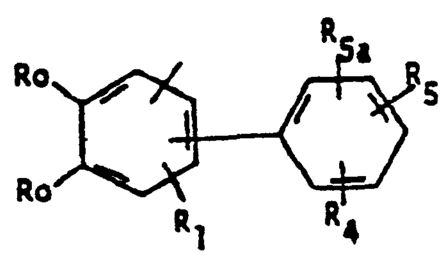

aufweist und Ro, $R_1$, $R_4$, $R_{5a}$, $R_5$, X und Z die im Anspruch 1 angegebene Bedeutung haben.
2. Ein Verfahren nach Anspruch 1, worin die zwei Ro-Gruppen zusammen ein Radikal der Formel

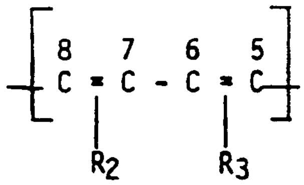 oder $-CH_2CH_2CH_2CH_2-$

bilden, worin $R_2$ Wasserstoff, $C_{1-3}$Alkyl, n-Butyl, i-Butyl, $C_{1-3}$Alkoxy, n-Butoxy, i-Butoxy, Trifluormethyl, Fluor, Chlor, Phenoxy oder Benzyloxy bedeutet,

$R_3$ Wasserstoff, $C_{1-3}$Alkyl, $C_{1-3}$Alkoxy, Trifluormethyl, Fluor, Chlor, Phenoxy oder Benzyloxy bedeutet,

mit der Massgabe, dass nicht mehr als einer von $R_2$ und $R_3$ Trifluormethyl bedeutet, dass nicht mehr als einer von $R_2$ und $R_3$ Phenoxy bedeutet und nicht mehr als einer von $R_2$ und $R_3$ Benzyloxy bedeutet,

$R_1$ Wasserstoff, $C_{1-3}$Alkyl, Fluor, Chlor oder Benzyloxy bedeutet,

$R_4$ Wasserstoff, $C_{1-3}$Alkyl, n-Butyl, i-Butyl, $C_{1-3}$Alkoxy, n-Butoxy, i-Butoxy, Trifluormethyl, Fluor, Chlor, Phenoxy oder Benzyloxy bedeutet,

$R_5$ Wasserstoff, $C_{1-3}$Alkyl, $C_{1-3}$Alkoxy, Trifluormethyl, Fluor, Chlor, Phenoxy oder Benzyloxy bedeutet,

$R_{5a}$ Wasserstoff bedeutet,

mit der Massgabe, dass nicht mehr als einer von $R_4$ und $R_5$ Trifluormethyl bedeutet und nicht mehr als einer von $R_4$ und $R_5$ Benzyloxy und nicht mehr als einer von $R_4$ und $R_5$ Phenoxy bedeutet, X $-(CH_2)n-$,

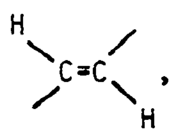 , 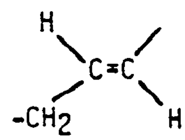 oder 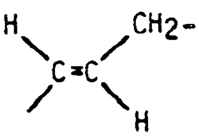

bedeutet, worin n 0, 1, 2 oder 3 bedeutet, und

Z

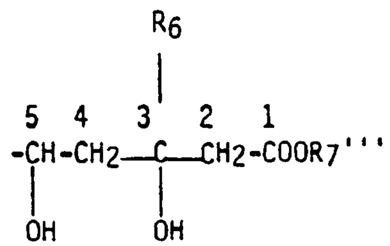 oder

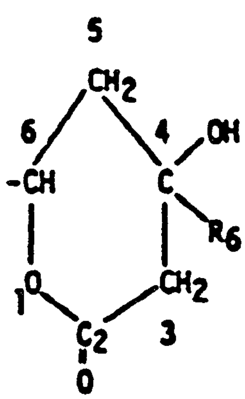

IIb

bedeutet, worin $R_6$ Wasserstoff oder $C_{1-3}$Alkyl bedeutet, und $R_7'''$ Wasserstoff, $C_{1-3}$Alkyl, n-Butyl, i-Butyl, t-Butyl, Benzyl oder M bedeutet, worin M ein pharmazeutisch akzeptables Kation bedeutet, mit der Massgabe, dass die -X-Z Gruppe und die $R_4$-tragende Phenylgruppe in ortho-Stellung zueinander stehen.

3. Ein Verfahren nach Anspruch 1, worin Ro + Ro wie im Anspruch 1 definiert ist, $R_1$ Wasserstoff oder $C_{1-3}$Alkyl bedeutet, $R_2$, $R_3$, $R_4$ und $R_5$ unabhängig voneinander Wasserstoff, Methyl, Methoxy, Fluor oder Chlor bedeuten, $R_{5a}$ Wasserstoff bedeutet, X-Z sich in 1-Stellung befindet und stellt

$$-(E)CH{=}CH-\underset{OH}{CH}-CH_2-\underset{OH}{CH}-CH_2-COOR_7$$

dar, worin $R_7$ Wasserstoff, $C_{1-2}$Alkyl oder ein pharmazeutisch akzeptables Kation bedeutet und die $R_4$-tragende Gruppe sich in 2-Stellung befindet.

4. Ein Verfahren nach Anspruch 3, worin X-Z sich in Stellung 2 und die $R_4$-tragende Gruppe sich in Stellung 1 befindet.

5. Ein Verfahren nach Anspruch 1, worin die Substituenten die folgende Bedeutung haben:

a) Z eine Gruppe IIa in Erythro-Form bedeutet, Ro + Ro =

$$-[\underset{R_2}{CH}{=}\underset{R_3}{CH}-CH{=}CH],$$

$R_2$, $R_3$, $R_5$, $R_{5a}$ und $R_6$ Wasserstoff bedeuten, X-Z sich in 1-Stellung, die $R_4$-tragende Gruppe sich in 2-Stellung befindet und

(i) X (E)CH=CH bedeutet, $R_4$ 4-F bedeutet und $R_1$ Wasserstoff oder 4-Methyl bedeutet und $R_7$ Aethyl, Natrium oder Wasserstoff bedeutet;
oder
$R_1$ 4-Chlor bedeutet und $R_7$ Aethyl, Kalium oder Wasserstoff bedeutet;
oder (ii) X (E)CH=CH, $R_4$ und $R_1$ Wasserstoff und $R_7$ Aethyl, Kalium oder Wasserstoff bedeuten;
oder (iii) X $CH_2CH_2$, $R_4$ 4-F bedeutet, $R_1$ Wasserstoff und $R_7$ Aethyl, Natrium oder Wasserstoff bedeutet:

b) wie a) ausgenommen X-Z befindet sich in 2-Stellung, so befindet sich die $R_4$-tragende Gruppe in 1-Stellung und
(i) X (E)CH=CH bedeutet, $R_4$ 4-F bedeutet, $R_1$ 3-Methyl bedeutet und $R_7$ Aethyl, Kalium oder Wasserstoff bedeutet,
oder (ii) X $CH_2CH_2$ bedeutet, $R_4$ 4-F bedeutet, $R_1$ 3-Methyl und $R_7$ Aethyl, Natrium oder Wasserstoff bedeutet;

c) wie a) ausgenommen X-Z befindet sich in 2-Stellung, so befindet sich die $R_4$ tragende Gruppe in 3-Stellung und
(i) X (E)CH=CH bedeutet, $R_4$ 4-F bedeutet, $R_1$ 1-Methyl und $R_7$ Aethyl, Kalium oder Wasserstoff bedeutet;
oder (ii) X $CH_2$-$CH_2$, $R_4$ 4-F bedeutet;

d) wie a) ausgenommen Z bedeutet eine Gruppe IIb in trans-Form und
(i) X (E)CH=CH bedeutet, $R_4$ 4-F bedeutet und $R_1$ Wasserstoff, 4-Fluor oder 4-Methyl bedeutet;
oder (ii) X (E)CH=CH bedeutet und $R_4$ und $R_1$ Wasserstoff bedeuten;
oder (iii) X $CH_2CH_2$, $R_4$ 4-F und $R_1$ Wasserstoff bedeuten;

e) wie b) ausgenommen, dass Z eine Gruppe IIb in trans-Form bedeutet und $R_4$ 4-F, $R_1$ 3-Methyl und X (E)CH=CH oder $CH_2CH_2$ bedeuten;

f) wie c) ausgenommen, dass Z eine Gruppe IIb in trans-Form bedeutet und $R_4$ 4-F bedeutet, $R_1$ 1-Methyl und X (E)CH=CH oder $CH_2CH_2$ bedeuten;

g) wie a) ausgenommen, dass Z eine Gruppe IIb in cis-Form bedeutet, X (E)CH=CH bedeutet, $R_4$ 4-F und $R_1$

Wasserstoff bedeuten;

h) wie a) ausgenommen, dass Ro + Ro = $(CH_2)_4$ und

(i) X (E)CH=CH, $R_4$ 4-F bedeuten und

$R_1$ Wasserstoff oder 4-Methyl bedeutet und $R_7$ Aethyl, Natrium oder Wasserstoff bedeutet;

oder

$R_1$ 4-Chlor und $R_7$ Aethyl, Kalium oder Wasserstoff bedeuten;

oder (ii) X $CH_2CH_2$, $R_4$ 4-F bedeutet,

$R_1$ Wasserstoff bedeutet und $R_7$ Aethyl, Kalium oder Wasserstoff bedeutet;

oder (iii) X (E)CH=CH bedeutet, $R_4$ und $R_1$ Wasserstoff bedeuten und $R_7$ Aethyl, Kalium oder Wasserstoff bedeutet;

i) wie b) ausgenommen, dass Ro + Ro = $(CH_2)_4$ und

(i) X (E)CH=CH, $R_4$ 4-F bedeuten,

$R_1$ 3-Methyl bedeutet und $R_7$ Aethyl, Kalium oder Wasserstoff bedeutet;

oder (ii) X $CH_2CH_2$, $R_4$ 4-F bedeutet, $R_1$ 3-Methyl und $R_7$ Aethyl, Natrium oder Wasserstoff bedeutet;

j) wie c) ausgenommen, dass Ro + Ro = $(CH_2)_4$ und

(i) X (E)CH=CH, $R_4$ 4-F bedeutet,

$R_1$ 1-Methyl bedeutet und $R_7$ Aethyl, Kalium oder Wasserstoff bedeutet;

oder (ii) X $CH_2CH_2$, $R_4$ 4-F bedeutet, $R_1$ 1-Methyl und $R_7$ Aethyl, Natrium oder Wasserstoff bedeutet;

k) wie d) ausgenommen, dass Ro + Ro = $(CH_2)_4$ und

(i) X (E)CH=CH, $R_4$ 4-F und $R_1$ Wasserstoff, 4-Chlor oder 4-Methyl bedeuten;

oder (ii) X (E)CH=CH bedeutet und $R_4$ und $R_1$ Wasserstoff bedeuten;

oder (iii) X $CH_2CH_2$, $R_4$ 4-F und $R_1$ Wasserstoff bedeuten;

l) wie e) ausgenommen, dass Ro + Ro = $(CH_2)_4$, X (E)CH=CH oder $CH_2CH_2$ bedeutet, $R_4$ 4-F und $R_1$ 3-Methyl bedeuten;

m) wie f) ausgenommen, dass Ro + Ro = $(CH_2)_4$, X (E)CH=CH oder $CH_2CH_2$ bedeutet, $R_4$ 4-F und $R_1$ 1-Methyl bedeuten;

n) wie g) ausgenommen, dass Ro + Ro = $(CH_2)_4$, X (E)CH=CH bedeutet, $R_4$ 4-F und $R_1$ Wasserstoff bedeuten;

o) $R_1$, $R_5$ und $R_{5a}$ Wasserstoff bedeuten, $R_2$ Phenoxy bedeutet, $R_3$ 7-Methyl bedeutet, $R_4$ 3-$CF_3$ bedeutet, X $CH_2$ bedeutet,

Ro + Ro =

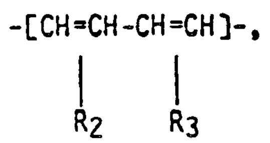

X-Z sich in 1-Stellung befindet, die $R_4$-tragende Gruppe sich in 2-Stellung befindet und

(i) $R_6$ Methyl bedeutet, Z eine Gruppe IIa in erythro-Form bedeutet und $R_7$ Methyl, Natrium oder Wasserstoff bedeutet;

oder (ii) $R_6$ Wasserstoff bedeutet und Z eine Gruppe IIb in trans-Form bedeutet:

p) $R_1$ und $R_{5a}$ Wasserstoff bedeuten, $R_4$ 3-Methyl bedeutet, $R_5$ 4-Methoxy bedeutet, Ro + Ro = $(CH_2)_4$, $R_6$ Methyl bedeutet, X $CH_2$ bedeutet, X-Z sich in 1-Stellung befindet, die $R_4$-tragende Gruppe sich in 2-Stellung befindet und

(i) Z eine Gruppe IIa in erythro-Form bedeutet und $R_7$ Methyl, Natrium oder Wasserstoff bedeutet;

oder (ii) Z eine Gruppe IIb in trans-Form bedeutet.

6. Ein Verfahren nach Anspruch 1, worin

a) Z eine Gruppe IIa in erythro-Form bedeutet, Ro + Ro =

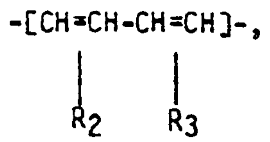

$R_2$, $R_3$, $R_4$, $R_5$, $R_{5a}$ und $R_6$ Wasserstoff bedeuten, $R_4$ 4-F bedeutet, X-Z sich in 2-Stellung befindet, die $R_4$-tragende Gruppe sich in 1-Stellung befindet und

(i) X (E)CH=CH bedeutet, $R_1$ 3-Aethyl oder 3-i-Propyl bedeutet und $R_7$ Aethyl, Natrium oder Wasserstoff bedeutet;

oder (ii) X (E)CH=CH oder $CH_2CH_2$ bedeutet, $R_1$ Wasserstoff bedeutet und $R_7$ Aethyl oder Natrium bedeutet;

oder (iii) X (E)CH=CH bedeutet, $R_1$ 4-Chlor oder 3-Methyl und $R_7$ Natrium bedeutet:

b) wie a) ausgenommen, dass X-Z sich in 2-Stellung befindet, die $R_4$-tragende Gruppe sich in 3-Stellung befindet, $R_1$ Wasserstoff bedeutet, X (E)CH=CH bedeutet und $R_7$ Aethyl, Natrium oder Wasserstoff bedeutet;

c) Z eine Gruppe IIb in trans-Form bedeutet, Ro + Ro =

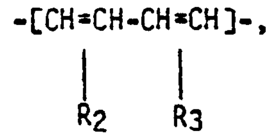

$R_2$, $R_3$, $R_5$, $R_{5a}$ und $R_6$ Wasserstoff bedeuten, $R_4$ 4-F bedeutet und

(i) X-Z sich in 2-Stellung befindet, die $R_4$-tragende Gruppe sich in 1-Stellung befindet, X (E)CH=CH bedeutet und $R_1$ 3-Aethyl oder 3-i-Propyl bedeutet;

oder (ii) X-Z sich in 2-Stellung befindet, die $R_4$-tragende Gruppe sich in Stellung 3 befindet, X (E)CH=CH und $R_1$ Wasserstoff bedeuten;

oder (iii) X-Z sich in 2-Stellung befindet, die $R_4$-tragende Gruppe sich in Stellung 1 befindet, X $CH_2CH_2$ und $R_1$ Wasserstoff bedeuten:

d) Z eine Gruppe IIa in erythro-Form bedeutet, Ro + Ro = $(CH_2)_4$, $R_1$, $R_5$, $R_{5a}$ und $R_6$ Wasserstoff bedeuten, $R_4$ 4-F bedeutet, X (E)CH=CH bedeutet, X-Z sich in 2-Stellung befindet, die $R_4$-tragende Gruppe sich in 3-Stellung befindet und $R_7$ Aethyl, Natrium oder Wasserstoff bedeutet;

e) Z eine Gruppe IIb in trans-Form bedeutet, Ro + Ro = $(CH_2)_4$, $R_1$, $R_5$, $R_{5a}$ und $R_6$ Wasserstoff bedeuten, $R_4$ 4-F bedeutet, X (E)CH=CH bedeutet, X-Z sich in 1- oder 2-Stellung befindet, wenn die $R_4$-tragende Gruppe sich in 2- bzw. 3-Stellung befindet.

7. Ein Verfahren nach Anspruch 1 zur Herstellung einer Verbindung, ausgewählt aus

Erythro-(E)-3R,5S,dihydroxy-7-(2'-[4"-fluorphenyl]naphth-1'-yl)hept-6-enoat oder ein Salz davon;

Erythro-(E)-3,5-dihydroxy-7-(1'-[4"-fluorphenyl]-3'-[1,-methyl-aethyl]naphth-2'-yl)hept-6-enoat oder ein Salz davon;

(E)-Trans-6-(2'-[3"-aethyl-1"-(4'"-fluorphenyl)naphth-2"-yl]-aethenyl)-4-hydroxy-3,4,5,6-tetrahydropyran-2-on.

8. Eine pharmazeutische Zusammensetzung, umfassend eine Verbindung entsprechend einem der Ansprüche 1 bis 7, geeignet in Form der freien Säure oder in Form eines physiologisch hydrolysierbaren und akzeptablen Esters oder eines Laktones davon oder in Form eines pharmazeutisch akzeptablen Salzes, zusammen mit einem pharmazeutisch akzeptablen Verdünner oder Träger.

9. Eine Verbindung entsprechend einem der Ansprüche 1 bis 7, geeignet in Form der freien Säure oder in Form eines physiologisch-hydrolysierbaren und akzeptablen Esters oder eines Laktones davon oder in Form eines pharmazeutisch akzeptablen Salzes zur Anwendung bei der Hemmung der Cholesterin-Biosynthese oder zur Behandlung der Atherosklerose.

10. Ein Verfahren zur Herstellung einer pharmazeutischen Zusammensetzung, indem man eine Verbindung der Formel I,

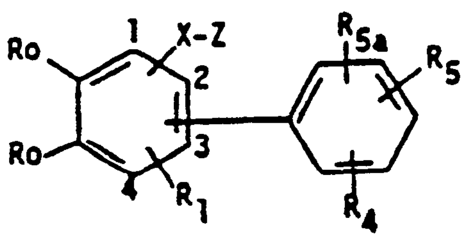

I

worin die Substituenten die gleiche Bedeutung haben wie in einem der Ansprüche 1 bis 6 und geeignet sind in Form der freien Säure oder in Form eines physiologisch hydrolysierbaren Esters oder eines Laktones davon oder in Form eines pharmazeutisch akzeptablen Salzes, zusammen mit einem pharmazeutisch akzeptablen Verdünner oder Träger mischt.

**Revendications**

pour les Etats Contractants: BE CH DE FR GB IT LI LU NL SE

1.- Un composé de formule I

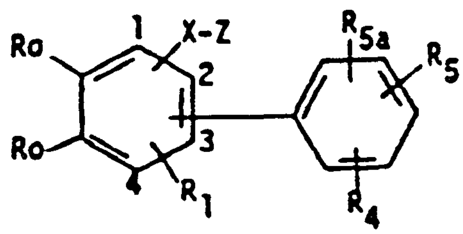

(I)

dans laquelle les deux groupes Ro forment ensemble un radical de formule

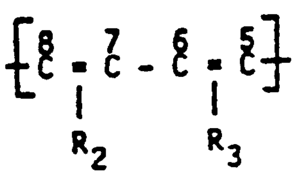

ou $-(CH_2)_4-$

et dans lesquelles

$R_2$ signifie l'hydrogène, alkyle en $C_1$-$C_4$, alcoxy en $C_1$-$C_3$, n-butoxy, i-butoxy, s-butoxy, trifluorométhyle, fluoro, chloro, phénoxy ou benzyloxy,

$R_3$ signifie l'hydrogène, alkyle en $C_1$-$C_3$, alcoxy en $C_1$-$C_3$, trifluorométhyle, fluoro, chloro, phénoxy ou benzyloxy, avec les conditions que au maximum un des symboles $R_2$ et $R_3$ signifie trifluorométhyle, au maximum un des symboles $R_2$ et $R_3$ signifie phénoxy et au maximum un des symboles $R_2$ et $R_3$ signifie benzyloxy,

$R_1$ signifie l'hydrogène, alkyle en $C_1$-$C_6$, fluoro, chloro ou benzyloxy,

$R_4$ signifie l'hydrogène, alkyle en $C_1$-$C_4$, alcoxy en $C_1$-$C_3$, n-butoxy, i-butoxy, s-butoxy, trifluorométhyle, fluoro, chloro, phénoxy ou benzyloxy,

$R_5$ signifie l'hydrogène, alkyle en $C_1$-$C_3$, alcoxy en $C_1$-$C_3$, trifluorométhyle, fluoro, chloro, phénoxy ou benzyloxy,

$R_{5a}$ signifie l'hydrogène, alkyle en $C_1$-$C_2$, alcoxy en $C_1$-$C_2$, fluoro ou chloro,

avec les conditions que au maximum un des symboles $R_4$ et $R_5$ signifie trifluorométhyle, au maximum un des symboles $R_4$ et $R_5$ signifie phénoxy et au maximum un des symboles $R_4$ et $R_5$ signifie benzyloxy,

X signifie $-(CH_2)$ - ou

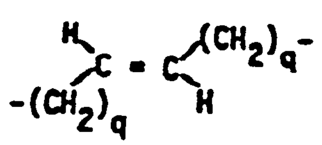

dans lesquels n signifie 0, 1, 2 ou 3 et les deux symboles q signifient 0 ou bien l'un signifie 0 et l'autre signifie 1, et

Z signifie

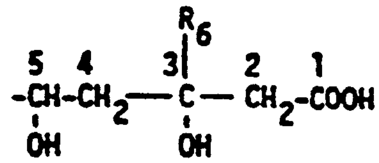

(II)

dans lequel $R_6$ signifie l'hydrogène ou alkyle en $C_1$-$C_3$,

avec la condition générale que -X-Z et le groupe phényle portant $R_4$ soit en ortho l'un par rapport à l'autre;

sous forme d'acide libre ou sous forme d'un ester physiologiquement hydrolysable et physiologiquement acceptable ou d'une δ lactone de ce composé, ou sous forme de sel.

2.- Un composé selon la revendication 1, dans lequel les deux groupes Ro forment ensemble un radical de formule

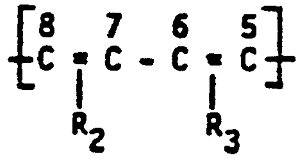

ou $-CH_2CH_2CH_2CH_2-$,

où $R_2$ signifie l'hydrogène, alkyle en $C_1$-$C_3$, n-butyle, i-butyle, alcoxy en $C_1$-$C_3$, n-butoxy, i-butoxy, trifluorométhyle, fluoro, chloro, phénoxy ou benzyloxy,

$R_3$ signifie l'hydrogène, alkyle en $C_1$-$C_3$, alcoxy en $C_1$-$C_3$, trifluorométhyle, fluoro, chloro, phénoxy ou benzyloxy,

avec les conditions que au maximum un des symboles $R_2$ et $R_3$ signifie trifluorométhyle, au maximum un des symboles $R_2$ et $R_2$ signifie phénoxy, et au maximum un des symboles $R_2$ et $R_3$ signifie benzyloxy,

$R_1$ signifie l'hydrogène, alkyle en $C_1$-$C_3$, fluoro, chloro ou benzyloxy,

$R_4$ signifie l'hydrogène, alkyle en $C_1$-$C_3$, n-butyle, i-butyle, alcoxy en $C_1$-$C_3$, n-butoxy, i-butoxy, trifluorométhyle, fluoro, chloro, phénoxy ou benzyloxy,

$R_5$ signifie l'hydrogène, alkyle en $C_1$-$C_3$, alcoxy en $C_1$-$C_3$, trifluorométhyle, fluoro, chloro, phénoxy ou benzyloxy,

$R_{5a}$ signifie l'hydrogène,

avec les conditions que au maximum un des symboles $R_4$ et $R_5$ signifie trifluorométhyle, au maximum un des symboles $R_4$ et $R_5$ signifie phénoxy, et au maximum un des symboles $R_4$ et $R_5$ signifie benzyloxy,

X signifie $-(CH_2)_n-$ ou

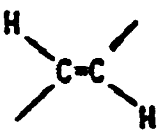

,

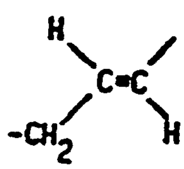

ou

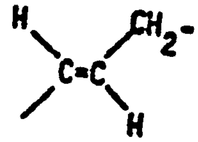

.

où n signifie 0, 1, 2 ou 3, et
Z signifie

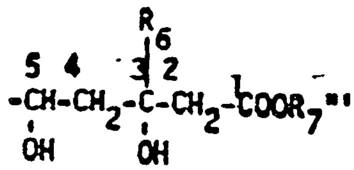

ou

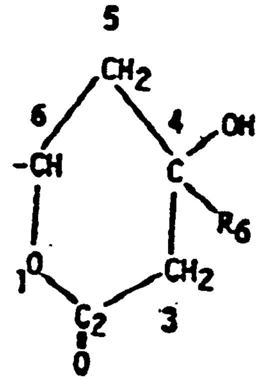

(IIb)

dans lesquels
$R_6$ signifie l'hydrogène ou alkyle en $C_1$-$C_3$, et
$R_7$'' signifie l'hydrogène, alkyle en $C_1$-$C_3$, n-butyle, i-butyle, t-butyle, benzyle ou M, où M signifie un cation pharmaceutiquement acceptable,
avec la condition que le groupe -X-Z et le groupe phényle portant $R_4$ soit en ortho l'un par rapport à l'autre.

3.- Un composé selon la revendication 1, dans lequel Ro + Ro est tel que défini à la revendication 1,
$R_1$ signifie l'hydrogène ou alkyle en $C_1$-$C_3$,
$R_2$, $R_3$, $R_4$ et $R_5$ signifient indépendamment l'hydrogène, méthyle, méthoxy, fluoro ou chloro,
$R_{5a}$ signifie l'hydrogène,
X-Z est en position 1 et représente

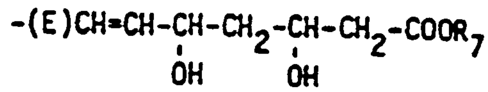

dans lequel $R_7$ signifie l'hydrogène, alkyle en $C_1$-$C_2$ ou un cation pharmaceutiquement acceptable, et le groupe portant $R_4$ est en position 2.

4.- Un composé selon la revendication 3, dans lequel X-Z est en position 2 et le groupe portant $R_4$ est en position 1.

5.- Un composé selon la revendication 1, dans lequel les substituants ont les significations suivantes:
a) Z signifie un groupe IIa sous forme erythro,

Ro + Ro =

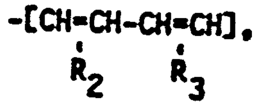

$R_2$, $R_3$, $R_5$, $R_{5a}$ et $R_6$ signifient l'hydrogène, X-Z est en position 1, le groupe portant $R_4$ est en position 2 et
i) X signifie (E)CH = CH, $R_4$ signifie 4-F et $R_1$ signifie l'hydrogène ou 4-méthyle et $R_7$ signifie éthyle, le sodium ou l'hydrogène;
ou
$R_1$ signifie 4-chloro et $R_7$ signifie éthyle, le potassium ou l'hydrogène;
ou
ii) X signifie (E)CH = CH, $R_4$ et $R_1$ signifient l'hydrogène et $R_7$ signifie éthyle, le potassium ou l'hydrogène;
ou
iii) X signifie $CH_2CH_2$, $R_4$ signifie 4-F,
$R_1$ signifie l'hydrogène et $R_7$ signifie éthyle, le sodium ou l'hydrogène;
ou
b) comme a) excepté que X-Z est en position 2, le groupe portant $R_4$ est en position 1 et
i) X signifie (E)CH = CH, $R_4$ signifie 4-F,
$R_1$ signifie 3-méthyle et $R_7$ signifie éthyle, le potassium ou l'hydrogène;
ou
ii) X signifie $CH_2CH_2$, $R_4$ signifie 4-F,
$R_1$ signifie 3-méthyle et $R_7$ signifie éthyle, le sodium ou l'hydrogène:
c) comme a) excepté que X-Z est en position 2, le groupe portant $R_4$ est en position 3 et
i) X signifie (E)CH = CH, $R_4$ signifie 4-F,
$R_1$ signifie 1-méthyle et $R_7$ signifie éthyle, le potassium ou l'hydrogène;
ou
ii) X signifie $CH_2CH_2$, $R_4$ signifie 4-F,
$R_1$ signifie l'hydrogène et $R_7$ signifie méthyle, le sodium ou l'hydrogène:
d) comme a) excepté que Z signifie un groupe IIb sous forme trans et
i) X signifie (E)CH = CH, $R_4$ signifie 4-F et
$R_1$ signifie l'hydrogène, 4-fluoro ou 4-méthyle; ou
ii) X signifie (E)CH = CH et $R_4$ et $R_1$ signifient l'hydrogène;
ou
iii) X signifie $CH_2CH_2$, $R_4$ signifie 4-F et $R_1$ signifie l'hydrogène:
e) comme b) excepté que Z signifie un groupe IIb sous forme trans et $R_4$ signifie 4-F, $R_1$ signifie 3-méthyle et X signifie (E)CH = CH ou $CH_2CH_2$:
f) comme c) excepté que Z signifie un groupe IIb sous forme trans et $R_4$ signifie 4-F, $R_1$ signifie 1-méthyle et X signifie (E)CH = CH ou $CH_2CH_2$:
g) comme a) excepté que Z signifie un groupe IIb sous forme cis, X signifie (E)CH = CH, $R_4$ signifie 4-F et $R_1$ signifie l'hydrogène:
h) comme a) excepté que Ro + Ro = $(CH_2)_4$ et
i) X signifie (E)CH = CH, $R_4$ signifie 4-F et
$R_1$ signifie l'hydrogène ou 4-méthyle et $R_7$ signifie éthyle, le sodium ou l'hydrogène; ou
$R_1$ signifie 4-chloro et $R_7$ signifie éthyle, le potassium ou l'hydrogène;
ou
ii) X signifie $CH_2CH_2$, $R_4$ signifie 4-F,
$R_1$ signifie l'hydrogène et $R_7$ signifie éthyle, le potassium ou l'hydrogène; ou
iii) X signifie (E)CH = CH, $R_4$ et $R_1$ signifient l'hydrogène et $R_7$ signifie éthyle, le potassium ou l'hydrogène:
i) comme b) excepté que Ro + Ro = $(CH_2)_4$ et
i) X signifie (E)CH = CH, $R_4$ signifie 4-F,
$R_1$ signifie 3-méthyle et $R_7$ signifie éthyle, le potassium ou l'hydrogène;
ou
ii) X signifie $CH_2CH_2$, $R_4$ signifie 4-F,
$R_1$ signifie 3-méthyle et $R_7$ signifie éthyle, le sodium ou l'hydrogène:
j) comme c) excepté que Ro + Ro = $(CH_2)_4$ et
i) X signifie (E)CH = CH, $R_4$ signifie 4-F,
$R_1$ signifie 1-méthyle et $R_7$ signifie éthyle, le potassium ou l'hydrogène;
ou
ii) X signifie $CH_2CH_2$, $R_4$ signifie 4-F,
$R_1$ signifie 1-méthyle et $R_7$ signifie éthyle, le sodium ou l'hydrogène:
k) comme d) excepté que Ro + Ro = $(CH_2)_4$ et
i) X signifie (E)CH = CH, $R_4$ signifie 4-F et $R_1$ signifie l'hydrogène;

4-chloro ou 4-méthyle;
ou
ii) X signifie (E)CH = CH et $R_4$ et $R_1$ signifient l'hydrogène,
ou
iii) X signifie $CH_2CH_2$, $R_4$ signifie 4-F et $R_1$ signifie l'hydrogène:
l) comme e) excepté que Ro + Ro = $(CH_2)_4$, X signifie (E)CH = CH ou $CH_2CH_2$, $R_4$ signifie 4-F et $R_1$ signifie 3-méthyle:
m) comme f) excepté que Ro + Ro = $(CH_2)_4$, X signifie (E)CH = CH ou $CH_2CH_2$, $R_4$ signifie 4-F et $R_1$ signifie 1-méthyle:
n) comme g) excepté que Ro + Ro = $(CH_2)_4$, X signifie (E)CH = CH, $R_4$ signifie 4-fluoro et $R_1$ signifie l'hydrogène
o) $R_1$, $R_5$ et $R_{5a}$ signifient l'hydrogène, $R_2$ signifie phénoxy, $R_3$ signifie 7-méthyle, $R_4$ signifie 3-$CF_3$, X signifie $CH_2$, Ro + Ro signifie-

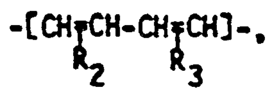

X-Z est en position 1, le groupe portant $R_4$ est en position 2 et
i) $R_6$ signifie méthyle, Z signifie un groupe IIa sous forme erythro et
$R_7$ signifie méthyle, le sodium ou l'hydrogène;
ou
ii) $R_6$ signifie l'hydrogène et Z signifie un groupe IIb sous forme trans:
p) $R_1$ et $R_{5a}$ signifient l'hydrogène, $R_4$ signifie 3-méthyle, $R_5$ signifie 4-méthoxy, Ro + Ro = $(CH_2)_4$, $R_6$ signifie méthyle, X signifie $CH_2$, X-Z est en position 1, le groupe portant $R_4$ est en position 2 et
i) Z signifie un groupe IIa sous forme erythro et $R_7$ signife méthyle, le sodium ou l'hydrogène;
ou
ii) Z signifie un groupe IIb sous forme trans.
6.- Un composé selon la revendication 1, dans lequel
a) Z signifie un groupe IIa sous forme erythro, Ro + Ro =

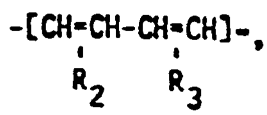

$R_2$, $R_3$, $R_4$, $R_5$, $R_{5a}$ et $R_6$ signifient l'hydrogène, $R_4$ signifie 4-F, X-Z est en position 2, le groupe portant $R_4$ est en position 1 et
i) X signifie (E)CH = CH, $R_1$ signifie 3-éthyle ou 3-i-propyle et $R_7$ signifie éthyle, le sodium ou l'hydrogène;
ou
ii) X signifie (E)CH = CH ou $CH_2CH_2$, $R_1$ signifie l'hydrogène et $R_7$ signifie éthyle ou le sodium;
ou
iii) X signifie (E)CH = CH, $R_1$ signifie 4-chloro ou 3-méthyle et $R_7$ signifie le sodium:
b) comme a) excepté que X-Z est en position 2, le groupe portant $R_4$ est en position 3, $R_1$ signifie l'hydrogène, X signifie (E)CH = CH et $R_7$ signifie éthyle, le sodium ou l'hydrogène:
c) Z signifie un groupe IIb sous forme trans, Ro + Ro =

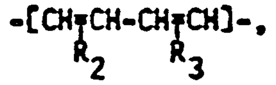

$R_2$, $R_3$, $R_5$, $R_{5a}$ et $R_6$ signifient l'hydrogène, $R_4$ signifie 4-F et
i) X-Z est en position 2, le groupe portant $R_4$ est en position 1,
X signifie (E)CH = CH et $R_1$ signifie 3-éthyle, 3-i-propyle,
ou
ii) X-Z est en position 2, le groupe portant $R_4$ est en position 3,
X = (E)CH = CH et $R_1$ signifie l'hydrogène;

ou

iii) X:Z est en position 2, le groupe portant $R_4$ est en position 1, X signifie $CH_2CH_2$ et $R_1$ signifie l'hydrogène:

d) Z signifie un groupe IIa sous forme erythro, Ro + Ro = $(CH_2)_4$, $R_1$, $R_5$, $R_{5a}$ et $R_6$ signifient l'hydrogène, $R_4$ signifie 4-F, X signifie (E)CH=CH, X-Z est en position 2, le groupe portant $R_4$ est en position 3 et $R_7$ signifie éthyle, le sodium ou l'hydrogène:

e) Z signifie un groupe IIb sous forme trans, Ro + Ro = $(CH_2)_4$, $R_1$, $R_5$, $R_{5a}$ et $R_6$ signifient l'hydrogène, $R_4$ signifie 4-F, X signifie (E)CH=CH et X-Z est en position 1 ou 2 lorsque le groupe $R_4$ est respectivement en position 2 ou 3.

7.- Erythro-(E)-3R,5S,dihydroxy-7-(2'-[4"-fluorophényl]napht -1'-yl)hept-6-énoate ou un sel de ce composé.

8.- Erythro-(E)-3,5-dihydroxy-7-(1'-[4"-fluorophènyl]-3'-[1'-méthyl-éthyl]napht-2'-yl)-hept-6-énoate ou un sel de ce composé.

9.- (E)-trans-6-(2'-[3"-éthyl-1"-(4"-fluorophényl)napht -2"-yl]-éthényl)-4-hydroxy-3,4,5,6-tétrahydropyranne-2-one.

10.- Un composé selon la revendication 7 ou 8 sous forme de sel de sodium.

11.- Une composition pharmaceutique comprenant un composé selon l'une quelconque des revendications 1 à 10 selon que cela est approprié sous forme d'acide libre ou sous la forme d'un ester physiologiquement hydrolysable et physiologiquement acceptable ou d'une lactone de ce composé ou sous forme d'un sel pharmaceutiquement acceptable, en association avec un diluant ou véhicule pharmaceutiquement acceptable.

12.- Un composé selon l'une quelconque des revendications 1 à 10, selon que cela est approprié sous forme d'acide libre ou sous la forme d'un ester physiologiquement hydrolysable et physiologiquement acceptable ou d'une lactone de ce composé ou sous forme d'un sel pharmaceutiquement acceptable, pour l'utilisation dans l'inhibition de la biosynthèse du cholestérol ou pour le traitement de l'athérosclérose.

13.- Un procédé de préparation d'un composé selon la revendication 1 qui comprend

a) lorsque $R_6$ signifie l'hydrogène, la réduction d'un composé de formule VI

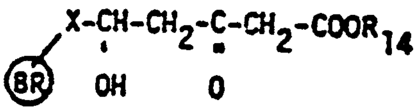

(VI)

dans laquelle $R_{14}$ signifie un radical formant un ester physiologiquement hydrolysable et physiologiquement acceptable et X est tel que défini ci-dessus,

b) lorsque $R_6$ = alkyle en $C_1$-$C_3$, l'hydrolyse d'un composé de formule XVII

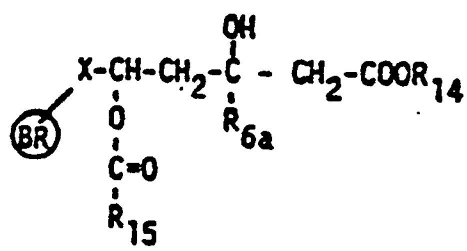

(XVII)

dans laquelle $R_{6a}$ signifie alkyle en $C_1$-$C_3$, $R_{15}$ signifie alkyle en $C_1$-$C_2$ et X et $R_{14}$ sont tels que définis ci-dessus,

c) lorsque X signifie

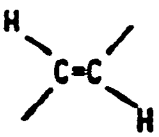

la déprotection d'un composé de formule LIX

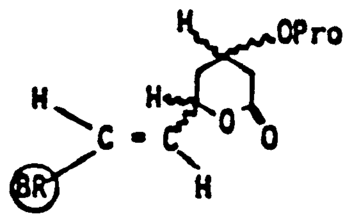

(LVIII)

dans laquelle Pro signifie un groupe protecteur,

d) l'hydrolyse d'un composé de formule I sous forme d'un ester physiologiquement hydrolysable ou d'une lactone ou

e) l'estérification ou la lactonisation d'un composé de formule I sous forme d'acide libre,

et lorsqu'un groupe carboxy libre est présent, la récupération du composé obtenu sous forme d'acide libre ou sous forme d'un sel,

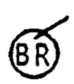 représentant la structure cyclique de base

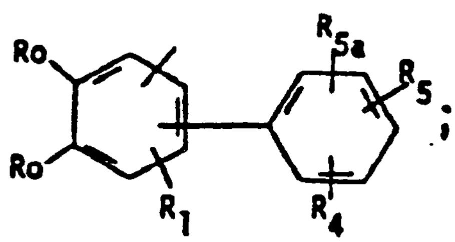

Ro, $R_1$, $R_4$, $R_{5a}$, $R_5$, X et Z étant tels que définis à la revendication 1.

**Revendications**

pour l'Etat Contractant AT

1.- Procédé de préparation d'un composé de formule I

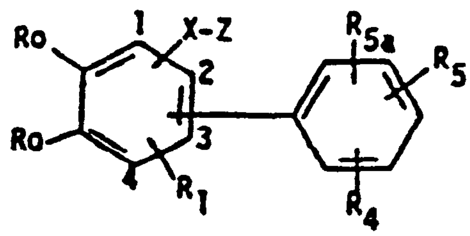

(I)

dans laquelle les deux groupes Ro forment ensemble un radical de formule

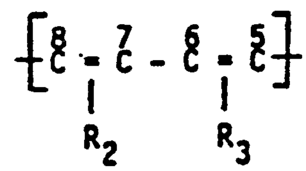 ou $-(CH_2)_4-$ ,

et dans lesquelles

$R_2$ signifie l'hydrogène, alkyle en $C_1$-$C_4$, alcoxy en $C_1$-$C_3$, n-butoxy, i-butoxy, s-butoxy, trifluorométhyle, fluoro, chloro, phénoxy ou benzyloxy,

$R_3$ signifie l'hydrogène, alkyle en $C_1$-$C_3$, alcoxy en $C_1$-$C_3$, trifluorométhyle, fluoro, chloro, phénoxy ou benzyloxy, avec les conditions que au maximum un des symboles $R_2$ et $R_3$ signifie trifluorométhyle, au maximum un des symboles $R_2$ et $R_3$ signifie phénoxy et au maximum un des symboles $R_2$ et $R_3$ signifie benzyloxy,

$R_1$ signifie l'hydrogène, alkyle en $C_1$-$C_6$, fluoro, chloro ou benzyloxy,

$R_4$ signifie l'hydrogène, alkyle en $C_1$-$C_4$, alcoxy en $C_1$-$C_3$, n-butoxy, i-butoxy, s-butoxy, trifluorométhyle, fluoro, chloro, phénoxy ou benzyloxy,

$R_5$ signifie l'hydrogène, alkyle en $C_1$-$C_3$, alcoxy en $C_1$-$C_3$, trifluorométhyle, fluoro, chloro, phénoxy ou benzyloxy,

$R_{5a}$ signifie l'hydrogène, alkyle en $C_1$-$C_2$, alcoxy en $C_1$-$C_2$, fluoro ou chloro,

avec les conditions que au maximum un des symboles $R_4$ et $R_5$ signifie trifluorométhyle, au maximum un des symboles $R_4$ et $R_5$ signifie phénoxy et au maximum un des symboles $R_4$ et $R_5$ signifie benzyloxy,

X signifie $-(CH_2)$ - ou

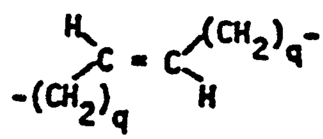

dans lesquels n signifie 0, 1, 2 ou 3 et les deux symboles q signifient 0 ou bien l'un signifie 0 et l'autre signifie 1, et

Z signifie

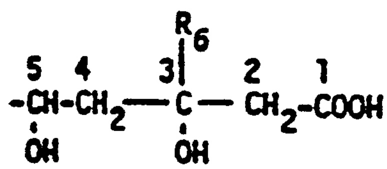

(II)

dans lequel $R_6$ signifie l'hydrogène ou alkyle en $C_1$-$C_3$,

avec la condition générale que -X-Z et le groupe phényle portant $R_4$ soit en ortho l'un par rapport à l'autre;

sous forme d'acide libre ou sous forme d'un ester physiologiquement hydrolysable et physiologiquement

acceptable ou d'une δ lactone de ce composé, ou sous forme de sel, qui comprend

a) lorsque $R_6$ signifie l'hydrogène, la réduction d'un composé de formule VI

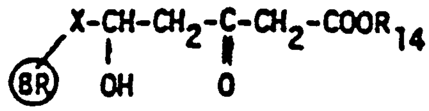

(VI)

dans laquelle $R_{14}$ signifie un radical formant un ester physiologiquement hydrolysable et physiologiquement acceptable et X est tel que défini ci-dessus,

b) lorsque $R_6$ = alkyle en $C_1$-$C_3$, l'hydrolyse d'un composé de formule XVII

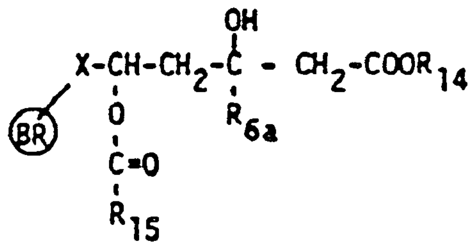

(XVII)

dans laquelle $R_{6a}$ signifie alkyle en $C_1$-$C_3$, $R_{15}$ signifie alkyle en $C_1$-$C_2$ et X et $R_{14}$ sont tels que définis ci-dessus,

c) lorsque X signifie

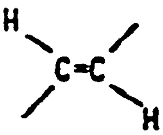

la déprotection d'un composé de formule LIX

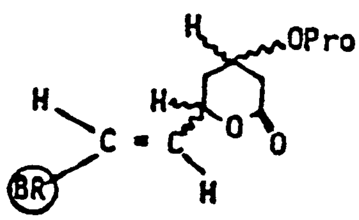

(LVIII)

dans laquelle Pro signifie un groupe protecteur,

d) l'hydrolyse d'un composé de formule I sous forme d'un ester physiologiquement hydrolysable ou d'une lactone ou

e) l'estérification ou la lactonisation d'un composé de formule I sous forme d'acide libre,

et lorsqu'un groupe carboxy libre est présent, la récupération du composé obtenu sous forme d'acide libre ou sous forme d'un sel,

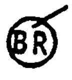 représentant la structure cyclique de base

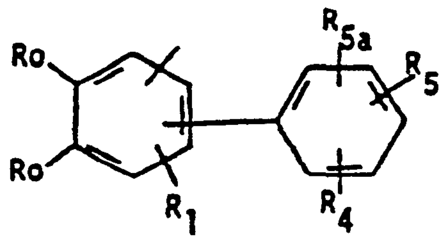

;

Ro, $R_1$, $R_4$, $R_{5a}$, $R_5$, X et Z étant tels que définis à la revendication 1.

2.- Un procédé selon la revendication 1, dans lequel les deux groupes Ro forment ensemble un radical de formule

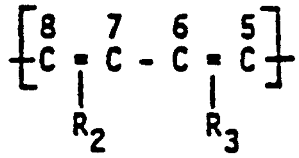

ou $-CH_2CH_2CH_2CH_2-$,

où $R_2$ signifie l'hydrogène, alkyle en $C_1$-$C_3$,n-butyle, i-butyle, alcoxy en $C_1$-$C_3$, n-butoxy, i-butoxy, trifluorométhyle, fluoro, chloro, phénoxy ou benzyloxy,

$R_3$ signifie l'hydrogène, alkyle en $C_1$-$C_3$, alcoxy en $C_1$-$C_3$, trifluorométhyle, fluoro, chloro, phénoxy ou benzyloxy,

avec les conditions que au maximum un des symboles $R_2$ et $R_3$ signifie trifluorométhyle, au maximum un des symboles $R_2$ et $R_2$ signifie phénoxy, et au maximum un des symboles $R_2$ et $R_3$ signifie benzyloxy,

$R_1$ signifie l'hydrogène, alkyle en $C_1$-$C_3$, fluoro, chloro ou benzyloxy,

$R_4$ signifie l'hydrogène, alkyle en $C_1$-$C_3$, n-butyle, i-butyle, alcoxy en $C_1$-$C_3$, n-butoxy, i-butoxy, trifluorométhyle, fluoro, chloro, phénoxy ou benzyloxy,

$R_5$ signifie l'hydrogène, alkyle en $C_1$-$C_3$, alcoxy en $C_1$-$C_3$, trifluorométhyle, fluoro, chloro, phénoxy ou benzyloxy,

$R_{5a}$ signifie l'hydrogène,

avec les conditions que au maximum un des symboles $R_4$ et $R_5$ signifie trifluorométhyle, au maximum un des symboles $R_4$ et $R_5$ signifie phénoxy, et au maximum un des symboles $R_4$ et $R_5$ signifie benzyloxy,

X signifie $-(CH_2)_n-$ ou

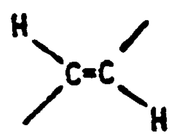

,

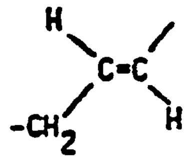

ou

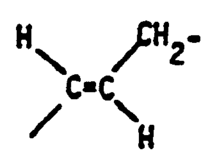

,

où n signifie 0, 1, 2 ou 3, et

Z signifie

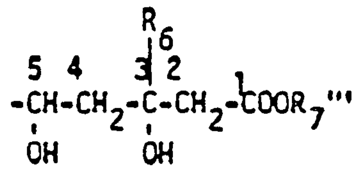

ou

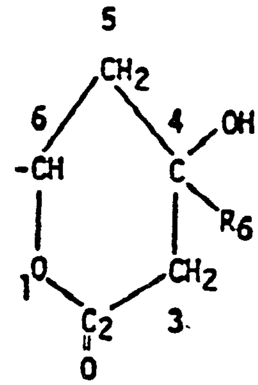

(IIb)

dans lesquels

$R_6$ signifie l'hydrogène ou alkyle en $C_1$-$C_3$, et

$R_7$'' signifie l'hydrogène, alkyle en $C_1$-$C_3$, n-butyle, i-butyle, t-butyle, benzyle ou M, ou M signifie un cation pharmaceutiquement acceptable,

avec la condition que le groupe -X-Z et le groupe phényle portant $R_4$ soit en ortho l'un par rapport à l'autre.

3.- Un procédé selon la revendication 1, dans lequel Ro + Ro est tel que défini à la revendication 1,

$R_1$ signifie l'hydrogène ou alkyle en $C_1$-$C_3$,

$R_2$, $R_3$, $R_4$ et $R_5$ signifient indépendamment l'hydrogène, méthyle, méthoxy, fluoro ou chloro,

$R_{5a}$ signifie l'hydrogène,

X-Z est en position 1 et représente

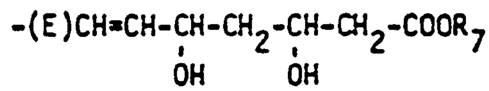

dans lequel $R_7$ signifie l'hydrogène, alkyle en $C_1$-$C_2$ ou un cation pharmaceutiquement acceptable, et le groupe portant $R_4$ est en position 2.

4.- Un procédé selon la revendication 3, dans lequel X-Z est en position 2 et le groupe portant $R_4$ est en position 1.

5.- Un procédé selon la revendication 1, dans lequel les substituants ont les significations suivantes:

a) Z signifie un groupe IIa sous forme erythro,

Ro + Ro = 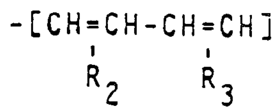

$R_2$, $R_3$, $R_5$, $R_{5a}$ et $R_6$ signifient l'hydrogène, X-Z est en position 1, le groupe portant $R_4$ est en position 2 et

i) X signifie (E)CH = CH, $R_4$ signifie 4-F et

$R_1$ signifie l'hydrogène ou 4-méthyle et $R_7$ signifie éthyle, le sodium ou l'hydrogène; ou

$R_1$ signifie 4-chloro et $R_7$ signifie éthyle, le potassium ou l'hydrogène;

ou

ii) X signifie (E)CH = CH, $R_4$ et $R_1$ signifient l'hydrogène et $R_7$ signifie éthyle, le potassium ou l'hydrogène;

ou
iii) X signifie $CH_2CH_2$, $R_4$ signifie 4-F,
$R_1$ signifie l'hydrogène et $R_7$ signifie éthyle, le sodium ou l'hydrogène;
ou
b) comme a) excepté que X-Z est en position 2, le groupe portant $R_4$ est en position 1 et
i) X signifie (E)CH = CH, $R_4$ signifie 4-F,
$R_1$ signifie 3-méthyle et $R_7$ signifie éthyle, le potassium ou l'hydrogène;
ou
ii) X signifie $CH_2CH_2$, $R_4$ signifie 4-F,
$R_1$ signifie 3-méthyle et $R_7$ signifie éthyle, le sodium ou l'hydrogène:
c) comme a) excepté que X-Z est en position 2, le groupe portant $R_4$ est en position 3 et
i) X signifie (E)CH = CH, $R_4$ signifie 4-F,
$R_1$ signifie 1-méthyle et $R_7$ signifie éthyle, le potassium ou l'hydrogène;
ou
ii) X signifie $CH_2CH_2$, $R_4$ signifie 4-F,
$R_1$ signifie l'hydrogène et $R_7$ signifie méthyle, le sodium ou l'hydrogène:
d) comme a) excepté que Z signifie un groupe IIb sous forme trans et
i) X signifie (E)CH = CH, $R_4$ signifie 4-F et
$R_1$ signifie l'hydrogène, 4-fluoro ou 4-méthyle; ou
ii) X signifie (E)CH = CH et $R_4$ et $R_1$ signifient l'hydrogène;
ou
iii) X signifie $CH_2CH_2$, $R_4$ signifie 4-F et R signifie l'hydrogène:
e) comme b) excepté que Z signifie un groupe IIb sous forme trans et $R_4$ signifie 4-F, $R_1$ signifie 3-méthyle et X signifie (E)CH = CH ou $CH_2CH_2$:
f) comme c) excepté que Z signifie un groupe IIb sous forme trans et $R_4$ signifie 4-F, $R_1$ signifie 1-méthyle et X signifie (E)CH = CH ou $CH_2CH_2$:
g) comme a) excepté que Z signifie un groupe IIb sous forme cis, X signifie (E)CH = CH, $R_4$ signifie 4-F et $R_1$ signifie l'hydrogène:
h) comme a) excepté que Ro + Ro = $(CH_2)_4$ et
i) X signifie (E)CH = CH, $R_4$ signifie 4-F et
$R_1$ signifie l'hydrogène ou 4-méthyle et $R_7$ signifie éthyle, le sodium ou l'hydrogène; ou
$R_1$ signifie 4-chloro et $R_7$ signifie éthyle, le potassium ou l'hydrogène;
ou
ii) X signifie $CH_2CH_2$, $R_4$ signifie 4-F,
$R_1$ signifie l'hydrogène et $R_7$ signifie éthyle, le potassium ou l'hydrogène;
ou
iii) X signifie (E)CH = CH, $R_4$ et $R_1$ signifient l'hydrogène et $R_7$ signifie éthyle, le potassium ou l'hydrogène:
i) comme b) excepté que Ro + Ro = $(CH_2)_4$ et
i) X signifie (E)CH = CH, $R_4$ signifie 4-F,
$R_1$ signifie 3-méthyle et $R_7$ signifie éthyle, le potassium ou l'hydrogène;
ou
ii) X signifie $CH_2CH_2$, $R_4$ signifie 4-F,
$R_1$ signifie 3-méthyle et $R_7$ signifie éthyle, le sodium ou l'hydrogène:
j) comme c) excepté que Ro + Ro = $(CH_2)_4$ et
i) X signifie (E)CH = CH, $R_4$ signifie 4-F,
$R_1$ signifie 1-méthyle et $R_7$ signifie éthyle, le potassium ou l'hydrogène;
ou
ii) X signifie $CH_2CH_2$, $R_4$ signifie 4-F,
$R_1$ signifie 1-méthyle et $R_7$ signifie éthyle, le sodium ou l'hydrogène:
k) comme d) excepté que Ro + Ro = $(CH_2)_4$ et
i) X signifie (E)CH = CH, $R_4$ signifie 4-F et $R_1$ signifie l'hydrogène;
4-chloro ou 4-méthyle;
ou
ii) X signifie (E)CH = CH et $R_4$ et $R_1$ signifient l'hydrogène,
ou
iii) X signifie $CH_2CH_2$, $R_4$ signifie 4-F et $R_1$ signifie l'hydrogène:
l) comme e) excepté que Ro + Ro = $(CH_2)_4$, X signifie (E)CH = CH ou $CH_2CH_2$, $R_4$ signifie 4-F et $R_1$ signifie 3-méthyle:
m) comme f) excepté que Ro + Ro = $(CH_2)_4$, X signifie (E)CH = CH ou $CH_2CH_2$, $R_4$ signifie 4-F et $R_1$ signifie 1-méthyle:
n) comme g) excepté que Ro + Ro = $(CH_2)_4$, X signifie (E)CH = CH, $R_4$ signifie 4-fluoro et $R_1$ signifie l'hydrogène:
o) $R_1$, $R_5$ et $R_{5a}$ signifient l'hydrogène, $R_2$ signifie phénoxy, $R_3$ signifie 7-méthyle, $R_4$ signifie 3-$CF_3$,
X signifie $CH_2$, Ro + Ro signifie f. p. 95
X-Z est en position 1, le groupe portant $R_4$ est en position 2 et

i) $R_6$ signifie méthyle, Z signifie un groupe IIa sous forme erythro et $R_7$ signifie méthyle, le sodium ou l'hydrogène;
ou
ii) $R_6$ signifie l'hydrogène et Z signifie un groupe IIb sous forme trans:
p) $R_1$ et $R_{5a}$ signifient l'hydrogène, $R_4$ signifie 3-méthyle, $R_5$ signifie 4-méthoxy, Ro + Ro = $(CH_2)_4$, $R_6$ signifie méthyle, X signifie $CH_2$, X-Z est en position 1, le groupe portant $R_4$ est en position 2 et
i) Z signifie un groupe IIa sous forme erythro et $R_7$ signife méthyle, le sodium ou l'hydrogène;
ou
ii) Z signifie un groupe IIb sous forme trans.

6.- Un procédé selon la revendication 1, dans lequel
a) Z signifie un groupe IIa sous forme erythro, Ro + Ro =

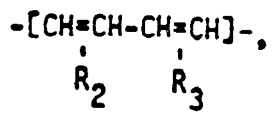

$R_2$, $R_3$, $R_4$, $R_5$, $R_{5a}$ et $R_6$ signifient l'hydrogène, $R_4$ signifie 4-F, X-Z est en position 2, le groupe portant $R_4$ est en position 1 et
i) X signifie (E)CH = CH, $R_1$ signifie 3-éthyle ou 3-i-propyle et $R_7$ signifie éthyle, le sodium ou l'hydrogène;
ou
ii) X signifie (E)CH = CH ou $CH_2CH_2$, $R_1$ signifie l'hydrogène et $R_7$ signifie éthyle ou le sodium;
ou
iii) X signifie (E)CH = CH, $R_1$ signifie 4-chloro ou 3-méthyle et $R_7$ signifie le sodium:
b) comme a) excepté que X-Z est en position 2, le groupe portant $R_4$ est en position 3, $R_1$ signifie l'hydrogène,
X signifie (E)CH = CH et $R_7$ signifie éthyle, le sodium ou l'hydrogène:
c) Z signifie un groupe IIb sous forme trans, Ro + Ro =

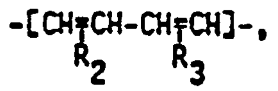

$R_2$, $R_3$, $R_5$, $R_{5a}$ et $R_6$ signifient l'hydrogène, $R_4$ signifie 4-F et
i) X-Z est en position 2, le groupe portant $R_4$ est en position 1,
X signifie (E)CH = CH et $R_1$ signifie 3-éthyle, 3-i-propyle,
ou
ii) X-Z est en position 2, le groupe portant $R_4$ est en position 3,
X = (E)CH = CH et $R_1$ signifie l'hydrogène;
ou
iii) X-Z est en position 2, le groupe portant $R_4$ est en position 1,
X signifie $CH_2CH_2$ et $R_1$ signifie l'hydrogène:
d) Z signifie un groupe IIa sous forme erythro, Ro + Ro = $(CH_2)_4$, $R_1$, $R_5$, $R_{5a}$ et $R_6$ signifient l'hydrogène, $R_4$ signifie 4-F, X signifie (E)CH = CH, X-Z est en position 2, le groupe portant $R_4$ est en position 3 et $R_7$ signifie éthyle, le sodium ou l'hydrogène:
e) Z signifie un groupe IIb sous forme trans, Ro + Ro = $(CH_2)_4$, $R_1$, $R_5$, $R_{5a}$ et $R_6$ signifient l'hydrogène, $R_4$ signifie 4-F, X signifie (E)CH = CH et X-Z est en position 1 ou 2 lorsque le groupe $R_4$ est respectivement en position 2 ou 3.

7.- Un procédé selon la revendication 1, dans laquelle le composé préparé est choisi parmi
l'erythro-(E)-3R,5S,dihydroxy-7-(2'-[4''-fluorophényl]napht-1'-yl)hept-6-énoate ou un sel de ce composé,
l'erythro-(E)-3,5-dihydroxy-7-(1'-[4''-fluorophényl]-3'-[1'-méthyl-éthyl]napht-2'-yl)-hept-6-énoate ou un sel de ce composé,
la (E)-trans-6-(2'-[3''-éthyl-1''-(4''-fluorophényl)napht-2''-yl]-éthényl)-4-hydroxy-3,4,5,6-tétrahydropyranne-2-one.

8.- Une composition pharmaceutique comprenant un composé préparé selon l'une quelconque des revendications 1 à 7 selon que cela est approprié sous forme d'acide libre ou sous la forme d'un ester physiologiquement hydrolysable et physiologiquement acceptable ou d'une lactone de ce composé ou sous forme de sel pharmaceutiquement acceptable, en association avec un diluant ou véhicule pharmaceutiquement acceptable.

9.- Un composé préparé selon l'une quelconque des revendications 1 à 7 selon que cela est approprié sous

forme d'acide libre ou sous la forme d'un ester physiologiquement hydrolysable et physiologiquement acceptable ou d'une lactone de ce composé ou sous forme de sel pharmaceutiquement acceptable,pour l'utilisation dans l'inhibition de la biosynthèse du cholestérol ou pour le traitement de l'athérosclérose.

10.- Un procédé de préparation d'une composition pharmaceutique comprenant l'admixtion d'un composé de formule I

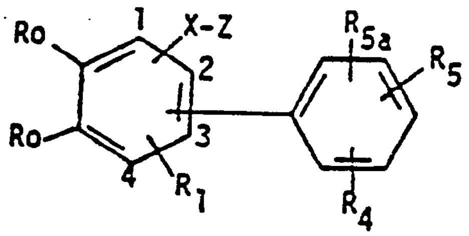

(I)

dans laquelle les substituants sont tels que définis à l'une quelconque des revendications 1 à 6, selon que cela est approprié sous forme d'acide libre ou sous la forme d'un ester physiologiquement hydrolysable et physiologiquement acceptable ou d'une lactone de ce composé ou sous forme de sel pharmaceutiquement acceptable, avec un diluant ou véhicule pharmaceutiquement acceptable.